# EUROPEAN PATENT APPLICATION

(11) **EP 2 412 316 A2**
(43) Date of publication of application: **01.02.2012**
(21) Application number: 11186500.2
(22) Date of filing: 19.03.2010
(51) Int. Cl.: A61B 17/00, A61B 17/064, A61B 17/068, A61F 2/24

(54) **Reconfiguring heart features**

(30) Priority: 19.03.2009 US 407656; 21.09.2009 US 563293
(62) Divisional of application: 10754160.9
(71) Applicant: Millipede, LLC, Ann Arbor, MI 48105 (US)
(72) Inventor: Bolling, Steven, F., Ann Arbor, MI Michigan 48105 (US); Abbs, Jeremy, A., Minneapolis, MN Minnesota 55410 (US); Biancucci, Brian, A., Chelsea, MI Michigan 48118 (US)
(74) Representative: Peterreins, Frank

(57) **Abstract**

Among other things, a heart tissue support has gripping elements, each element having a free end that is sharp enough to penetrate heart tissue when pushed against the tissue, and a feature to resist withdrawal from the tissue after the sharp free end has penetrated the tissue. Among other things, the shape of a heart valve annulus is corrected in a catheter laboratory by orienting a tip of a catheter holding a heart tissue support that has gripping elements at the valve annulus, applying a radial force from the catheter against the annulus by opening a structure at the tip of the catheter, and while the structure is opened, forcing the support onto the annulus. Among other things, the shape of a heart valve annulus is corrected during a surgical procedure by pushing a heart tissue support that has gripping elements onto the annulus.

## Description

### Reconfiguring Heart Features

This application is a continuation-in-part of and claims the benefit of the priority of United States patent application 12/563,293, filed on September 21, 2009, which is a continuation-in-part of United States patent application 12/407,656, filed on March 19, 2009, which is a continuation-in-part of United States patent application 11/620,955, filed on January 8, 2007, all of which are incorporated here in their entirety by reference.

### Background

This description relates to reconfiguring heart features.

The annulus of a heart valve (a fibrous ring attached to the wall of the heart), for example, maintains the shape of the valve opening and supports the valve leaflets. In a healthy heart, the annulus is typically round and has a diameter that enables the leaflets to close the valve tightly, ensuring no blood regurgitation during contraction of the heart. Because the annulus of the tricuspid valve, for example, is supported more stably by the heart tissue on one side of the annulus than on the other side, and for other reasons, the size and shape of the annulus may become distorted over time. The distortion may prevent the valve from closing properly, allowing blood to regurgitate backwards through the valve. The distortion can be corrected, for example, during open heart surgery, by attaching a ring or other support around the annulus to restore its shape and size.

### Summary

In general, in an aspect, a heart tissue support has gripping elements, each gripping element having a free end that is sharp enough to penetrate heart tissue when pushed against the tissue, and a feature to resist withdrawal of the gripping element from the tissue after the sharp free end has penetrated the tissue.

Implementations may include one or more of the following features. The free ends of the gripping elements may project away from a surface of the support. The feature that resists withdrawal of the gripping element from the tissue may comprise a finger projecting laterally from the gripping element. The heart tissue support may comprise an annular surface bearing the gripping elements. The support may be expandable and contractible. The support may have a native size that is configurable. A wire may configure the native size. The support may comprise at least one of stainless steel, gold, Nitinol, or a biologically compatible elastomer. The support may comprise a torus. The support may comprise a helically wound portion. Some portions of the support may bear no gripping elements. The gripping elements may be organized in a pattern. The pattern may comprise rows. The pattern may comprise a group in which the gripping elements are more densely placed and a group in which the gripping elements are less densely placed. The pattern may comprise arcs. The pattern may comprise clusters. The pattern may comprise random placement. At least some of the gripping elements may comprise at least one of platinum, gold, palladium, rhenium, tantalum, tungsten, molybdenum, nickel, cobalt, stainless steel, Nitinol, and alloys of any combination of them. The gripping elements may have the same size. Some of the gripping elements may be of different sizes. At least some of the gripping elements may have more than one of the feature that resists withdrawal. At least some of the gripping elements may project from the surface orthogonally. At least some of the gripping elements may be curved. The heart tissue support may also include a sleeve through which tissue can grow. The sleeve may comprise polyethylene terephthalate. There may be between about 15 and a million gripping elements on the support. There may be between about 100 and about 100,000 gripping elements. The gripping elements may comprise burr hooks. The gripping elements may comprise arrows. The gripping elements may comprise hooks.

The support may include annular elements, pairs of which are connected along edges of the elements to form a ring. Each of annular elements may bear at least one of the grippers. Each of the annular elements may also bear a pointed element aimed in an opposite direction from the gripper. Each of the annular elements may comprise a hexagon.

In general, in an aspect, the shape of a heart valve annulus is corrected in a catheter laboratory by orienting a tip of a catheter holding a heart tissue support that has gripping elements at the valve annulus, applying a radial force from the catheter against the annulus by opening a structure at the tip of the catheter, and while the structure is opened, forcing the support onto the valve annulus.

In general, in an aspect, the shape of a heart valve annulus is corrected during a surgical procedure by pushing a heart tissue support that has gripping elements onto the valve annulus.

In general, in an aspect, a method comprises attaching, to different sized heart valve annuli in different patients, supports that can be expanded in preparation for attachment and allowed to contract to a common relaxed, non-expanded native size when they are in place on the annuli, and reducing the sizes of at least some of the in-place supports to be smaller than the common relaxed non-expanded native size, to accommodate the different sized heart valve annuli of different patients.

In general, in an aspect, a heart tissue support comprises a large number of small grippers, each having a tissue penetration feature and a retention feature, and the configuration of the grippers relative to a configuration of a given area of heart tissue to which the support is to be attached by force being such that the penetration features of a failed set of the grippers will fail to penetrate the tissue, the penetration features of a second set of the grippers will successfully penetrate the tissue, the retention features of a subset of the second set of grippers will fail to retain the grippers in the tissue, and the retention features of the remaining grippers of the second set will successfully retain the grippers in the tissue and hold the support in an intended configuration on the tissue.

In general, in an aspect, a method comprises pushing a support onto a region of heart tissue to cause only a portion of a number of small grippers on the support to embed themselves and be retained in the tissue, the portion being sufficient to attach the support securely to the heart tissue.

In general, in an aspect, an annular heart valve support is expandable and contractible and bears gripping elements configured to penetrate heart tissue and to retain the elements in the tissue after penetration.

In general, in an aspect, a tool to attach a support to a heart valve annulus comprises mechanisms to hold the support in an expanded configuration prior to attachment, to expand the heart valve annulus prior to attachment, to enable the attachment of the support in its expanded configuration to the expanded valve annulus, and to release the expanded support to a contracted configuration after the attachment.

Implementations may include one or more of the following features. The tool may be attached to an end of a catheter. The tool may also comprise an inflatable balloon. The balloon may play a role in positioning the tool. The mechanisms may also be to remove the tool from the heart after attachment.

In general, in an aspect, tool to attach a support to a heart valve annulus comprises a structure to expand the annulus of the heart to a predetermined shape under control of an operator.

Implementations may include one or more of the following features. The structure of the tool may have a conical outer surface at least a portion of which conforms to the predetermined shape. The structure of the tool may have an outer surface that can be expanded to the predetermined shape.

In general, in an aspect, a support for living tissue includes gripping elements to attach to the living tissue and to hold the support securely in place. An annular structure is coupled to the gripping elements. The annular structure adjusts the support between a first configuration for installing the support and a second configuration in which the support is held securely in place after installation. The annular structure is self-supporting in the first and second configurations.

Implementations may include one or more of the following features. The living tissue includes heart tissue, for example, a heart valve annulus. The gripping elements are configured to penetrate the tissue during installation and to grip the tissue after installation. The annular structure is round. The annular structure has elements that move annularly relative to one another to adjust the support between the first configuration and the second configuration. The annular structure is larger when the support is in the first configuration than when the support is in the second configuration. The annular structure changes its shape to adjust the support. The annular structure changes its shape during installation without altering locations of the gripping elements relative to the living tissue.

The annular structure includes a first element to which the gripping elements are attached and a second element that can slide relative to the first element when the annular structure adjusts the support between the first configuration and the second configuration. The first element includes a resilient annular tube to which the gripping elements are attached and the second element includes a rigid adjustable piece that can slide within the tube. The second element includes a self-supporting coil. The self-supporting coil includes a rigid strip material having two free ends that are movable relative to one another to adjust the support between the first configuration and the second configuration. The annular structure includes features that define spacings of the gripping elements that are coupled to the annular structure.

The gripping elements are adjustable between a first arrangement for installing the support and a second arrangement in which the support is held securely in place after installation. The annular structure and the gripping elements are configured so that, when the support is adjusted between the first configuration and the second configuration, the gripping elements are automatically adjusted between the first arrangement and the second arrangement. The annular structure includes a cross-sectional area that increases when the support is adjusted between the first configuration and the second configuration, and the gripping elements are coupled to the annular structure to cause the gripping elements to be adjusted between the first arrangement and the second arrangement when the cross-sectional area of the annular structure decreases.

Each of the gripping elements includes a loop and a piercing element attached to the loop, and the orientation of the piercing element changes as a configuration of the loop changes between the first arrangement and the second arrangement. The adjustment of the support between the first configuration and the second configuration is reversible without the gripping elements damaging the tissue.

The annular structure includes a lock that prevents a change in the configuration of the support. The lock includes a pair of mating elements. One of the mating elements includes a tab and the other includes a slot. One of the mating elements includes a pin and the other includes a hole for the pin. The annular structure has a central axis and includes two portions that can be moved relative to one another around the central axis to a position at which the mating elements mate.

In general, in an aspect, a gripper includes a point to penetrate living tissue and a resilient loop to support the point. The resilient loop has a relaxed configuration and a non-relaxed configuration. The point has different orientations respectively associated with the relaxed configuration and the non-relaxed configuration.

Implementations may include one or more of the following features. The point has at least one barb. The gripper includes more than one such point. The orientation of the point the resilient support having a configuration that

The gripper includes wire. The loop is round. The gripper includes a formed strip of a material. There are two such points that face each other and act as a pincer in at least one of the relaxed configuration and the non-relaxed configuration.

In general, in an aspect, a heart valve repair ring includes a round self-supporting resilient ring that has a relaxed diameter that corresponds to a healthy heart valve and an enlarged diameter that fits an insertion tool. Grippers are attached to the resilient ring and oriented in a direction that changes automatically between an insertion orientation and an installed orientation during installation.

In general, in an aspect, a method includes forcing pointed grippers on a support to penetrate tissue of a heart valve annulus and to hold the support securely on the annulus to correct a shape of the annulus. Then, at least a portion of the support is removed from the annulus by withdrawing at least some of the pointed grippers without damaging the heart value annulus.

In general, in an aspect, a method includes mounting an expanded resilient support on a heart valve annulus, allowing the support to contract to a diameter of a healthy annulus, and locking the contracted support in its contracted diameter by causing elements of the support to mate.

In general, in an aspect, a method includes causing gripping elements that are coupled to a heart annulus support ring to seat in tissue of the annulus by permitting the support ring to contract from an expanded size to a smaller size during installation. Among advantages of these and other aspects and features are one or more of the following. The operator need not work as slowly in order to correctly attach the heart tissue support to the annulus, nor does placement require as much precision. Not all of the burr hooks or grippers need be attached to the annulus to keep the support in place. Some of the burr hooks or grippers might fail to grab onto tissue, or be pulled away from tissue by force. Nonetheless, as long as a minimum threshold percentage of the burr hooks or grippers remain in place, so will the tissue support. Further, because of its ease and simplicity, this procedure can be done in a catheterization laboratory, as well as in surgery.

These and other aspects and features, and combinations of them, may be expressed as apparatus, methods, systems, and in other ways.

Other features and advantages will be apparent from the description and the claims.

### Description

Figures 1A through 1H and 13A through 13D show delivery of a heart valve support.
Figures 2A through 2D are perspective views of a heart valve support.
Figure 2E is a plan view of a recurved hook.
Figure 3 is a section side view of a heart valve support.
Figures 4A through 4C are side and detailed views of a delivery tool and heart valve support.
Figure 5 is a side view of a delivery tool.
Figures 6A and 6B are sectional side views of a catheter delivery tool.
Figures 7A through 8I show delivery of a heart valve support.
Figures 9A, 9R, 9T and 9U are plan views of a heart tissue support.
Figures 9B, 9P, and 9S are perspective views of fragments of heart tissue supports.
Figures 9C through 9E, 9G and 9H are side views of burr hooks.
Figure 9F is a schematic view of a heart tissue support attached to annular tissue.
Figures 9I through 9M and 90 are close-up views of portions of heart tissue support surfaces.
Figures 9N and 9Q are views of a heart tissue support and a delivery tool..
Figures 10A and 10B are side views of a delivery tool, and a cross-section of a sheath.
Figures 10C and 10D are cross-sectional views of a delivery tool and sheath.
Figure 11A is a perspective view of a delivery tool in a heart annulus.
Figure 11B is a view of the operator end of a delivery tool.
Figures 11C and 11F are close-up views of a heart tissue support attached to a delivery tool.
Figures 11D and 11E are close-up views of a portion of a heart tissue support attached to annular tissue.
Figures 12A and 12B are views of a core of a delivery tool.
Figure 12C is a perspective view of a core of a delivery tool.
Figures 14A through 14D are perspective views of portions of supports.
Figure 15 is a perspective view of an anchor.
Figure 16 is a perspective view of a gripper.
Figure 17 is a side view of a gripper.
Figure 18 is a perspective view of a covering.
Figure 19 is a cutaway perspective view of a support.
Figure 20 is a perspective view of a support.
Figure 21 is an enlarged perspective view of a portion of a support.
Figures 22 through 25 are top views of a gripper.
Figures 26 and 27 are top views of a gripper.
Figures 28, 29, 30, and 31 are a perspective view, a sectional perspective view, a perspective view, and a sectional perspective view, respectively, of a support.
Figure 32 is a top view of a gripper.
Figures 33 through 35 are a top view, a top view, and a perspective view of a support on a hypothetical insertion tool.
Figures 36 through 39 are side views of an insertion tool.
Figure 40 is a side view of an insertion tool.
Figure 41 is a perspective view of an insertion tool.
Figures 42 and 43 are side views of an insertion tool.
Figure 44 is a side view of an insertion tool.
Figures 45 and 46 are perspective and enlarged perspective views of a portion of a support.
Figures 47 and 52 are perspective views of a support.
Figures 48 and 53 are perspective and side views of anchors.
Figure 49 is a perspective view of a coil.
Figure 50 is a perspective view of a resilient ring.
Figure 51 is a perspective view of a ring and coil assembly.
Figures 54 and 55 are a perspective and side view of an interlock.

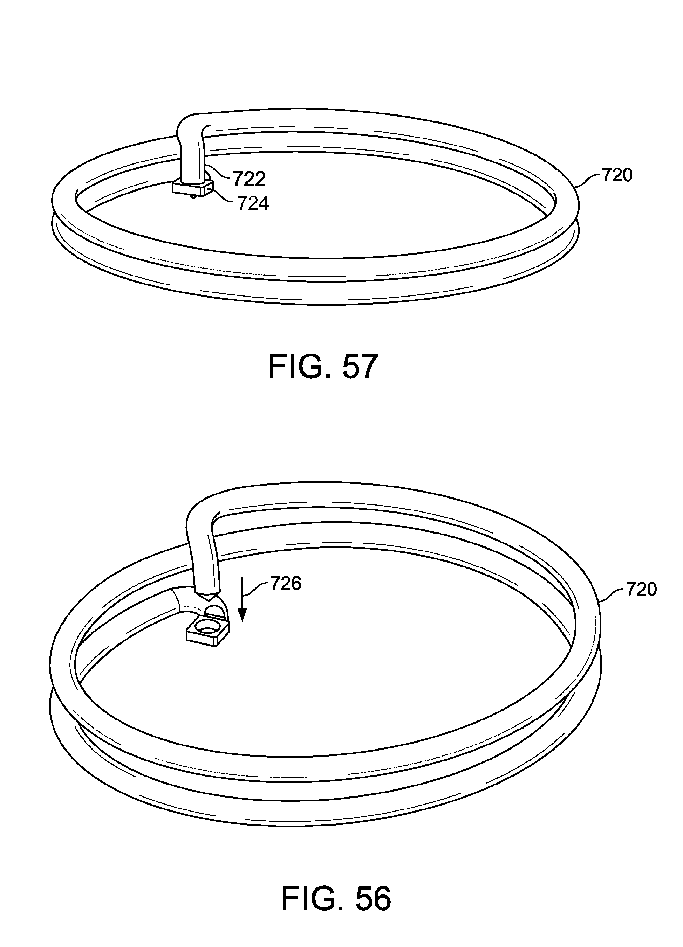

Figures 56 and 57 are perspective views of an interlock.
Figures 58 and 59 are perspective views of a support.
Figures 60A and 60B are views of a portion of a support.
Figures 61A and 61B are top views of a support.

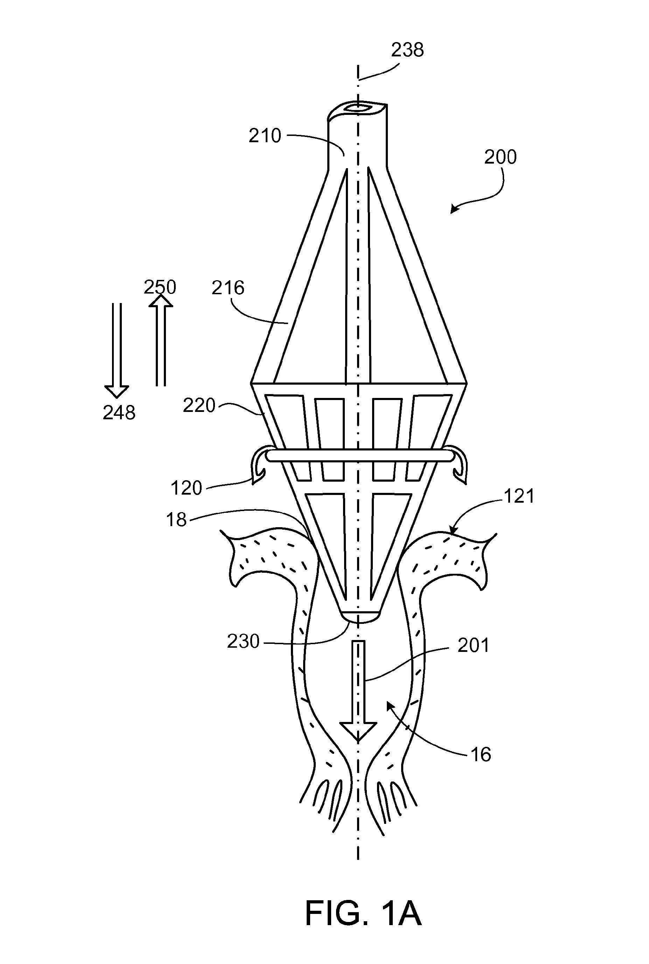

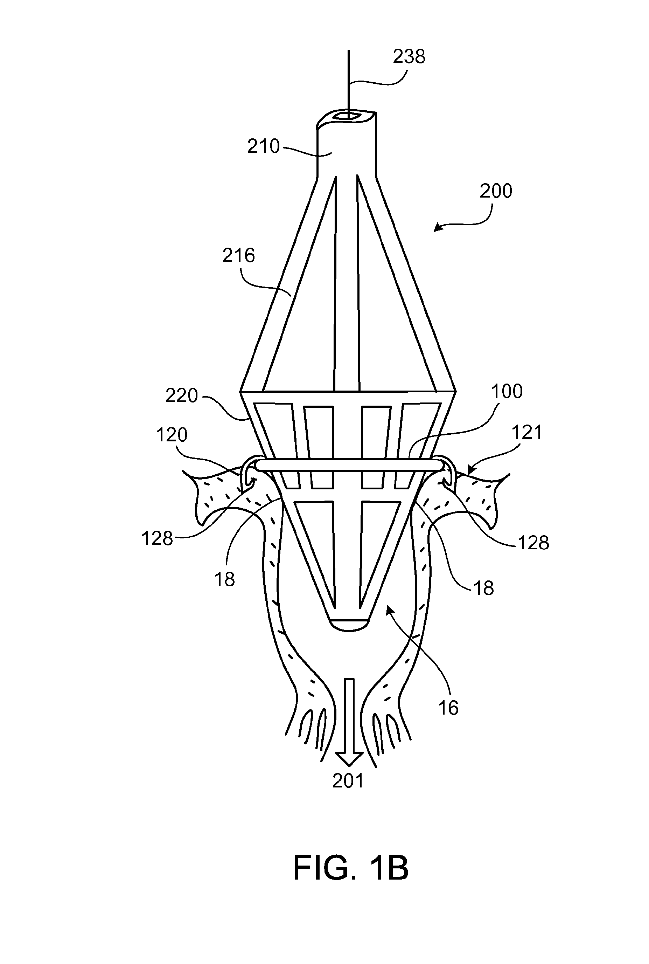

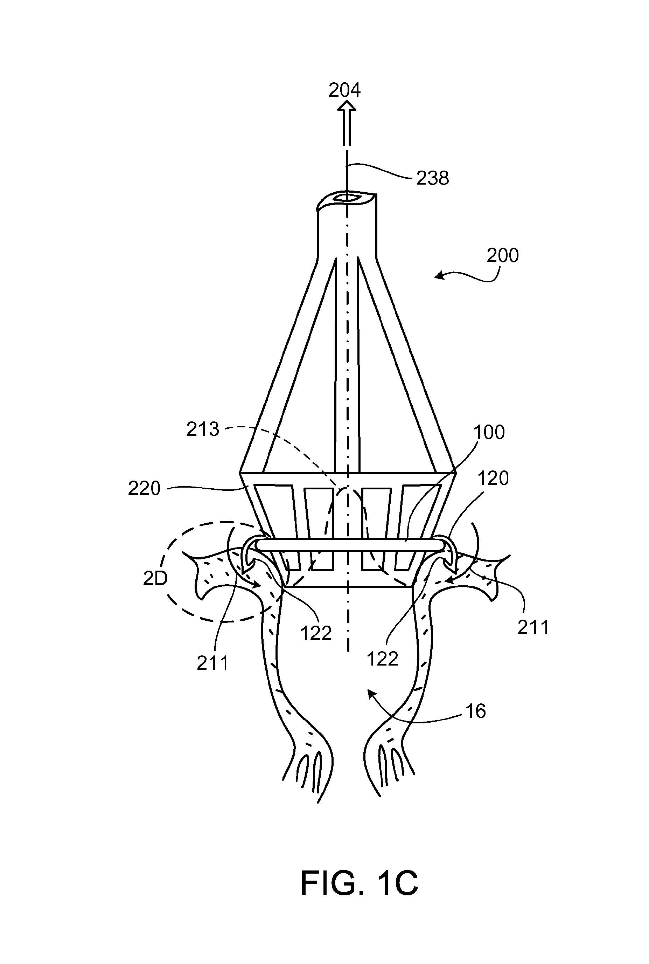

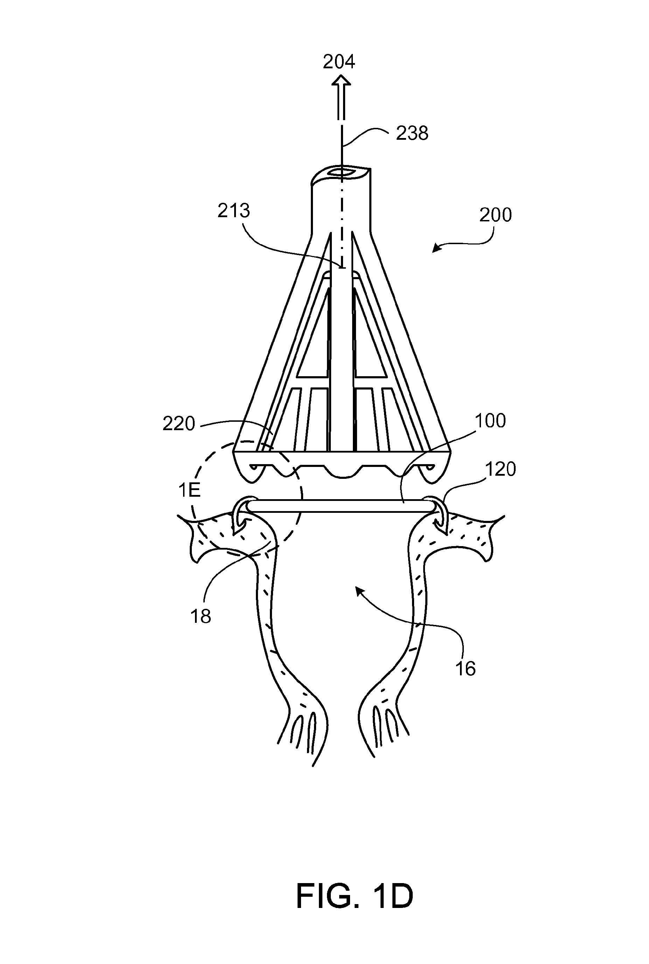

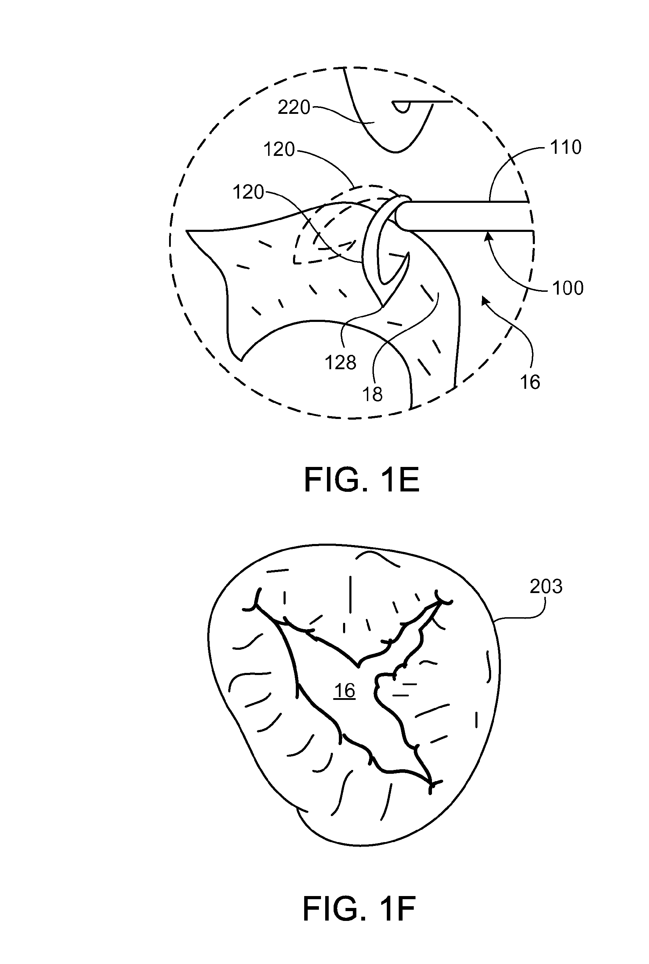

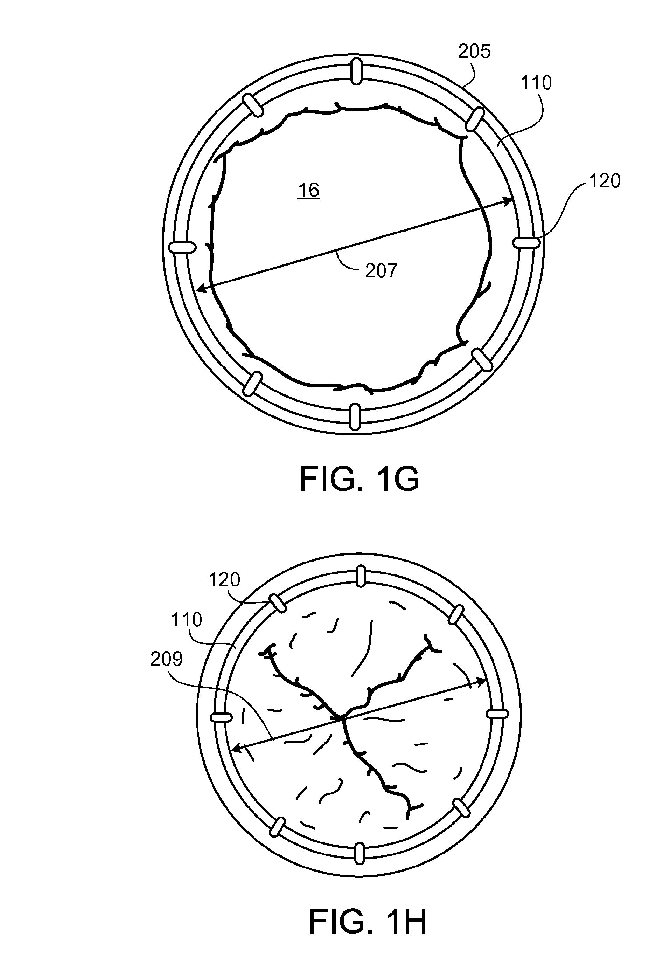

As shown in the examples of figures 1A through 1G distortion of an annulus 18 of a heart valve 16 can be corrected simply and quickly by the following steps:
A. Push 201 (figure 1A) a conical head-end basket 220 of a delivery tool 200 into the valve to force the distorted annulus (203, figure IF) to conform to a desired configuration (e.g., a circle 205, figure 1G) and to a size that is larger (e.g., in diameter 207) than a desired final diameter 209 of the annulus (figure 1H). (The tool including the basket are shown in side view and the valve and annulus are shown in sectional side view.)
B. Continue to push 201 the delivery tool to drive an expanded heart valve support 100 (which has the desired configuration and the larger size and is temporarily held in its expanded configuration on the basket of the tool) towards the annulus to seat multiple (for example, eight, as shown, or a larger or smaller number of) recurved hooks 120 located along the periphery of the support simultaneously into the valve tissue at multiple locations along the periphery 121 of the annulus (figure 1B).
C. After the hooks are seated, pull 204 (figure 1C) on and evert the tip 230 of the head end basket from the inside to cause the support to roll so that the tips 122 of the hooks rotate 211 and embed themselves more securely into the annulus tissue (figure 1C).
D. After the hooks are further embedded, continue to pull 204 (figure 1D) on the inside 213 of the tip of the head-end basket to break the tool away from the support (figure 1E), allowing the support to contract to its final size and shape 215 (figure 1H) and leaving the support permanently in place to maintain the annulus in the desired final configuration and size.

The entire procedure can be performed in less than a minute in many cases. By temporarily forcing the annulus of the valve to expand to the desired circular shape, it is possible to attach the support quickly, easily, and somewhat automatically by forcing multiple gripping elements into the tissue at one time. Hooks are used in this example, although other types of gripping elements may be used as well. The physician avoids the time consuming steps of having to attach individual sutures or clips one at a time along the periphery of a distorted annulus and then cinch them together to reform the supported annulus to a desired shape and size. Thus, the physician does not even need to be able to see the annulus clearly (or at all). Once attached, when the tool is removed, the support automatically springs back to its final shape and size.

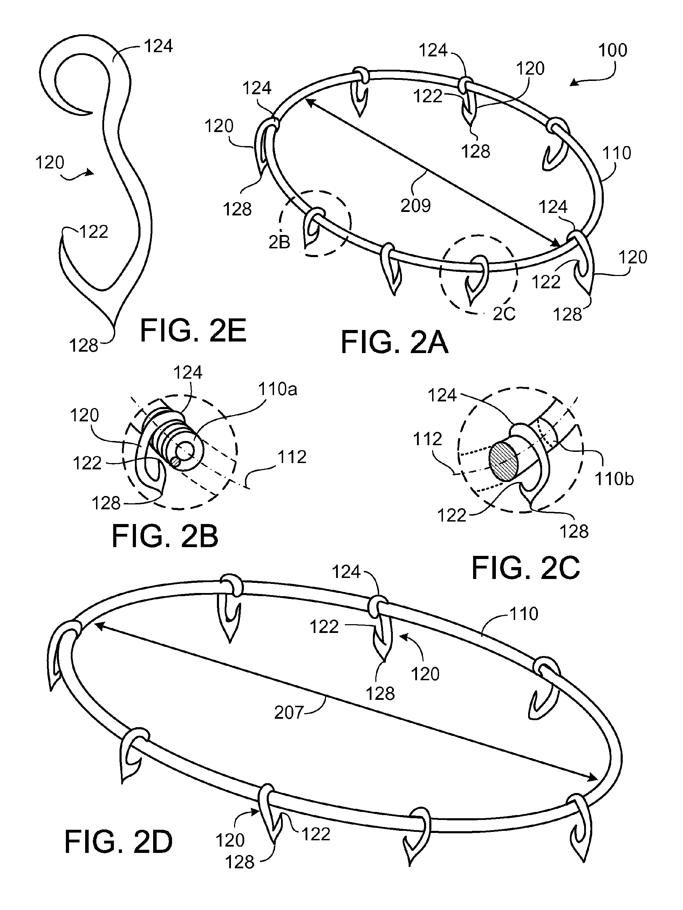

As shown in figures 2A and 2D, in some implementations the support includes a circular ring body 110 that bears the hooks 120. The body 110 can be expanded from (a) a minimal-diameter long-term configuration (figure 2A) to which it conforms after it has been attached to the annulus to (b) an expanded delivery configuration (figure 2D) to which it conforms when it is held on the head-end basket of the tool and while it is being attached in the steps shown in figures 1A, 1B, and 1C. The long-term configuration is normally circular and has the diameter of a healthy annulus for a particular patient. When attached, the support maintains the healthy configuration of the annulus so that the valve will work properly.

In some examples, the body 110 has the same (e.g., circular) shape but different diameters in the delivery configuration and the long-term configuration. The body is constructed of a material or in a manner that biases the body to contract to the long-term configuration. For example, all or portions of the body 110 may be formed as a helical spring 110a such as a continuous helical spring connected at opposite ends to form a circular body or one or more interconnected helical spring segments (figure 2B). In some examples, the support body 110b may be a band of shape memory material such as Nitinol or a biologically compatible elastomer (or other material) that will return to the long-term configuration after being expanded to the delivery configuration (figure 2C).

The hooks 120 may number as few as three or as many as ten or twenty or more and may be arranged at equal intervals along the body or at unequal intervals as needed to make the body easy and quick to deliver, permanent in its placement, and effective in correcting distortion of the valve annulus. The hooks are configured and together mounted along the circular outer periphery so that they can be inserted simultaneously into the tissue along the periphery of the annulus and then firmly embedded when the tool is pulled away and the basket is everted.

In some examples, a portion or portions of the support body may not have hooks attached if, for example, a segment of the valve annulus shares a boundary with sensitive or delicate tissue, such as the atrioventricular (AV) node of the heart. This tissue should not be pierced by the hooks. A support body configured to avoid interfering with the AV node could have a section having no hooks attached or otherwise covered or protected to prevent penetration by hooks into the AV node. The support body should be positioned so that this special section of the support body is adjacent the sensitive or delicate tissue as the support body is put into place. The support body may have more than one special section lacking hooks, so that the operator has more than one option when placing the support body near the sensitive tissue. In some examples, the support body could have a section removed entirely, and would be shaped somewhat like the letter "C" instead of a complete ring. In any of these examples, the procedure described above could have an additional step preceding step A, in which the operator rotates the delivery head to position the section having no hooks or to position the gap in the support body to be adjacent to the sensitive tissue at the moment when the hooks are to be embedded in the other tissue. The support body may have radiopaque marks to help the operator view the positioning.

For this reason, as shown in figure 2E, for example, each of the hooks has two pointed features. One pointed feature is a sharp free end 122 pointing away from the valve leaflets during delivery. The other pointed feature is a barb128 formed at a bend between the sharp free end 122 and an opposite connection end 124 where the hook is attached, e.g., welded or glued, to the body 110. The barb points toward the valve leaflets during delivery. Thus, the barb is arranged to penetrate the tissue when the tool is pushed toward the valve, and the sharp free end is arranged to embed the hook into the tissue when the tool is pulled away from the valve.

Each hook 120 can be formed of biologically compatible materials such as platinum, gold, palladium, rhenium, tantalum, tungsten, molybdenum, nickel, cobalt, stainless steel, Nitinol, and alloys, polymers, or other materials. During delivery the barbs of the hooks are together (and more or less simultaneously) forced into the tissue at a series of locations around the outer periphery of the temporarily expanded annulus. In a later step, the sharp free ends are forced to rotate somewhat away from the leaflets for secure (e.g., permanent) attachment.

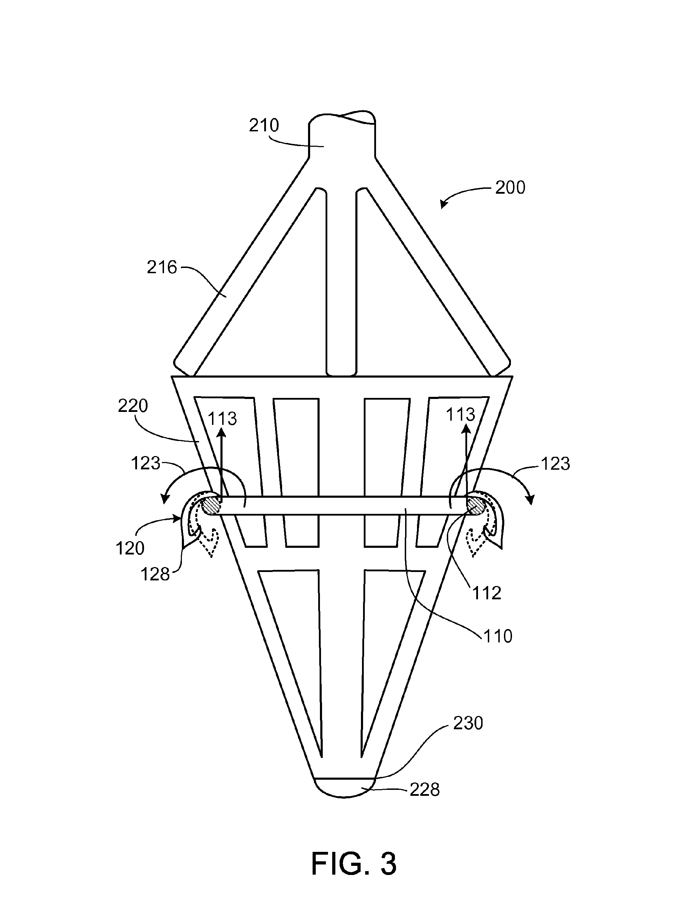

To cause the hooks to rotate during delivery, the hooks 120 are attached permanently to the support body 110 and the support body can be rolled 123 (figure 3) about a central annular axis 112 of the support body, as indicated. One way to cause the rolling of the support body and the associated rotation of the hooks is to enable the body to change its configuration by rotation of the entire body about an axis represented by the central circular axis 123, much as a rubber o-ring can be rolled about its central circular axis. The reconfiguration of the body to cause the rotation of the hooks can be achieved in other ways.

In some examples, applying an axial force (arrows 113) to the inner peripheral edge of the ring (we sometimes refer to the support broadly as a ring) will cause the ring to tend to roll and the hooks to embed themselves in the annulus as intended. By appropriately mounting the inner periphery of the ring on the outer periphery of the delivery tool, the axial force 113 can be applied by pulling the tool away from the leaflets of the valve, as explained earlier.

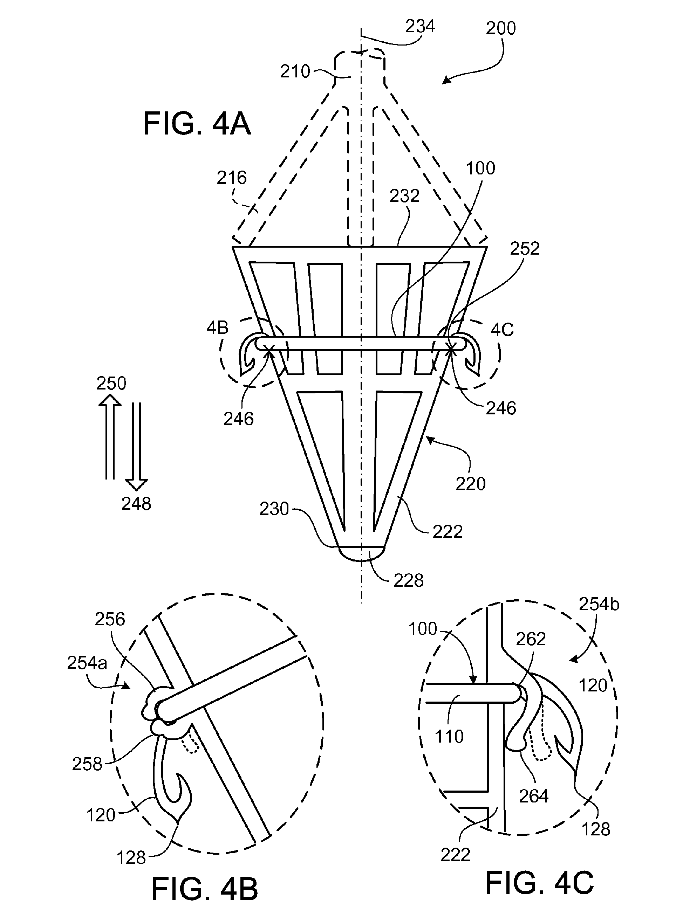

For delivery to the valve annulus, the valve support 100 is first expanded to its delivery configuration and temporarily mounted on a delivery head 220 of the tool 200 (figure 4A). The support could be expanded enough in its temporary mounting on the tool and mounted far enough away from the tip along the conical head-end basket so that when the head-end basket of the tool is pushed against the annulus to force it to expand to the size and shape of the expanded support, the annulus first has reached a circular, non-distorted shape before the support hook barbs begin to penetrate the tissue. The tapered profile of the head-end basket of the delivery tool allows the tool to accommodate supports of various sizes. In some implementations, different shapes and sizes of baskets could be used for supports of different sizes.

The heart valve support 100 is held in place on the delivery head 220 using one or more releasable connections 246. The connections 246 are arranged to translate forces from the tool 200 to the support 100 in each of two opposite directions 248 and 250, toward or away from the leaflets of the valve. When the support has been embedded in the annulus and the tool is pulled in the direction 250 to release it from the support, the force on the connections 246 exceeds a predetermined threshold, and the connections break, releasing the tool from the support at the end of the delivery process. The connections 246 may be, in some examples, breakable sutures 252 (figure 4A), or some other breakaway structure such as clips or adhesive or a structure that can be manipulated from the tool by unscrewing or other manipulation.

In some examples, the connections 246 include retainers that can take, e.g., the configurations shown as 254a or 254b (figures 4B & 4C, respectively). In the example shown in figure 4B, the retaining element 254a has one rigid finger 256 to translate forces from the tool 200 to the support 100 when the tool is moved in direction 248 while the support is attached to the tool and being pushed into the heart tissue. A second deformable finger 258 aids in maintaining the connection between the support 100 and the tool 200 when the tool is moved in direction 250 and is deformable (dashed lines) to release the valve support 100 from the tool 200 when the force in direction 250 relative to the embedded support exceeds a predetermined threshold.

In the example shown in figure 4C, the retaining element 254b includes a finger 260 having a crook 262 to receive the support 100 and to translate forces from the tool 200 to the support 100 when the tool is moved in direction 248. The finger has a resiliently deformable tip 264 that is biased towards the tapered body 222 and helps to maintain the connection between the support 100 and the tool 200 and is deformable (shown in hidden lines) to release the valve support 100 from the tool 200 when the tool is moved in the second axial direction 250 against an embedded support and the force exceeds a predetermined threshold.

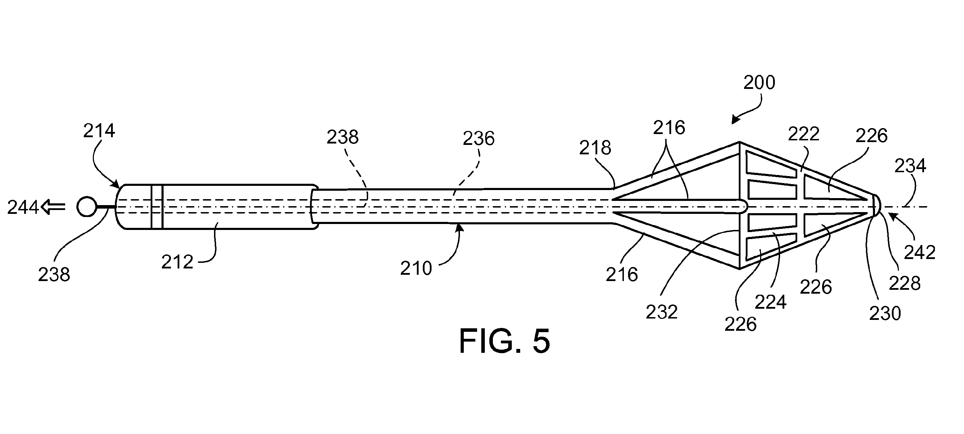

As shown in figure 5, in an example of a tool 200 that can be used for delivery of the support during open heart surgery, a basket 220 is connected at its broad end to a set of stiff wires or other rigid projections 216 that are splayed from a long shaft 210 having a handle 212 at the operator's end 214. Thus the projections 216 connect the shaft 210 to the basket 220 and transfer pulling or pushing force between the shaft and the basket (and in turn to the support).

The example of the basket shown in figure 5 includes a tapered body 222 having a network of interconnected struts 224 defining an array of openings 226 together forming a tapered semi-rigid net. In this example, the basket (which we also sometimes refer to as a delivery head) 220 has a rounded tip 228. The head 222 tapers radially outwardly with distance along a longitudinal axis 234 of the head 220 from the tip 228 towards the operator. The broad end 232 of the tapered body 222 is firmly attached to the projections 216, which taper in the opposite direction from the taper of the basket. The net formed by the struts 224 is semi-rigid in the sense of having enough stiffness to permit the operator to force the valve support against the heart tissue to cause the barbs of the hooks of the support to penetrate the tissue, and enough flexibility to permit the head-end basket to be everted when the operator pulls on the handle to evert the basket and release the support from the basket.

In some implementations, the shaft 210 defines a lumen 236 extending between the heart valve end 218 of the shaft 210 and the handle 212. A wire 238 is arranged to move freely back and forth within the lumen 236. The wire 238 has one end 240 that extends from the handle 212 and an opposite end 242 that is connected to the inside of tip 228. The wire 238 can be pulled (arrow 244) to cause the delivery head 220 to collapse (hidden lines) and evert radially inwardly starting at the tip 228 as mentioned earlier.

Returning to a more detailed discussion of figures 1A through 1E, the operator begins the delivery of the support by pushing the tapered end 230 of the head basket 220 into the valve 16 (e.g., the tricuspid valve) to cause the valve leaflets 14 to spread apart. The tip 230 is small and rounded which makes it relatively easy to insert into the valve without requiring very precise guidance. Because the head-end basket is tapered, by continuing to push, the operator can cause the annulus 18 of the tricuspid valve 16 to expand in size and to conform to a desired shape, typically circular. During insertion, because of its symmetrical taper, the head-end basket tends to be self-centering. The taper of the basket 220 translates the insertion force in direction 248 into a radial force that causes the annulus 18 to expand and temporarily assume a desired shape (and a larger than final diameter).

As the operator continues to push on the tool, the ring of barbs of the hooks touch and then enter (pierce) the heart tissue along a ring of insertion locations defined by the outer periphery of the annulus, and the sharp free ends of the hooks enter and seat themselves within the tissue, much like fish hooks. Depending on how the operator guides the tool, the basket can be oriented during insertion so that essentially all of the hooks enter the tissue at the same time. Or the tool could be tilted during insertion so that hooks on one side of the support enter the tissue first and then the tool delivery angle could be shifted to force other hooks into the tissue in sequence.

Generally, when the number of hooks is relatively small (say between 6 and 20, comparable to the number of sutures that the physician would use in conventional stitching of a ring onto an annulus), it is desirable to assure that all of the hooks penetrate the tissue and are seated properly.

Once the hooks are embedded in the tissue, the operator pulls on the near end 240 of wire 238 to cause the basket 220 to collapse, evert, and be drawn out of the valve 16. Eventually, the everted portion of the basket reaches the valve support 100. By further tugging, the operator causes the body 110 of the support 100 to roll about its central axis (as in the o-ring example mentioned earlier) which causes the hooks 120 to embed more firmly in the tissue of the annulus 18 of the valve 16.

Using a final tug, the operator breaks the connections between the tool 200 and the valve support 100 and removes the tool 200, leaving the valve support 100 in place. As the everting basket 220 passes the points of connection 246, the retaining forces exerted by the embedded hooks 120 of the support body 110, acting in direction 248, exceed the forces exerted by the withdrawing basket 220 on the support body 110 (through the connections 246), acting in direction 250, thereby causing the connections 246 to break or release, in turn releasing the support 100.

The tool 200 is then withdrawn, allowing the valve support 100, along with the annulus 18, to contract to the long-run configuration.

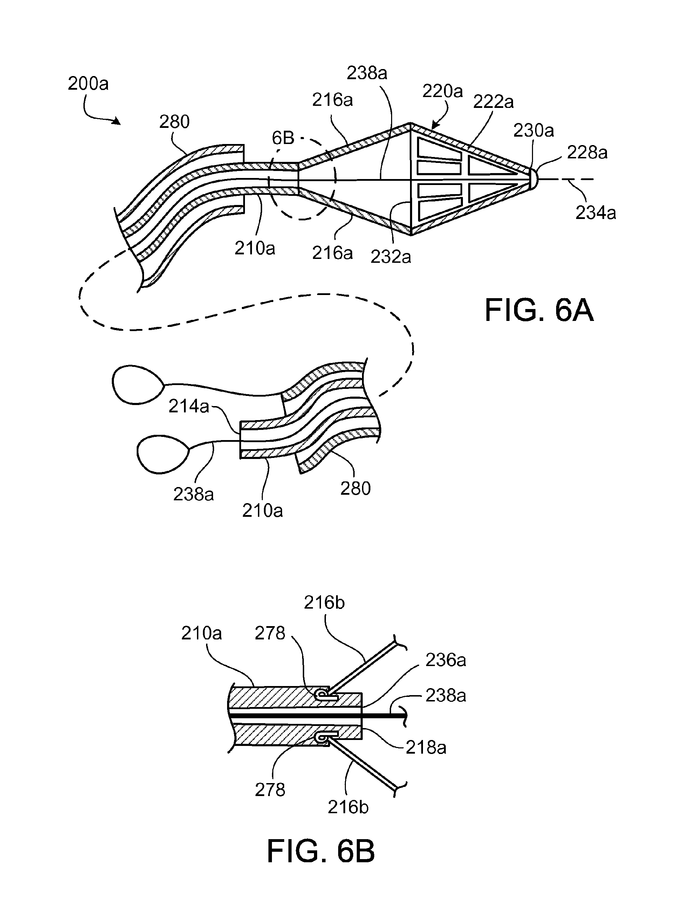

In implementations useful for delivery of the support percutaneously, as shown in figure 6A, the delivery head 220a can be made, for example, from a shape memory alloy, such as Nitinol, which will allow the body 222a to be collapsed radially toward the longitudinal axis 234a prior to and during delivery of the head from a percutaneous entry point (say the femoral vein) into the heart. The delivery head 220a is biased towards the expanded, tapered configuration shown in figure 6A. Thus, the delivery head 220a, in the form of a tapered semi-rigid net, is connected to a catheter shaft 210a through projections 216a that splay radially outwardly from the catheter shaft 210a and taper in a direction opposite the taper of the delivery head 220a. (Here we refer to the delivery head as the head-end basket.)

The projections 216a are resiliently mounted to the catheter shaft 210a and are biased towards the expanded, tapered orientation shown, for example, by spring biased projections 216b shown in figure 6B. The projections 216a include springs 278, e.g., torsion springs (as shown), mounted to the catheter shaft 210a and forming a resilient connection.

A wire 238a slides within a lumen 236a of the shaft 210a in a manner similar to the one described earlier.

The tool 200a also includes a sheath 280 in which the catheter shaft 210a can slide during placement of the support. The sheath 280, the catheter shaft 210a, and the wire 238a are all flexible along their lengths to allow the tool 200a to be deflected and articulated along a blood vessel to reach the heart and to permit manipulation of the delivery head once inside the heart.

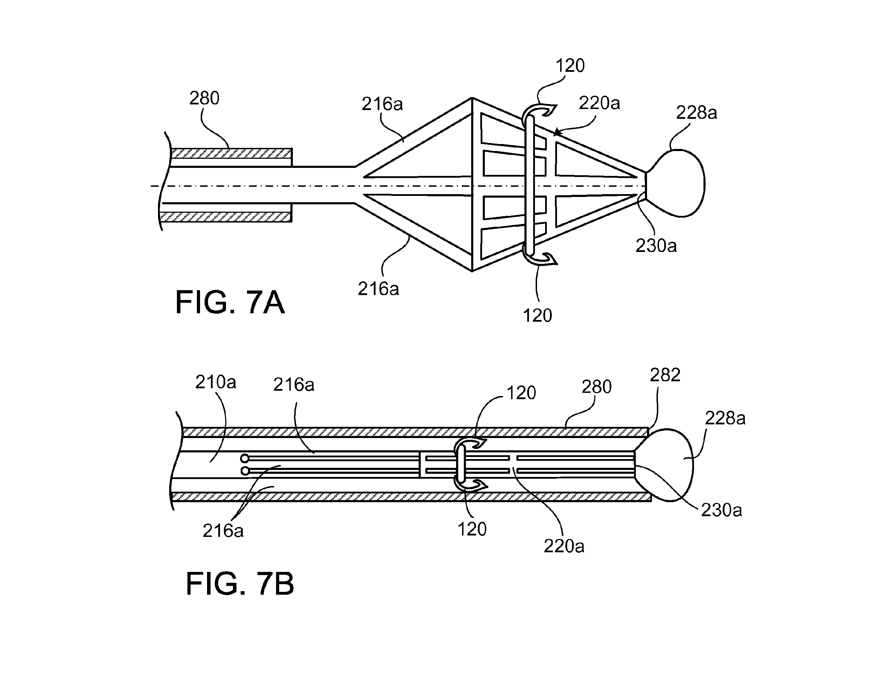

To deliver the support percutaneously, as shown in figure 7A, when the delivery head is prepared for use, the sheath 280 is retracted beyond the projections 216a, allowing the delivery head 220a to expand. The valve support 100 is then expanded to the delivery configuration (either by hand or using an expansion tool) and mounted on the tapered body 222a. The valve support 100 is connected to the delivery head 220a using releasable connections, e.g., breakable sutures and/or retaining elements (as described earlier).

The sheath 280 is then moved along the catheter shaft 210a towards the delivery head 220, causing the projections 216a and the delivery head 220a to contract radially inwardly to fit within the sheath 280, as shown in figure 7B. In the contracted configuration, the tip 228a of the delivery head 220a bears against the end 282 of the sheath 280. The rounded tip 228a may, e.g., provide easier delivery and maneuverability in navigating the blood vessels to reach the heart.

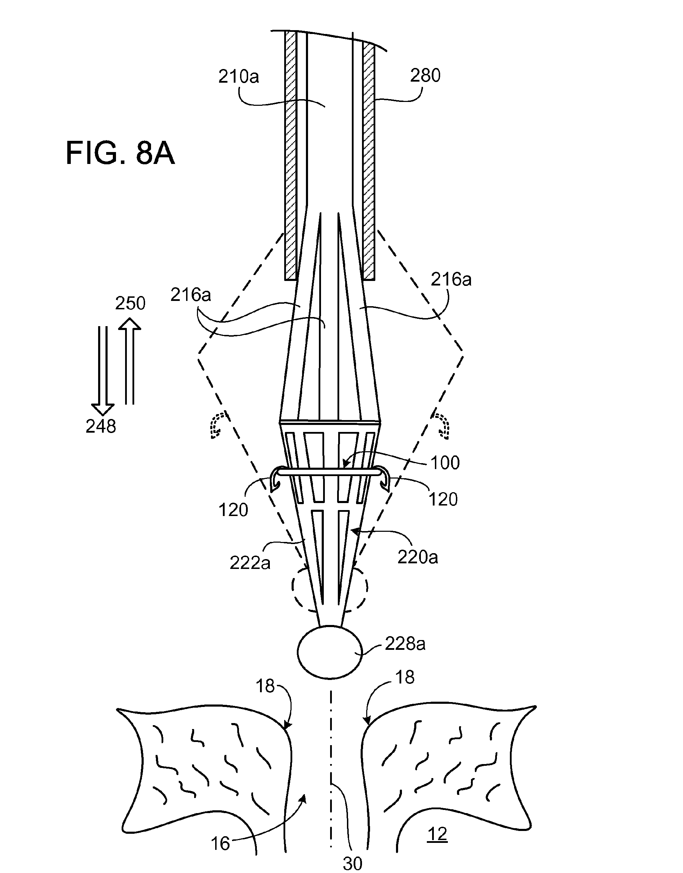

To deliver the support to the valve annulus, the end 230 of the tool 200a is fed percutaneously through blood vessels and into the right atrium 24 (figure 8A). The sheath 280 is then retracted, exposing the valve support 100 and allowing the projections 216a, the delivery head 220a, and the support 100 to expand, as shown in figure 8A.

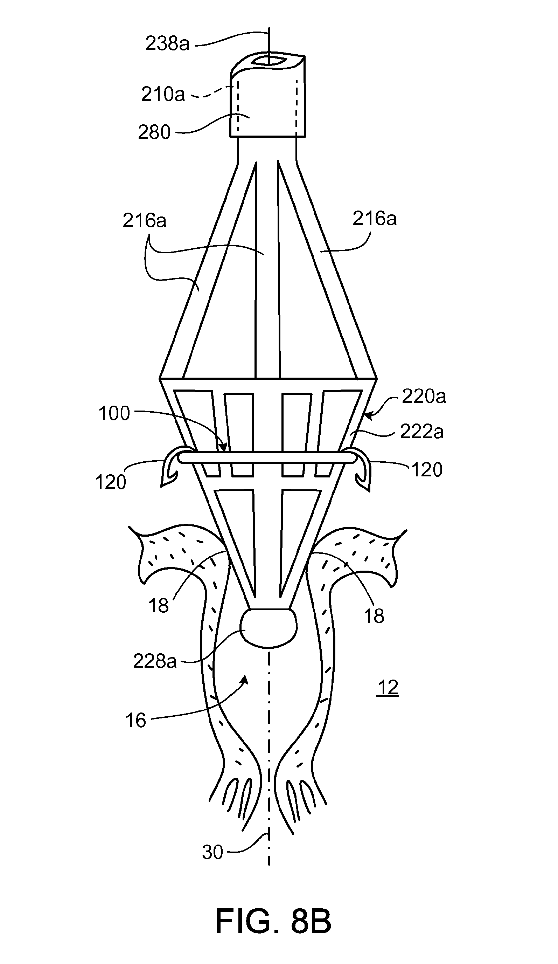

In steps that are somewhat similar to the open heart placement of the support, the catheter shaft 210a is then advanced, e.g., under image guidance, in the direction 248a along an axis 30 of the annulus 18. The operator forces the distal end 230a of the self-centering delivery head 220a into the valve 16 (figure 8B) using feel or image guidance, without actually seeing the valve 16.

Once the tip is in the valve 16, the operator pushes on the end 214a of the catheter shaft 210a to force the tool further into the valve 16. This causes the tapered body 222a of the delivery head 220a to restore the shape of the annulus 18 to a circle or other desired shape (such as the distinctive "D" shape of a healthy mitral valve). The tool 200a tends to be self-centering because of its shape. The net-like construction of the delivery head 220a (and the head used in open heart surgery, also) allows blood to flow through the valve even while the delivery head 220a is inserted.

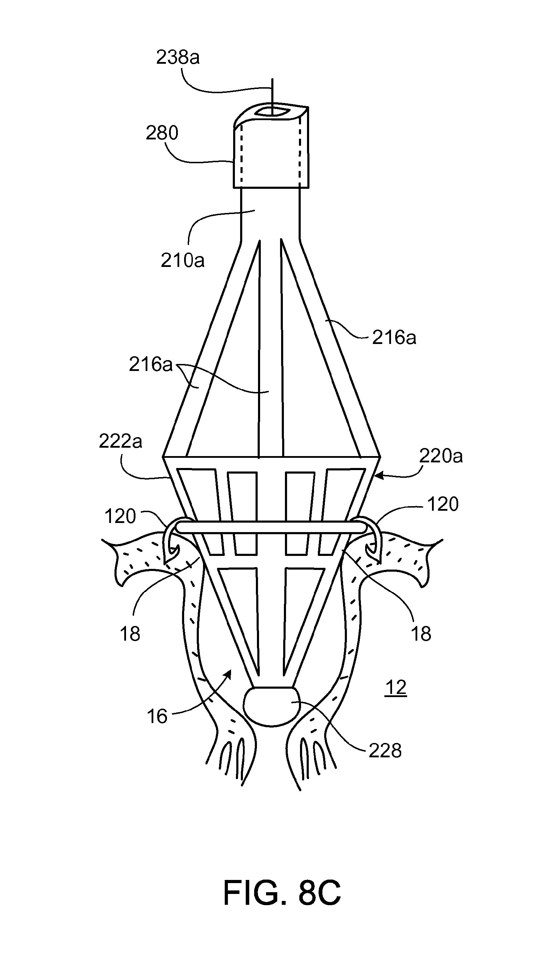

As tool 200a reaches the position at which the support hooks touch the annulus, by giving an additional push, the operator drives the hooks 120 of the valve support 100 together into all of the annular locations at which it is to be attached, as shown in figure 8C. In some examples, it may be possible for the operator to tilt the delivery head deliberately to cause some of the hooks to penetrate the tissue before other hooks. The configuration of the valve support 100 and the tool 200a and the manner of temporary attachment of the support 100 to the tool 200a tend to assure that the hooks 120 will penetrate the valve 16 at the correct positions, just along the outer edge of the annulus 18.

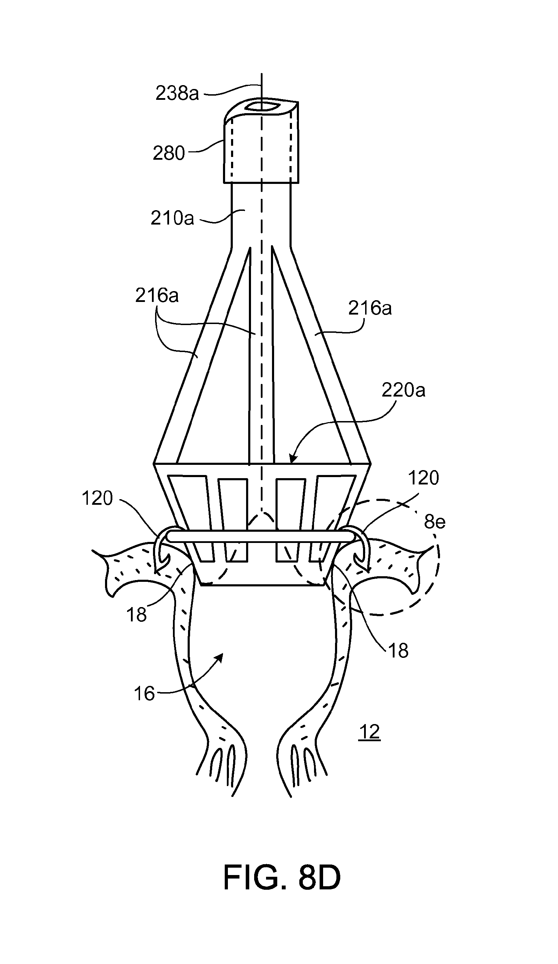

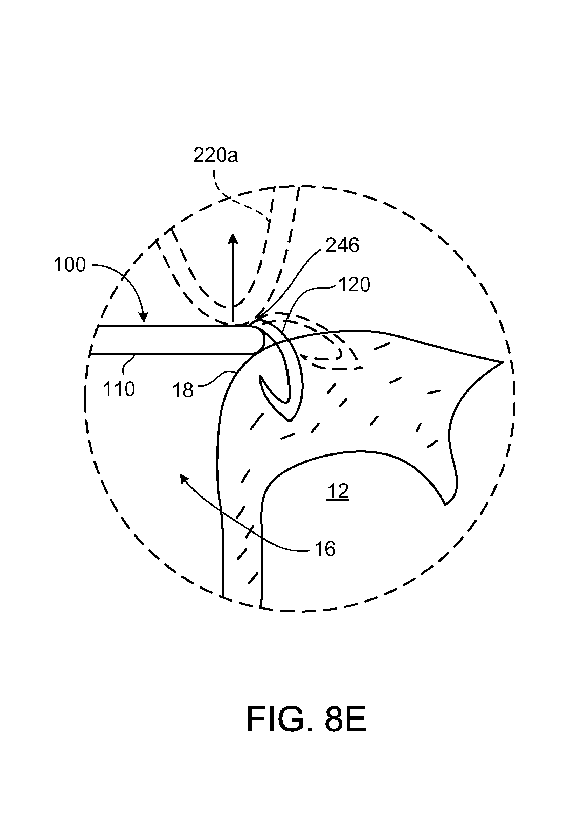

Once the valve support 100 has been attached to the valve 16, the operator pulls on the proximal end 240a causing the delivery head 220a to evert (hidden dashed lines) and be drawn out of the valve 16 (shown in figure 8D). Eventually the everted portion of the tool 200a reaches the valve support 100. By further tugging, the operator causes the torus of the support 100 to roll around its periphery which jams the free ends of the hooks 120 securely into the annulus 18 of the valve 16, as illustrated in figure 8E, seating the support permanently and permitting later growth of tissue around the support 100. The depth and radial extent of each of the placed hooks 120 can be essentially the same as a conventional suture so that their placement is likely to be as effective and familiar to the operator and others as conventional sutures.

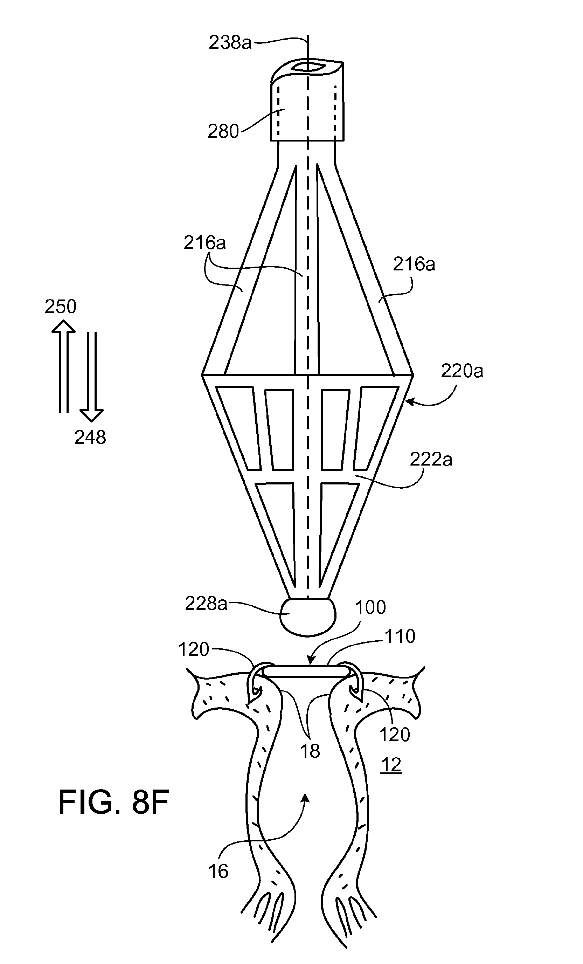

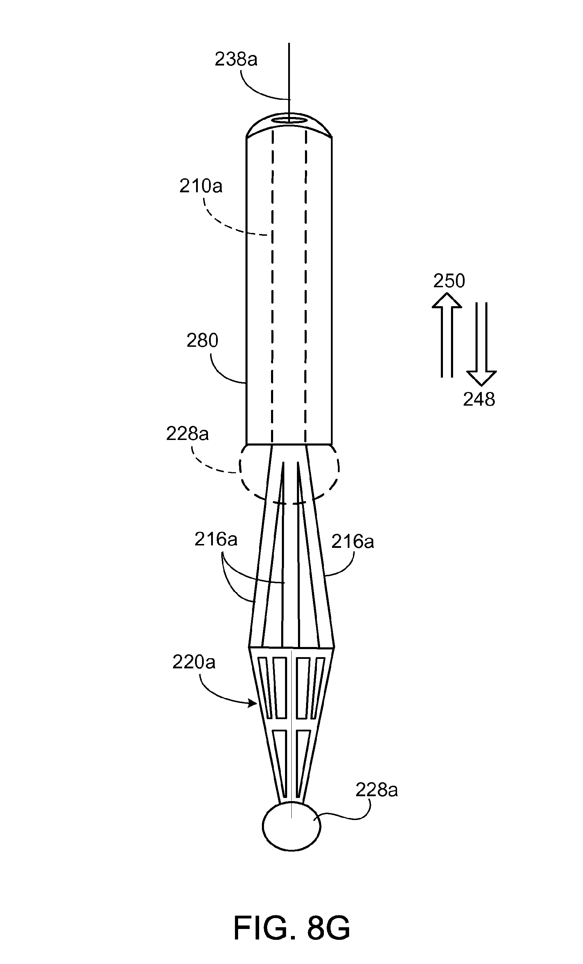

Using a final tug, the operator breaks the connections 246 between the tool 200a and the valve support 100 and retracts the catheter shaft 210, leaving the support 100 in place. The catheter shaft 210 is retracted to a position beyond the valve annulus 18 and the wire is advanced in the first direction allowing the delivery head 220a to assume its original tapered shape (figure 8F). The catheter shaft 210a is then retracted into the sheath 280 (figure 8G), and the tool 200a is withdrawn.

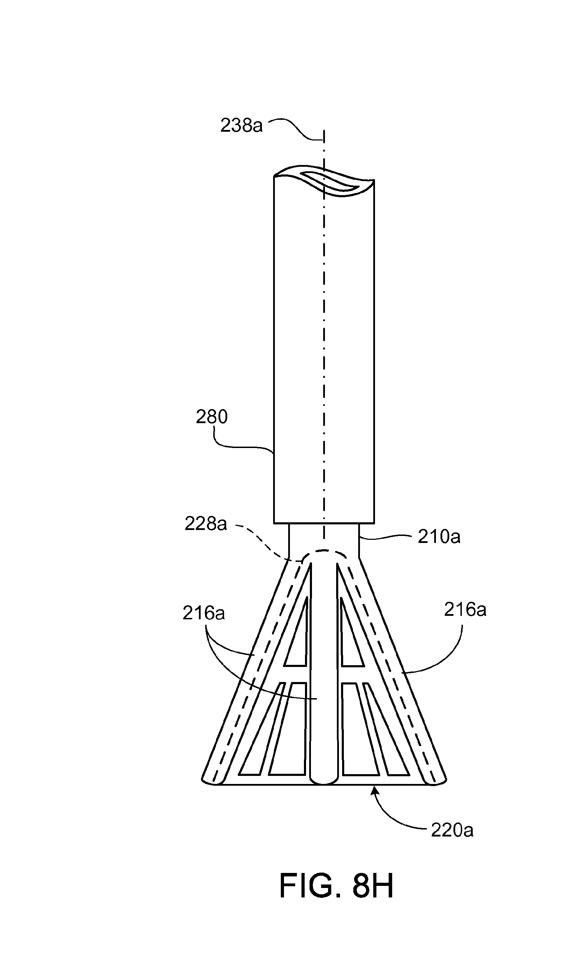

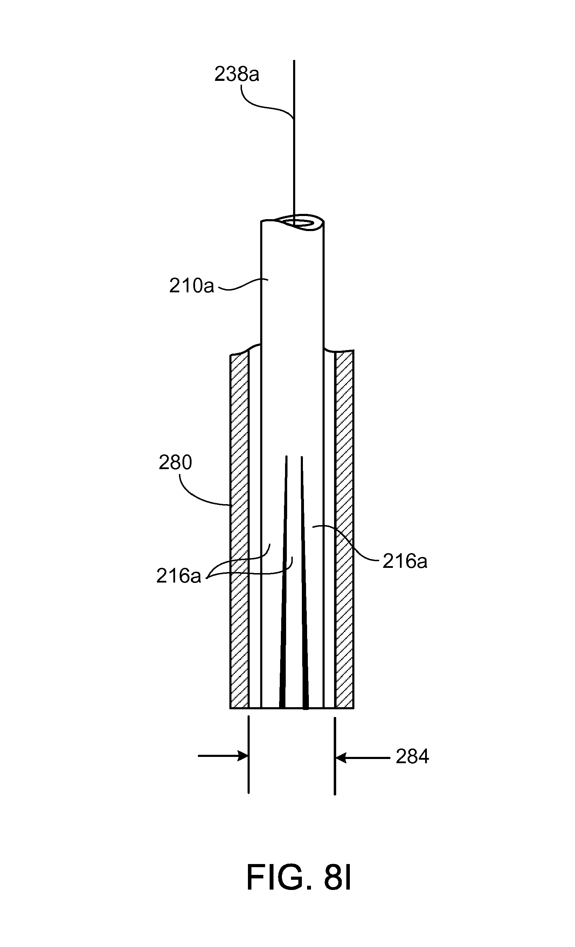

In some examples, as shown in figures 8H and 8I, the tip 228a of the tool 200a, when everted, has a compressed dimension that is smaller than an internal diameter 284 of the sheath 280, permitting the catheter shaft 210a to be retracted directly into the sheath 280 after deployment, with the everted tip held within the collapsed delivery basket, as shown in figure 8I.

With the tool 200a withdrawn, the valve support 100 contracts, reshaping the annulus 18 such that the valve leaflets 14 coapt to prevent a backflow of blood during systole.

Other implementations are within the scope of the claims.

For example, distortion of either the tricuspid valve or mitral valve can be corrected. For tricuspid valve repair, the hooks can be arranged around only about three-quarters of the support and therefore the annulus. During the placement procedure, the operator will rotate the support to position the portion of the support having hooks. For mitral valve repair, the hooks can cover the entire periphery of the annulus. In this scenario, the hooks are arranged around the full circumference of the support. Alternatively, the hooks can cover only the posterior section of the annulus of the mitral valve. In this scenario, the hooks can be arranged around two-thirds of the support. Similarly to the tricuspid valve example, the operator will position the portion of the support having hooks against the posterior section of the mitral valve annulus. Further, for mitral valve repair, a back-up valve can be provided as part of the delivery tool to maintain heart function during the delivery procedure. Materials other than shape memory materials may be used as the material for the support body, and other ways can be used to force the support back to a desired size following expansion, including, for example, cross-bars that span the opening of the support.

In addition, the left atrial appendage of the heart can be closed by a similar technique. For example, the tool can be pushed into an opening of an atrial appendage causing the opening to assume a predetermined shape. The tool can continue to be pushed in order to embed the hooks of the expanded support into the periphery of the opening of the appendage. The tool can then be withdrawn, releasing the support, and allowing the support to contract. The support can have a relatively small contracted diameter such that, when the tool is withdrawn, releasing the support, the support can contract to a relatively small size, effectively closing off the appendage.

In addition to the open heart and percutaneous deployment procedures, the valve support can also be deployed through the chest.

The head-end of the tool need not be a basket, but can take any form, mechanical arrangement, and strength that enables the valve annulus to be forced open to a shape that corresponds to the shape of the support. The basket can be made of a wide variety of materials. The basket can be held and pushed using a wide variety of structural mechanisms that permit both pushing and pulling on the support both to seat and embed the support in the annulus tissue and disconnect the support from the tool.

The tool need not be conical.

The support could take a wide variety of configurations, sizes, and shapes, and be made of a wide variety of materials.

The hooks could be replaced by other devices to seat and embed the support using the pushing force of the tool.

The hooks of the support need not be embedded directly in the annulus but might be embedded in adjacent tissue, for example.

The support could take other forms and be attached in other ways.

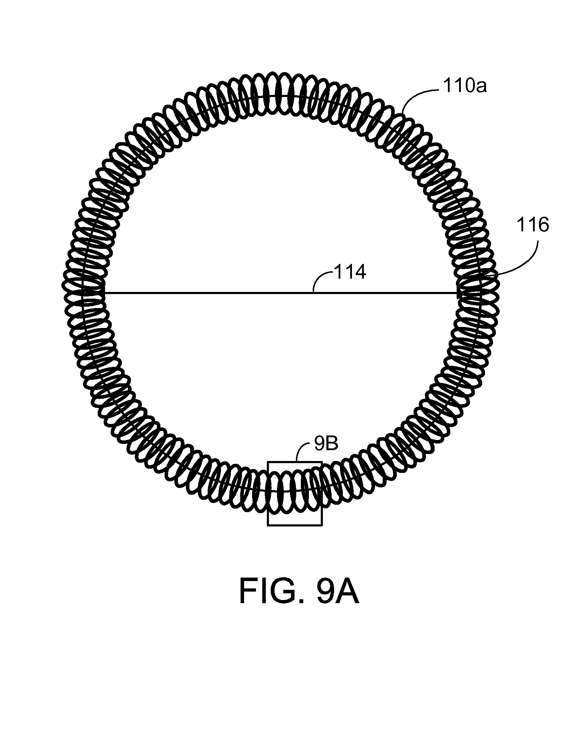

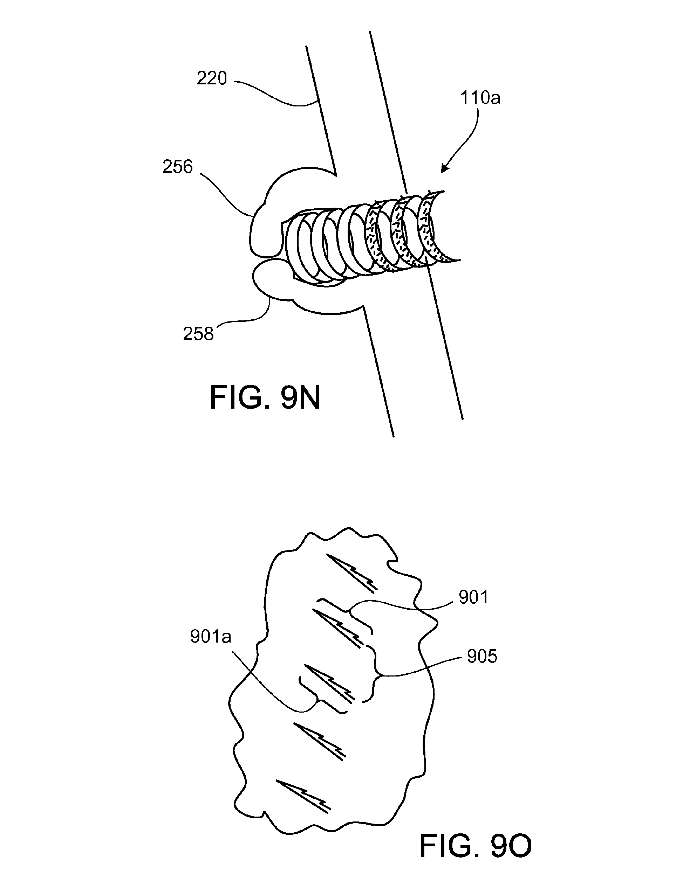

In figure 9A, the support body 110a can be a torus in the form of a helical spring (as mentioned earlier). Such a support body can have a native circumference 116 on the order of ten centimeters in its contracted state, and a proportional native diameter 114. The circumference can be selected based on the physical requirements of a particular patient.

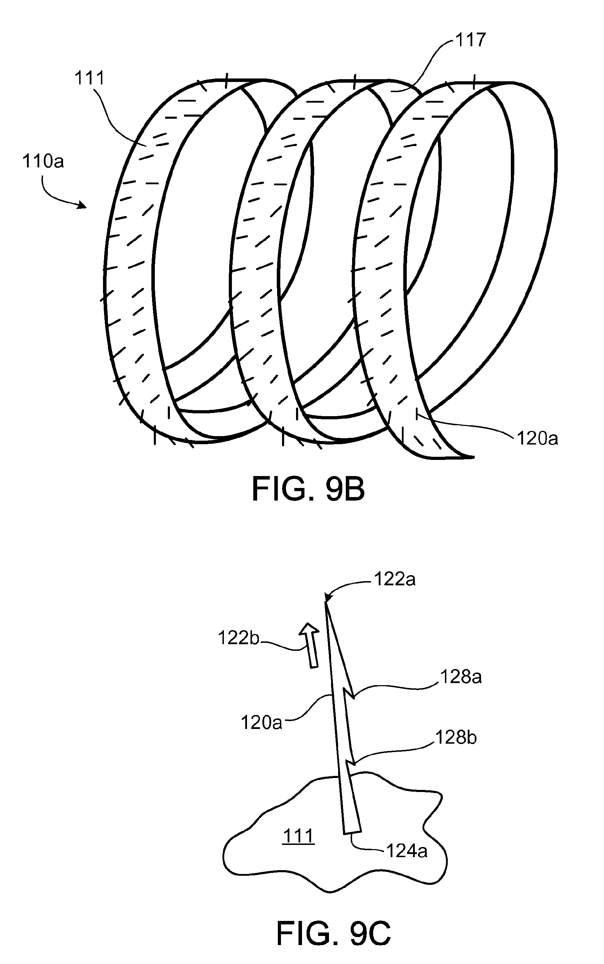

A close-up view of a fragment of this support body, figure 9B, shows that some implementations have a number (e.g., a large or very large number, for example, as few as say 15, or 100, and up to hundreds or even thousands) of burr hooks 120a attached to an outer surface 111 of the support body 110a. In the example shown in figure 9B, the helical support body is wound from a flat strip that has the outer surface 111 and an inner surface 117. Although figure 9B shows the burr hooks attached only to the outside surface, burr hooks could also be attached to the inner surface for manufacturing reasons or for other purposes.

The burr hooks, which are small relative to the body, are each configured to partially or fully pierce annular tissue when the part of the body to which the burr hook is attached is pushed against the tissue.

As shown in figure 9C, in some examples, each burr hook 120a has a sharp free end 122a for piercing tissue and at least one barbed end 128a, 128b (two are shown in figure 9C) for keeping the burr hooks embedded in tissue. Each burr hook also has an end 124a that is attached to the surface of the support body. Once the support (we sometimes refer to the support structure simply as the support) is in contact with heart tissue, the embedded burr hooks hold the body in a proper position and configuration on the annulus. Burr hooks can be attached to the surface of the support body using glue, cement, or another type of adhesive, or formed from the support body as part of an industrial process, such as molding, etching, die cutting, welding, or another process, or can be attached by a combination of these techniques. Different burr hooks on a given support can be attached by different mechanisms.

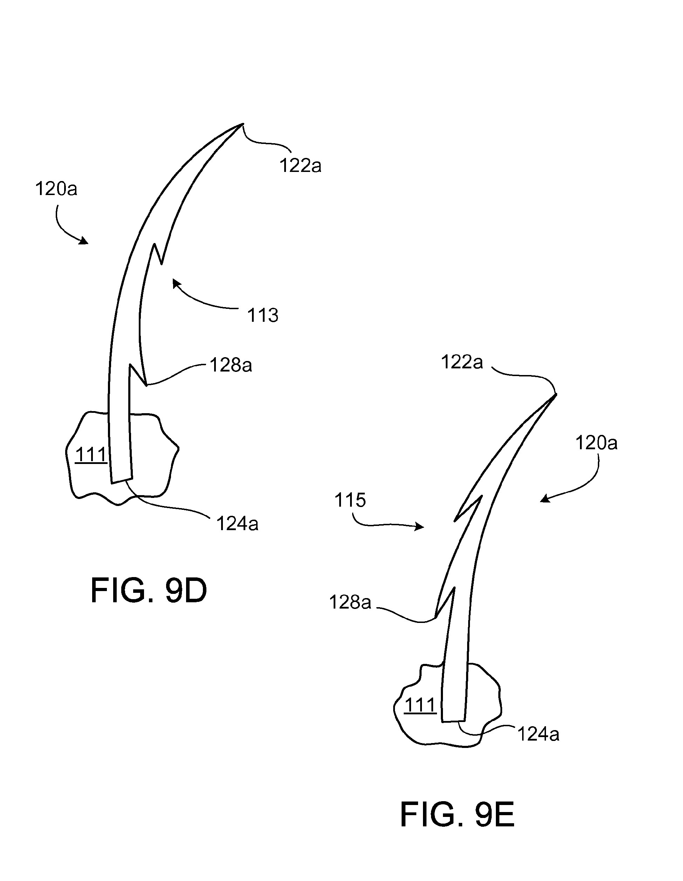

Each burr hook 120a can be structured and attached so that the free end 122a points in a direction 122b perpendicular (or some other selected effective direction, or deliberately in random directions) to the body surface 111. In some cases, the burr hook can be curved. A barbed end 128a could be located on a concave edge 113 (figure 9D) or a convex edge 115 (figure 9E) of a curved burr hook.

The burr hooks bear a resemblance to burr hooks on natural plant burrs. A different kind of attachment device could be used by analogy to metal tipped hunting arrows in which a sharp point has two broad and sharp shoulders that cut the tissue as the point enters. The tips of the two shoulders serve a similar function to the barbs, keeping the arrow embedded once it enters the tissue.

In some implementations, the burr hooks on a support body have two or more (in some cases, many) different shapes, sizes, orientations, materials, and configurations. By varying these features, for example, the orientations of the burr hooks, it may be more likely that at least some of the burr hooks will become embedded in the tissue, no matter how the support body is oriented at the moment that it comes into contact with the annulus. Varying the number, orientation, and curvature of the hooks may make it more likely that the support body will remain in place. For example, in such a support, a force applied to the support body in a particular direction may unseat or partially unseat some of the burr hooks by disengaging the barbed ends from the tissue, but the same force may not affect other burr hooks that have barbed ends oriented in a different direction or in a different configuration than the unseated burr hooks. The force applied to seat the support may cause some burr hooks to embed more securely than other burr hooks.

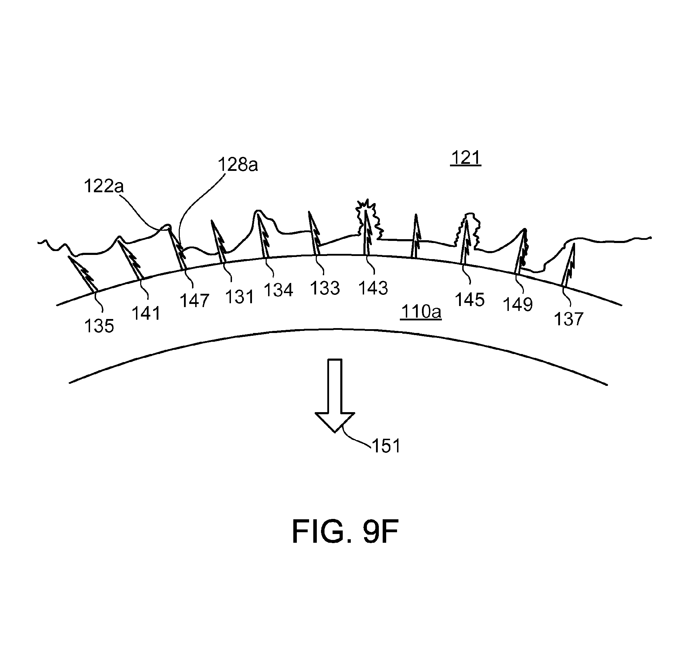

In use, typically not all of (in some cases not even a large portion of) the burr hooks will embed themselves in the tissue when the support body is pushed against the tissue, or remain embedded after placement. As shown in figure 9F, there are enough burr hooks arranged in an appropriate way so only a fraction of the total hooks need be embedded in annular tissue (and in some cases only in certain regions) to create a physical bond to keep the support body properly in place. The proportion of burr hooks on a support that need to embed securely in the tissue could range from 1% to 10% or 40% or more. The averaging spacing of the successfully embedded burr hooks could range from, say, one burr hook per millimeter of support body length to one burr hook per two or three or more millimeters (or more) to secure the support appropriately. When burr hooks are grouped rather than arranged evenly on the support, the percentages of and distances between successfully embedded hooks may differ.

When the burr hooks come into contact with the annular tissue during delivery, some 131, 133, but not necessarily all, of the burr hooks pierce the tissue and (when a retracting force is applied to the delivery tool) their barbs grip the tissue. Of the remaining burr hooks, some 135, 137 may (because of the contours of the tissue, for example) not even come into contact with the tissue, and others 139, 141 may not come into contact with the tissue with sufficient force or in the right orientation to pierce the tissue and have their barbs seat securely in the tissue. Some of the burr hooks 143, 145 may penetrate the tissue but fail to grip the tissue. Some of the burr hooks 147, 149 may only penetrate the tissue at the barbed end 128a, and not with respect to the free end 122a, providing a physical bond that may be weaker than one in which the free end has been embedded in the tissue. For some or many or most of the burr hooks that enter the tissue, however, the barbed ends 128a seat properly and resist forces in the direction 151 that would otherwise unseat the burr hook. Even though a wrenching force applied to a particular burr hook in direction 151 could still be large enough to unseat the barbed end, overall the combination of many burr hooks embedded in tissue tends to keep the support body set in place and in the proper configuration. Over time, some of the burr hooks that were not embedded when the support was placed may become embedded, and some of the burr hooks that were embedded when the support was placed may become unseated.

The resistance provided by each of the barb or barbs to removal of a given burr hook from the tissue may be relatively small. However, the aggregate resistance of the burr hooks that successfully embed themselves will be higher and therefore can reliably keep the support body in place and the annulus of the valve in a desirable shape. In addition, because there are a number (potentially a very large number) of small burr hooks spread over a relatively large area, the stress on any part of the tissue of the annulus is quite small, which helps to keep the support body properly seated and the valve shape properly maintained along its entire periphery, all without damaging the tissue. The fact that a large number of burr hooks at close spacings may become embedded along the length of the support means that the support may become attached to the annulus more evenly and continuously than might be the case with the relatively smaller number of hooks described earlier, and therefore perform better.

With respect to the implementations described beginning with figure 1A, the implementations shown beginning at figure 9A tend to have more and smaller hooks not all of which need to become embedded successfully. A common concept between the two arrangements is that the hooks penetrate by being pushed into the tissue and have retaining elements that become securely embedded in the tissue when a pulling force is applied at the end of the placement process. The two concepts are not mutually exclusive. Supports like those shown in figure 1A could also have burr hooks and supports like those shown in figure 9A could also have hooks of the kind shown in figure 1A. Placement of the support could rely on a combination of both kinds of hooks.

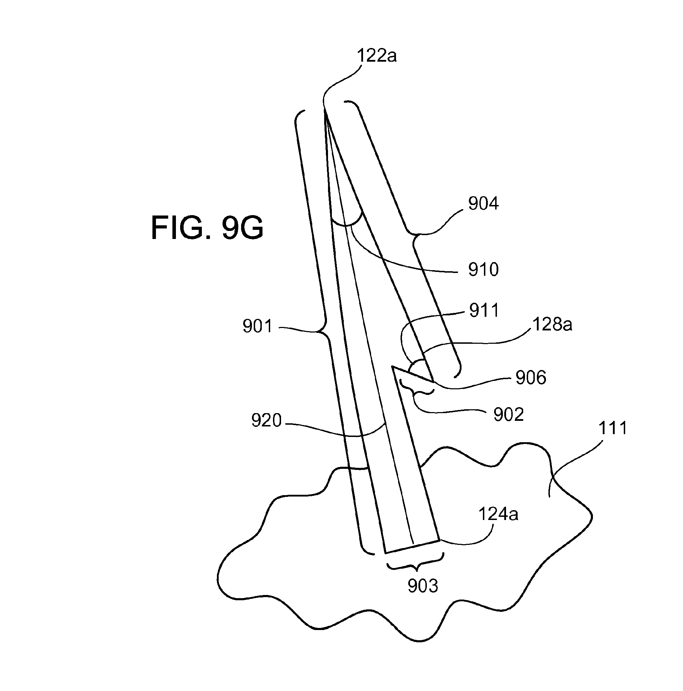

Each burr hook can be formed of a biologically compatible material such as platinum, gold, palladium, rhenium, tantalum, tungsten, molybdenum, nickel, cobalt, stainless steel, Nitinol, and alloys, polymers, or another material. As for the hooks shown beginning with figure 1A, the hooks can also be formed of a combination of such materials. An individual support body may exhibit burr hooks having a range of compositions. Some of the burr hooks attached to a support body may be composed of one material or combination of materials, and some of the burr hooks may be composed another material or combination of materials. Each burr hook may be unique in composition. Further, some parts of a burr hook may be composed of one set of materials, and other parts may be composed of another set of materials. In some examples, the region of the burr hook at the barbed end is composed of one set of materials, alloys, polymers, or mixtures, and the region of the burr hook at the free end is composed of another set of materials, alloys, polymers, or mixtures, and the rest of the burr hook is composed of a further set of materials, alloys, polymers, or mixtures. Figure 9G shows an example burr hook that only has one barbed end 128a. The burr hook extends from an attached end 124a to a free end 122a along the path of a principal axis 920 that (in this case) is perpendicular to the support body surface 111. The barbed end spans a length 904 from the burr hook's free end 122a to the barbed end's free end 906. This free end 906 forms a point spanning an acute angle 910 and the barbed end 128a spans an acute angle 911 to grab the tissue in response to any force that would otherwise pull an embedded burr hook away from tissue.

The length 901 of each burr hook could be between about 1 and 12 millimeters, as measured from the attached end 124a to the free end 122a along the principal axis. Each barbed end could extend a distance 902 from the burr hook lesser or greater than a principal width or diameter 903 of the burr hook as measured at the attached end. The cross-section of the body of the burr hook could be flat or cylindrical or ovoid or any other of a wide variety of shapes.

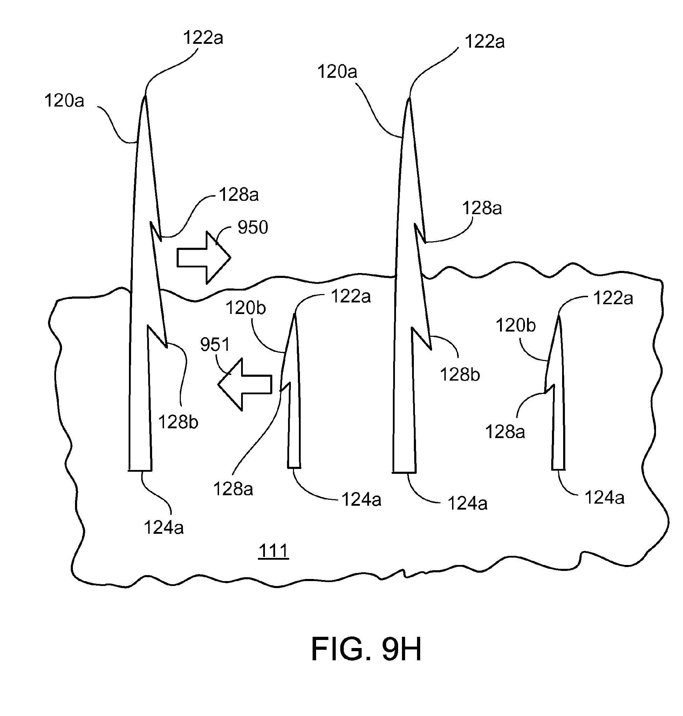

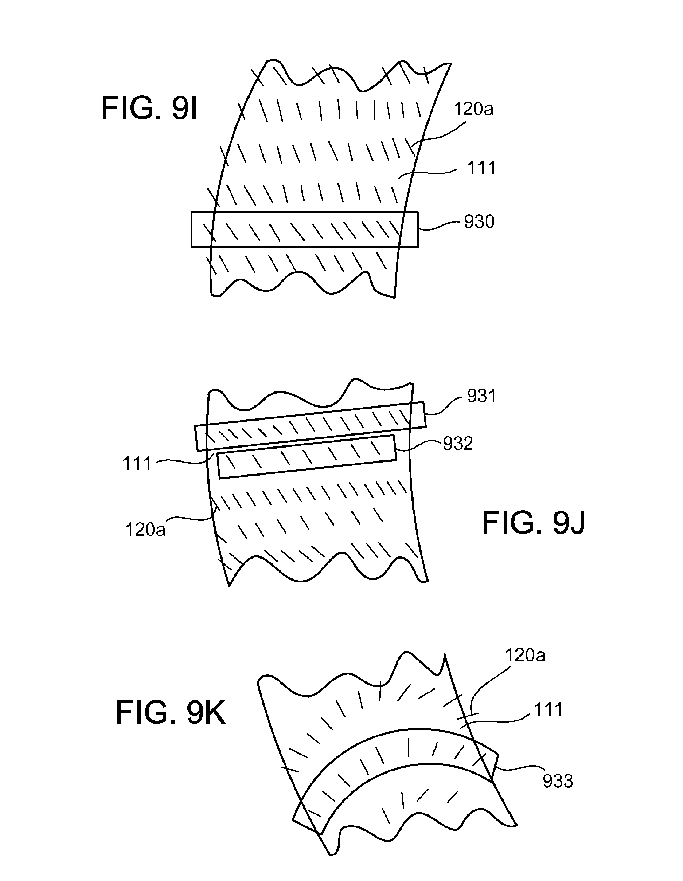

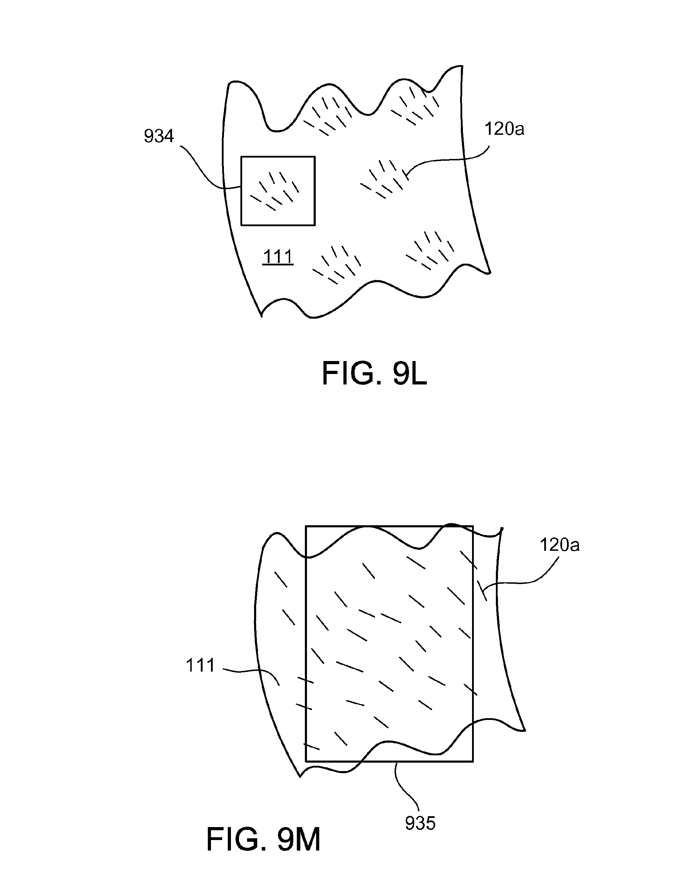

Different burr hooks may be placed on the support body surface in different sizes and configurations. For example, different burr hooks may have different lengths and different numbers and placement of barbed ends. As shown in figure 9H, for example, a portion of support body surface 111 contains burr hooks 120a that each have two barbed ends 128a, 128b facing in a first direction 950 and shorter burr hooks 120b each having one barbed end 128a facing in a second direction 951. Also, the burr hooks may be arranged on the body surface in various densities and patterns of distribution. For example, as shown in figure 9I, the burr hooks may be placed on the surface of the body in repeating rows 930. As shown in figure 9J, the burr hooks may be placed on the surface in rows of different lengths and densities 931, 932. As shown in figure 9K, the burr hooks may be placed on the surface along arc formations 933. As shown in figure 9L, the burr hooks may be placed on the surface as cluster formations 934. As shown in figure 9M, the burr hooks may be distributed randomly 935. Other patterns may also be used.

A single support body can include a wide variety of patterns of burr hooks on its surface, because the physical characteristics of a particular heart valve may mean that the valve tissue is either more receptive or less receptive to a particular pattern of burr hook distribution. Some patterns may be more effective on some types of tissue, and other patterns may be more effective on other types of tissue.

In addition, as shown in figure 9N, the burr hooks need not be present at the points where the body 110a contacts the delivery tool 220, including in the area near the rigid fingers 256, 258. This tends to prevent the burr hooks from causing the support body to stick to the tool.

As shown in figure 90, any two burr hooks may be placed at a distance 905 from each other greater than or less than the length 901, 901a of either one.

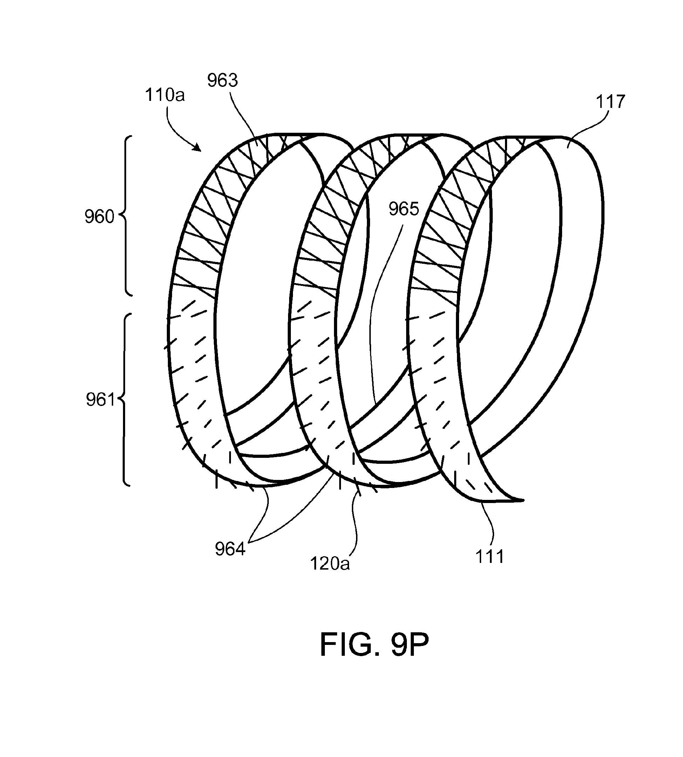

As shown in figure 9P, when a support is formed helically, the ring can be considered to have a front side 961 (which faces the valve when the support is delivered), and a back side 960 that faces away from the valve. In some examples, the support body 110a does not have burr hooks 120a on the back side 960. In these implementations of the support body, the back side 960 is covered by a sleeve 963. After the support body has been attached to the annulus, the sleeve assists in the long-term process of integration with valve tissue. Over a period of time, heart tissue will attach to the support body as part of the process of healing. The sleeve is made of a material that allows this process to occur faster than without the sleeve. For example, the sleeve may be composed of a porous material, which allows tissue to grow into the sleeve, thus securing the support to the tissue more effectively than without the sleeve. The sleeve material may be a thermoplastic polymer such as Dacron (polyethylene terephthalate). The sleeve material may alternatively be a metal or another type of material. The sleeve can be placed on the support body at a location other than the back side. For example, the sleeve could be placed on the inner side 965 of the body, with burr hooks remaining on the outer side 964.

The sleeve is formed as a half-torus in this example, but could have a wide variety of other configurations. Such a sleeve may be used with any kind of support, including the one shown beginning in figure 1A, could cover all or only part of the support, and could cover portions of the support that include hooks or barb hooks or both. In the latter case, the hook may be arranged to penetrate the sleeve during setup and before the support is placed into the heart. The sleeve could also cover a portion of the support meant to contact delicate or sensitive tissue, such as the AV node. In this case, the sleeve is made of a material that is less likely to damage or interfere with the operation of the delicate or sensitive tissue, as compared to other materials that may be used in the support.

Using burr hooks may make attaching the support faster, simpler, more reliable, and easier than for the larger hooks described earlier. The delivery tool operator may not need to apply as much force as might be necessary to embed larger hooks in the annular tissue. In some cases, the barbs would not need to be rotated as described for the larger hooks in order to embed them securely. The operator need not be concerned whether all of the burr hooks have become embedded. Once the operator has determined that the support body has made contact with the tissue and by inference that many of the burr hooks have become attached, the operator can tug on the support to confirm that it has been seated and then release the support body from the delivery tool using one of the mechanisms described earlier. Because of the ease of positioning, the procedure could be performed easily in a non-surgical context, such as in a catheterization laboratory.

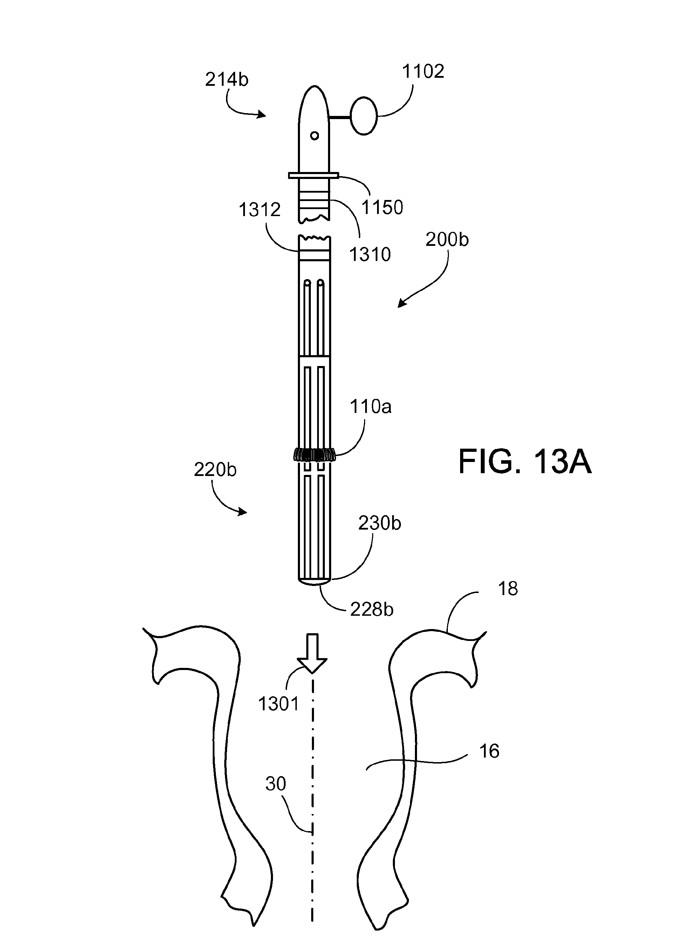

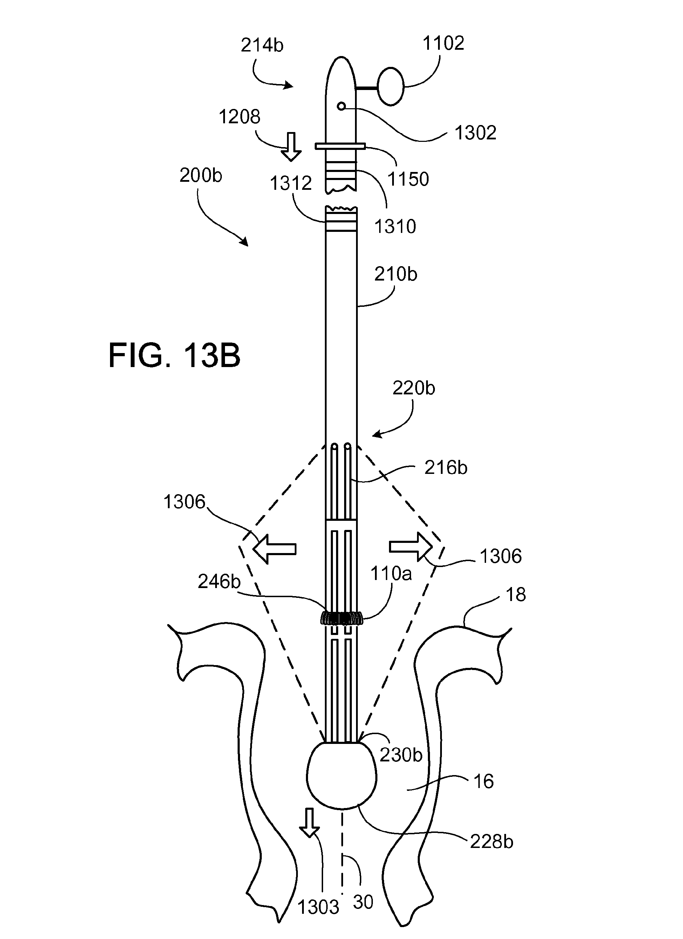

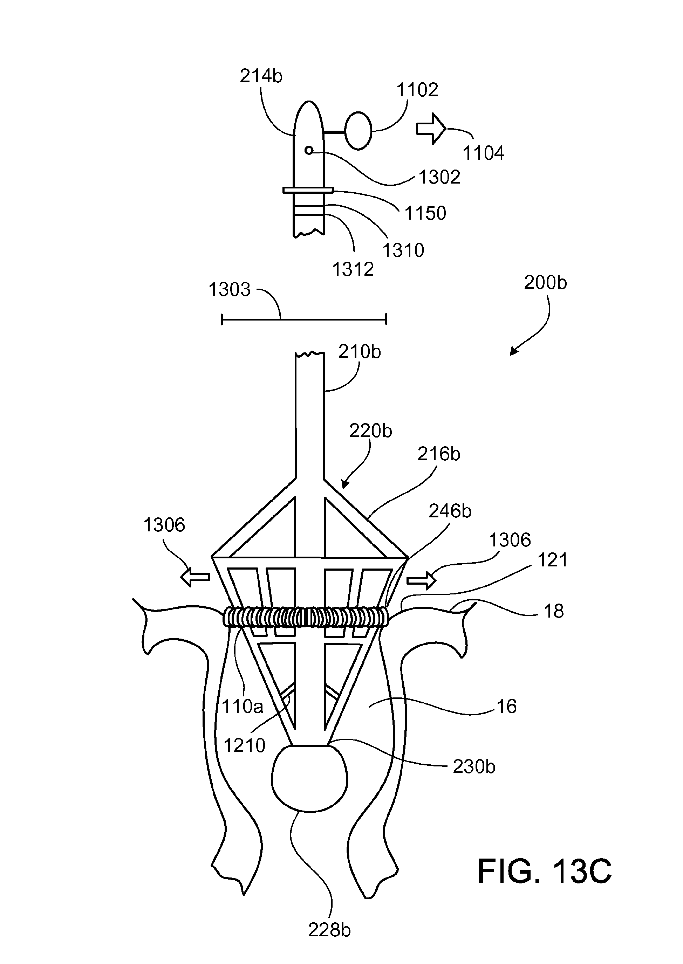

As shown in figures 13A-13D, in the catheterization context, for a burr-hook support or any other kind of support being placed, the catheter may include a balloon 228b at the tip of the delivery tool. The balloon remains deflated as the catheter is passed through the patient's blood vessels into the heart, as in figure 13A. When the tip of the catheter reaches the heart, the balloon can be inflated, shown in figure 13B. The inflated balloon floats in the blood being pumped through the heart and (along with the delivery tool) is carried easily and to some extent automatically toward and into the valve that is to be repaired. The balloon can continue to move beyond the valve annulus, and, when located as shown in figure 13C, supports the distal end of the catheter while the operator supports the proximal end of the catheter. The shaft of the catheter then serves as a "rail" supported at both ends and along which operations involving the delivery tool and the support can be performed with confidence that the rail is being held generally on axis with the valve.

In some of the examples described earlier, the annulus of the heart valve is expanded to the desired shape by pushing a conical surface, such as the basket, along the axis of and into the heart valve. Whether the delivery is done in the context of open heart surgery or in a catheterization lab, or elsewhere, the pushing of the conical surface into the annulus can be supplemented by or replaced by a technique in which the expansion of the annulus is done after the delivery tool is inserted into the valve.

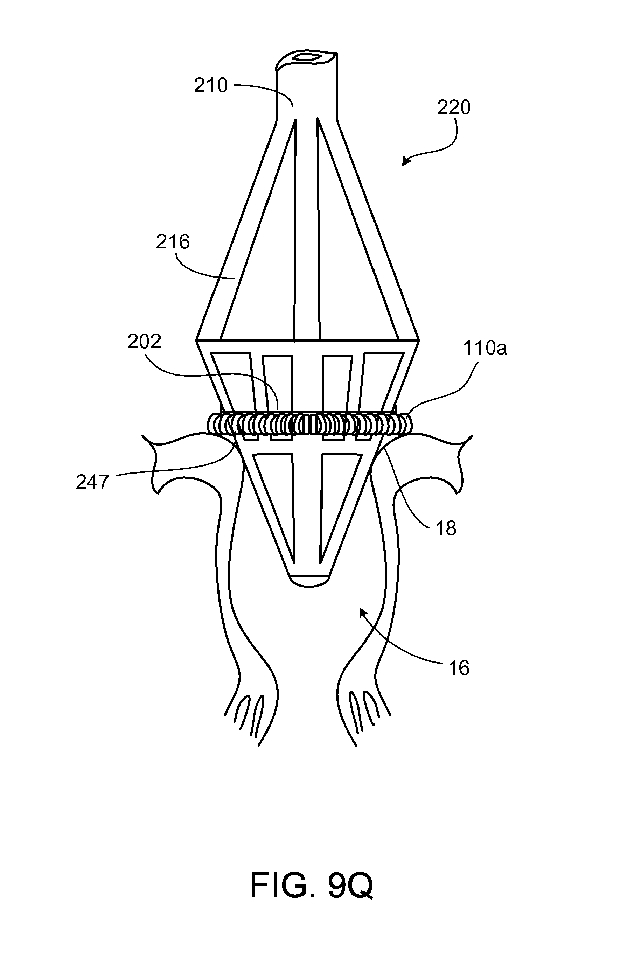

Figure 9A shows one diameter of the support body, the native (long-term configuration) diameter 114. Recall that this diameter is different from the diameter in the delivery configuration. The former diameter 114 is, as shown in figure 9Q, smaller than the latter diameter 202 of the delivery tool at the point of support body attachment 247. When the support body is placed on the delivery head 220, the coils of the helical spring stretch outward as the body expands to fit on the tool.

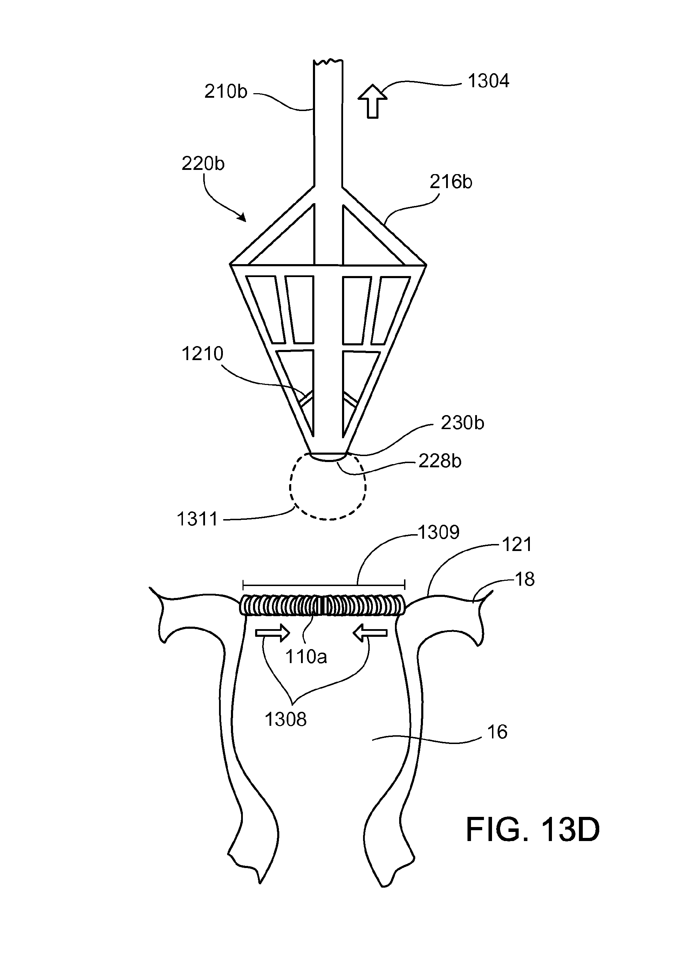

During delivery, shown in figures 13A ― 13D, when the support body has been attached to the annulus 18, the operator releases the support from the delivery tool. Figure 13D shows that, in the absence of the outward force previously applied by the delivery tool, the coils of the helical spring contract inwardly 1308 so that the support body returns to a final diameter 1309 of approximately its native diameter. Referring again to figure 1H, recall that because the annulus is attached to the support body, the support body will also pull the annulus inward, reforming the annulus to a desired smaller diameter 209.

If the support body is made of a material or alloy that is appropriately plastic, the support body may not fully contract to its original native diameter. However, if the support body is made of a shape memory alloy such as Nitinol, the memory effect of the alloy will tend to cause the support body to contract to a diameter nearly identical or identical to its original diameter.

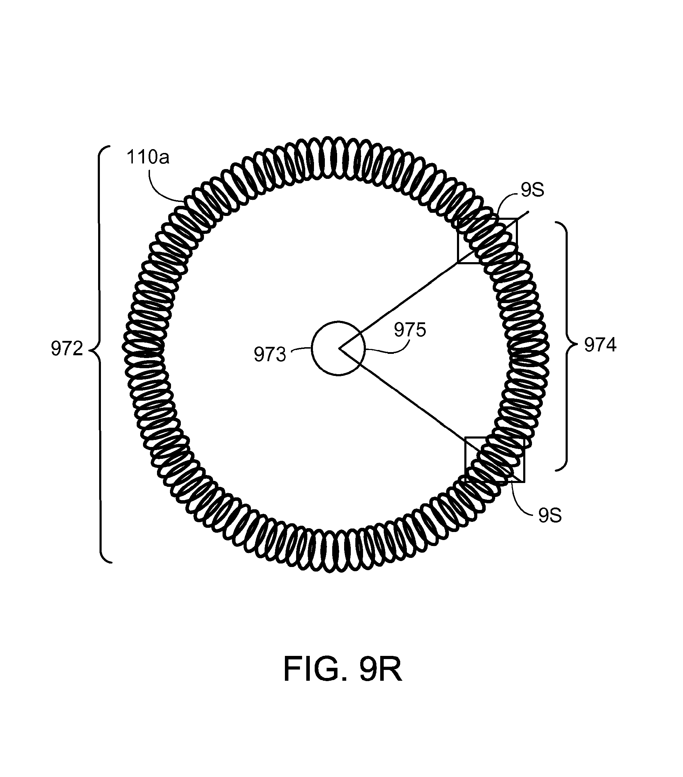

As shown in figure 9R, the support body 110a may have other portions bearing no burr hooks. As mentioned earlier, sensitive or delicate tissue such as the AV node should not be punctured or bound to hooks. In some examples, the support body 110a can have a binding section 972 having burr hooks and a non-binding section 974 having no burr hooks. A non-binding section 974 of sufficient length to abut the AV node spans an angle 975 between about 40 and 60 degrees of the support body circumference. The binding section 972 will span an angle 973 of the remaining circumference. In some examples, a non-binding section 974 is covered in a sleeve made of a material suited to contact the AV node or other sensitive tissue.

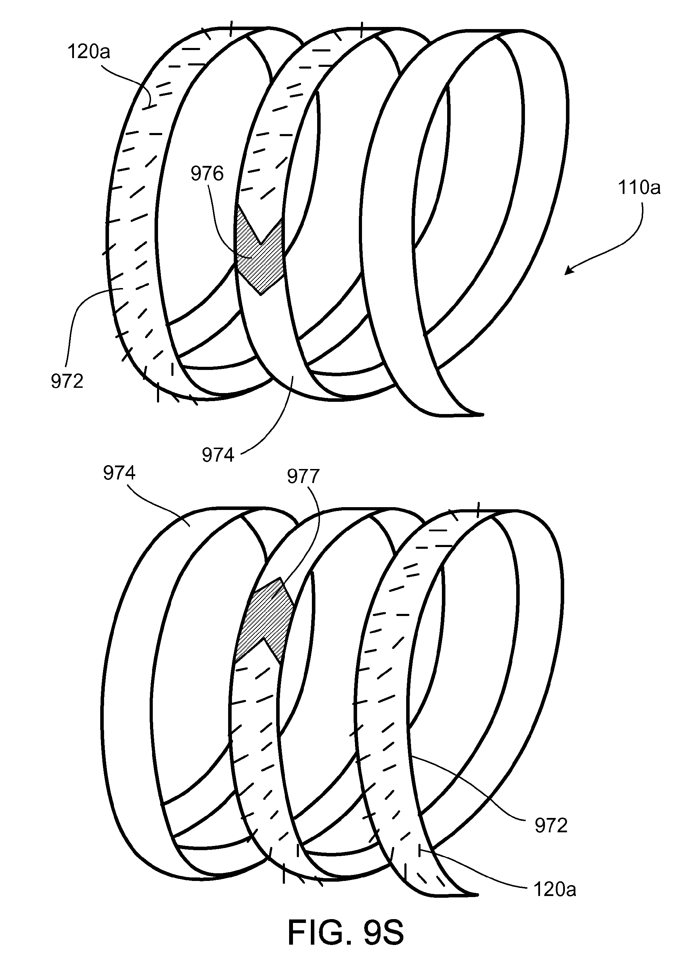

As shown in figure 9S, the two sections 972, 974 can have radiopaque markers 976, 977 indicating the borders between the two sections. The markers 976, 977 are each in the shape of an arrow pointing to the non-binding section. During delivery, an operator can use the radiopaque markers 976, 977 to view the boundary of the non-binding section 974 and position the non-binding section 974 against the AV node or other sensitive tissue.

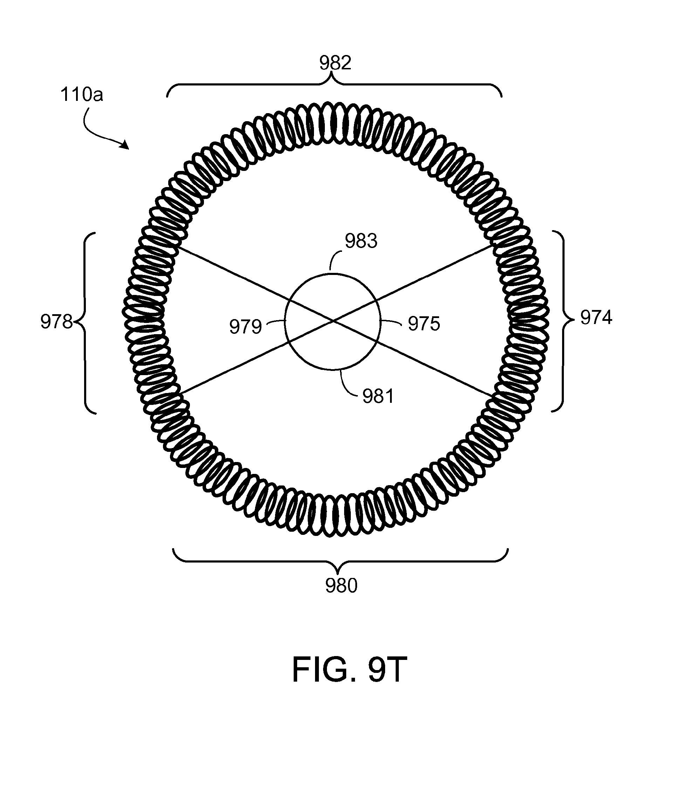

As shown in figure 9T, the support body 110a can have multiple sections 974, 978 having no burr hooks. In some situations, the operator may be limited in the degree to which the delivery head can be rotated. In this example, the operator has multiple options for positioning the support body in order to avoid puncturing the AV node, and the operator would not have to rotate the delivery head more than about 90 degrees in any direction. Two non-binding sections are shown, but the support body can also have three or more of these sections. The non-binding sections 974, 978 span angles 975, 979 between about 40 and 60 degrees of the total circumference. In the example of two non-binding sections, there will also be two binding sections 980, 982 spanning angles 981, 983 of the remaining two lengths of circumference.

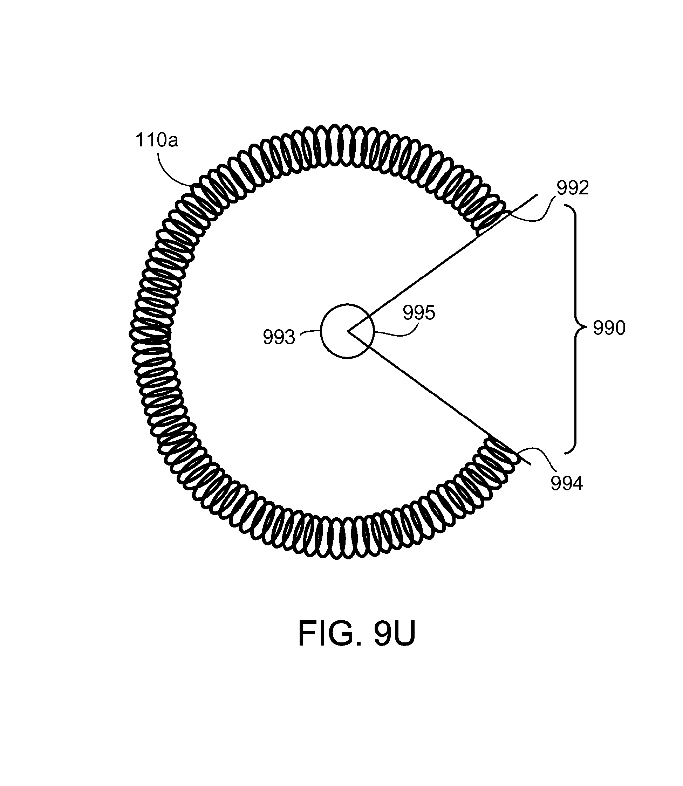

As shown in figure 9U, the feature of the support body 110a that should abut the AV node can take the form of an open section 990. As with the non-binding section described above, the open section 990 may span an angle 995 between about 40 and 60 degrees of the circle defined by the support body 110a, while the support body spans the remaining angle 993. The open section 990 can also have radiopaque markers on the open ends 992, 994 of the support body 110a to assist an operator in positioning the open section 990 against the AV node or other sensitive tissue.

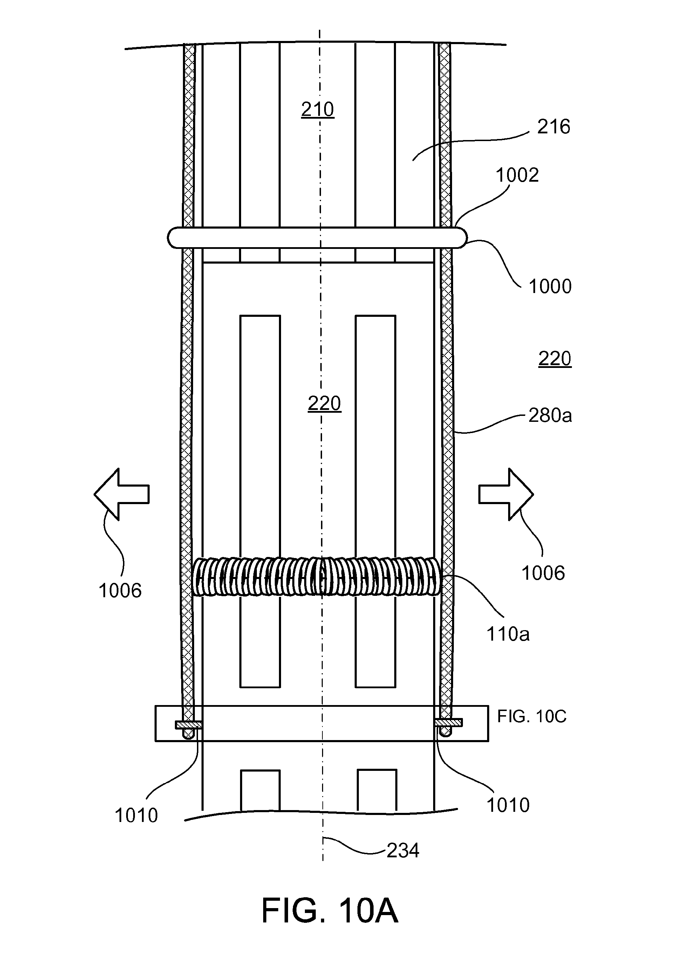

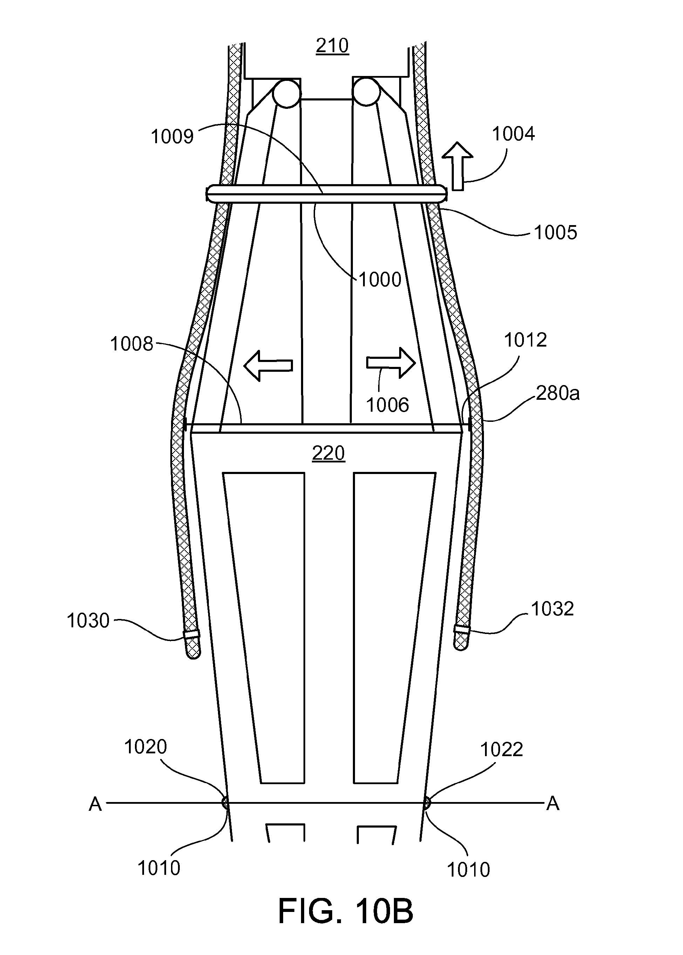

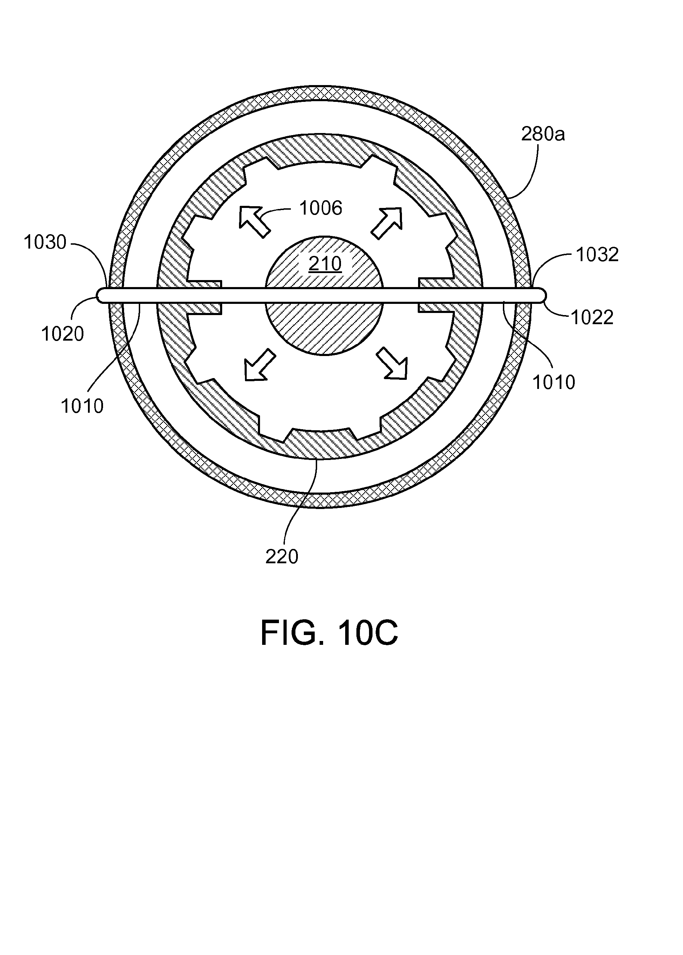

As shown in figures 10A ― 10D, the delivery head 220 can include a sheath 280a for covering the support body during insertion. Figures 10A and 10B show the sheath in a side section, and figures 10C ― 10D show the sheath as well as the delivery head in a cross-section at A ― A in Figure 10B. The sheath 280a wraps around the delivery head 220, including the support body 110a, so that the burr hooks do not accidentally puncture or attach to any other tissue or devices prior to reaching the annulus. The sheath is made of a flexible material, such as rubber, silicone rubber, latex, or another biologically compatible material or combination of materials. The sheath can also be made of the same material or materials as the catheter. Recall that one implementation of the sheath is shown in Figures 6A ― 6B and described in the corresponding text. Other implementations of the sheath are possible.

For example, the implementation of the sheath 280a shown in side section in figure 10A is kept in place by attachment to an elastic retainer ring 1000 and a crossbar 1010 permanently affixed through and extending outward from the catheter shaft 210 perpendicular to the longitudinal axis 234. The retainer ring 1000 is positioned closer to the operator and farther from the distal end than is the support body 110a, and the crossbar 1010 is positioned farther from the operator and closer to the distal end than is the support body. This sheath 280a is permanently attached 1002 to the retainer ring 1000. The sheath 280a is also attached to the crossbar temporarily at holes 1030, 1032 (visible in figure 10B) sized to fit the projecting tips 1020, 1022 of the crossbar 1010.

As shown in figures 10B ― 10D, after insertion of the catheter into the valve and when the delivery head 220 is expanded in preparation for attaching the support body 110a, the combination of the retainer ring and crossbar allows the sheath to automatically detach from the crossbar and retract upward away from the support body as part of the expansion procedure. The process by which this happens is as follows.

Referring to figure 10B, when the delivery head expands outward 1006, the diameter 1008 of the delivery head at the original point of retainer ring attachment 1012 increases to a diameter greater than the diameter 1009 of the retainer ring 1000. As a result, the retainer ring rolls upward 1004 from a point 1012 to a point 1005 on the delivery head of smaller diameter. As the retainer ring rolls, it pulls the distal end of the sheath in the same upward direction 1004 along the delivery head 220 and away from the support body 110a. Part of the sheath 280a wraps around the ring as part of the rolling process; in a sense, the retainer ring is "rolling up" the sheath, in the fashion of a scroll wrapping around a roller. The retainer ring 1000 is rubber or another biologically-compatible material with sufficient elasticity to allow the ring to roll up the expanding delivery head.

When the delivery head 220 expands, the sheath 280a is also released from the crossbar. A cross-section of the delivery head 220 including the crossbar 1010 is shown in figure 10C. When the delivery tool is in transit to a heart valve, the delivery head 220 is in the collapsed configuration. The sheath 280a has holes 1030, 1032 configured to allow the crossbar 1010 to pass through, holding the distal end of the sheath to the crossbar. Because the crossbar projects beyond the sheath, the ends 1020, 1022 of the crossbar are rounded and smooth to prevent the crossbar from piercing or tearing any tissue that it contacts before the delivery head reaches its destination. Once the delivery head is positioned near or inside a heart valve and begins expanding outward 1006 from the shaft 210, the delivery head pushes the sheath 280a outward.

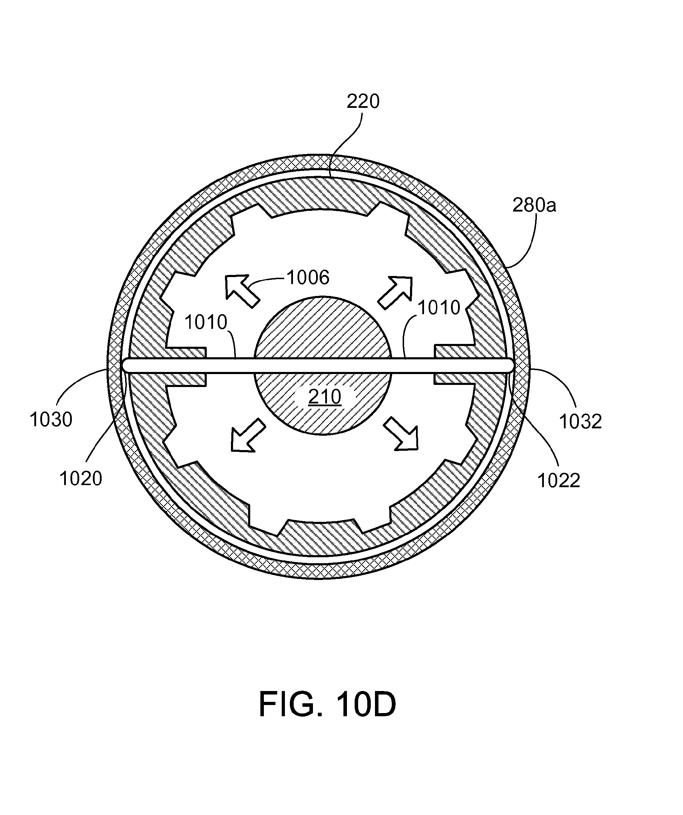

During the expansion process, as shown in figure 10D, the crossbar remains in place and does not extend outward or change configuration, because the crossbar is permanently and securely attached to the shaft 210. As a result, the delivery head pushes the sheath beyond the tips 1020, 1022 of the crossbar, releasing the sheath from the crossbar. Thus, the sheath can move freely when the retainer ring rolls upward along the delivery head, as described above. The crossbar 1010 may be made of any of the materials used in the delivery tool, or another biologically-compatible material, provided that the crossbar is sufficiently rigid to keep the sheath 280a in place, as described.

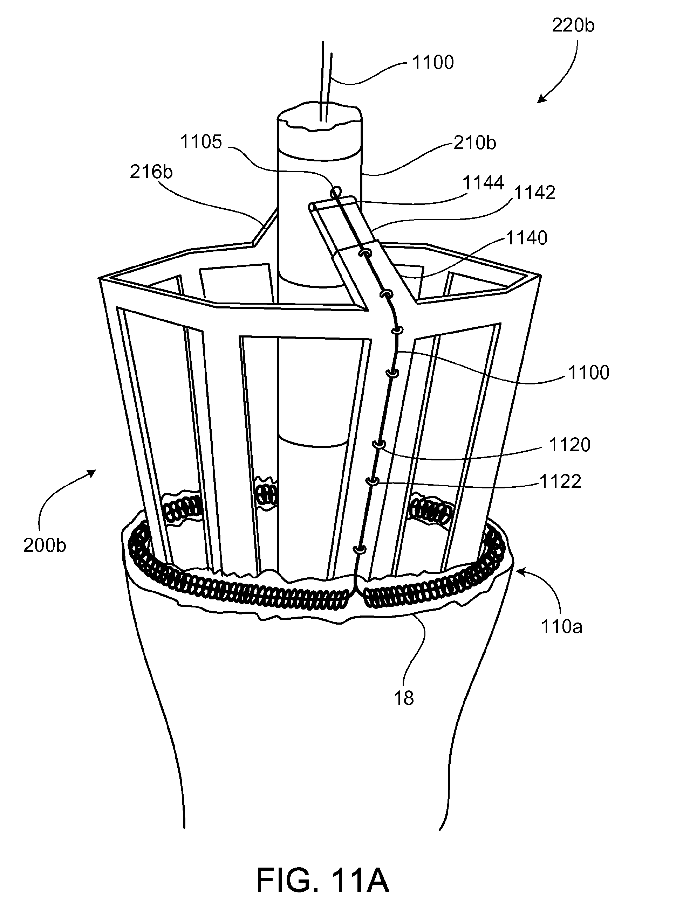

Figure 11A shows another version of the delivery head 220b. This version differs slightly from the versions of the delivery head already shown. Specifically, in this version 220b, the rigid projections 216b are composed of an outer sleeve 1140 that encloses an inner arm 1142 attached to the shaft 210b by a hinge 1144. When this version of the delivery head expands, the sleeve 1140 extends from the inner portion 1142, and when the delivery head contracts, the sleeve withdraws along the length of the inner arm. This version of the delivery head is used in figure 11A to demonstrate the use of a tightening wire 1100, but this tightening wire can be used with other versions of the delivery head as well.

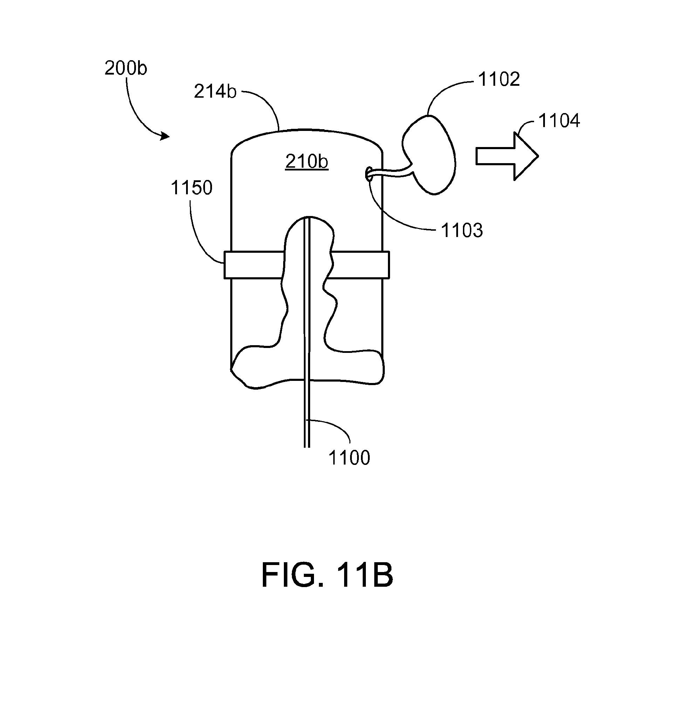

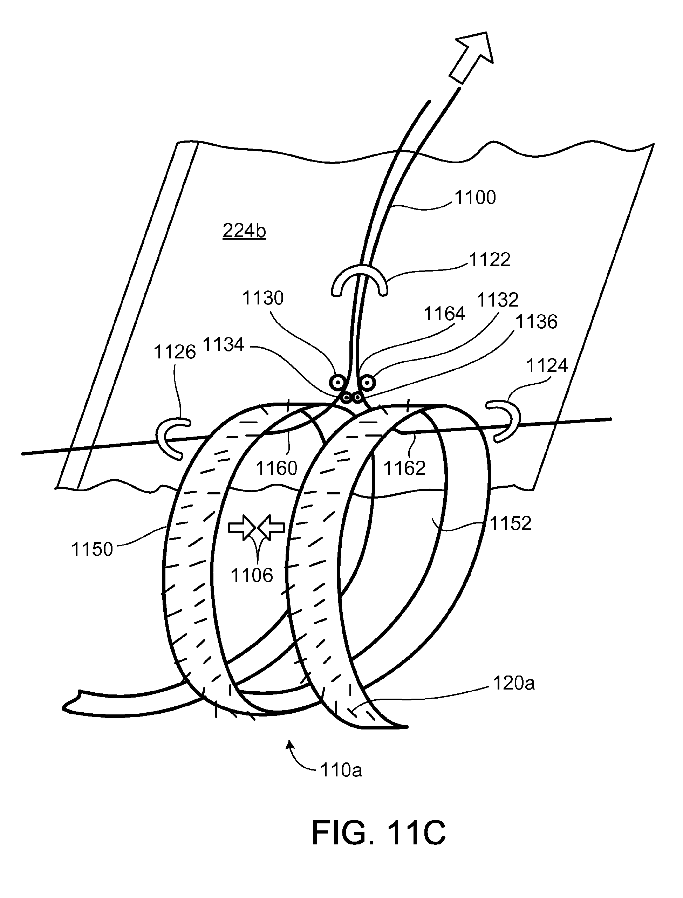

As shown in figure 11B, this tightening wire 1100 is threaded into and back out of a hole 1103 at the operator end 214b of the delivery tool 200b. In doing so, the wire traverses the interior of the shaft 210b of the delivery tool 200b. The ends of the wire exterior to the operator end 214b form a loop 1102 to be manipulated by an operator. This wire 1100 can be used to activate a mechanism to adjust the shape of the support body 110a to a small degree, with the goal of contracting the final diameter 1309, an example of which is shown in figure 13B. Referring back to figure 11A, at the other end of the delivery tool 200b, the wire exits the shaft 210b at a hole 1105 placed at a point above the delivery head 220b. The wire extends down the side of the delivery head 220b, guided by hoops 1120, 1122. As shown in figure 11C, the wire is threaded along the interior of the helical coil 1150, 1152 of the support. At the position 1164 where the wire has completed a circumference of the support body 110a, the wire returns up the side of the delivery head and back into the shaft.

Figure 11C also shows hoops 1124, 1126 that are placed on the struts 224b of the delivery head at regular intervals to keep the wire properly positioned. At the position 1164 where the wire meets itself and returns up the side of the delivery head, spools 1130, 1132, 1134, 1136 attached to the strut 224b guide the wire and prevent the wire from scraping against 1160, 1162 the helical loops 1150, 1152 at the wire exit region. The end of the wire that re-enters the hole 1105 (figure 11A) continues back up the shaft alongside itself, and exits the delivery tool (figure 11B) to form the loop 1102 by connecting with the other end.

When the support body 110a is firmly seated at the heart valve annulus 18 (for example, in the scenario shown in figure 13C), an operator can pull 1104 the loop 1102 (figure 11B) to reduce the final diameter of the support. When pulled, the wire tightens; as shown in figure 11C, this brings 1106 the coils 1150, 1152 of the support closer together.

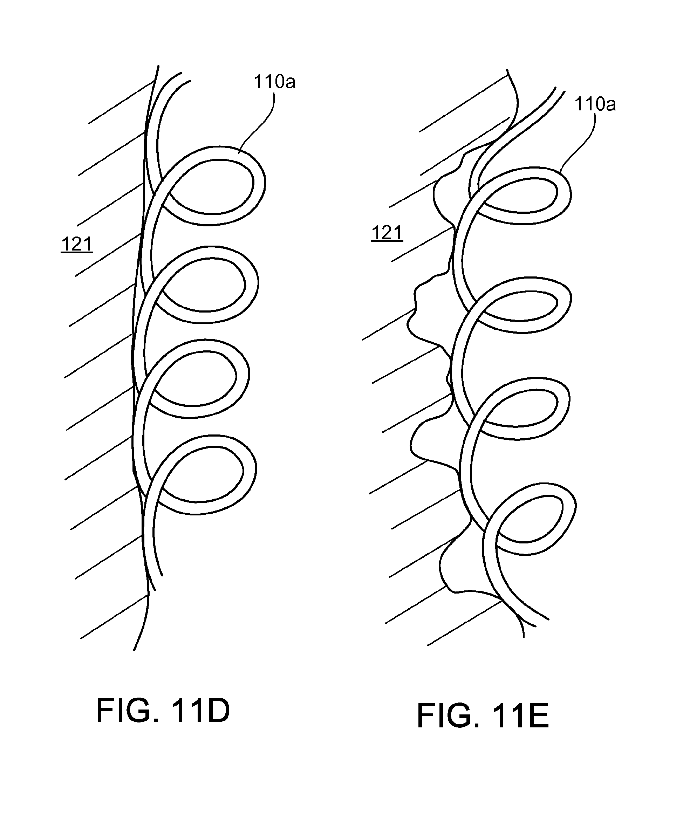

The adjusted circumference becomes permanent as the burr hooks of the support embed themselves in the annular tissue. Although some burr hooks will already have been embedded, the tightening procedure will pull out some of those burr hooks and embed other burr hooks in the tissue. This "bunches" annular tissue closer together. Figure 11D shows an example of a portion of the support body 110a attached to the periphery 121 of an annulus before the support body is tightened. As shown in figure 11E, after tightening, the support body 110a pulls the tissue at the periphery 121 closer together. The final diameter of the annulus will be slightly smaller due to this bunching effect. Once the delivery head is removed, the support body, and thus the attached annulus, will contract to the desired size.

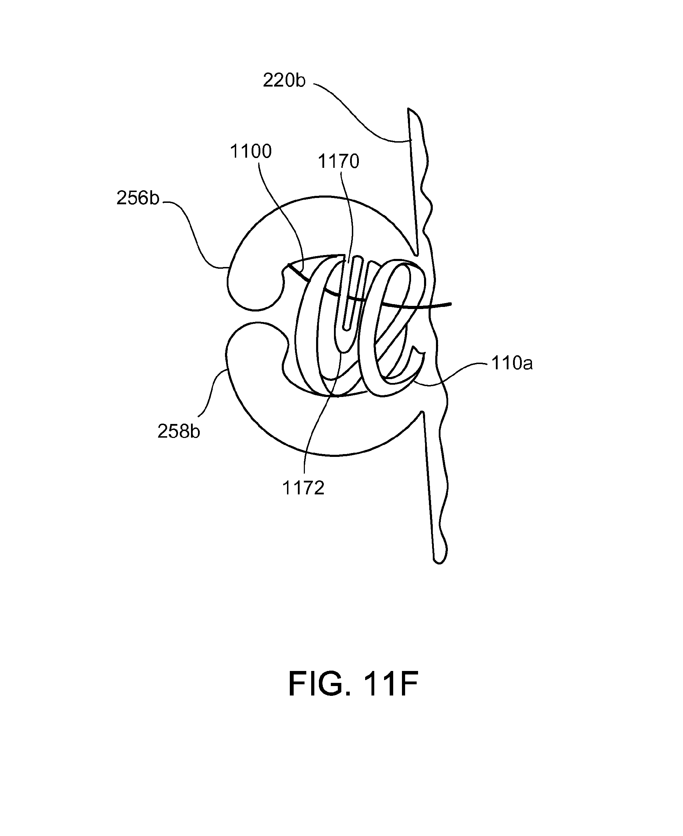

Referring to figure 11F, to detach the wire from the support body 110a, the delivery head 220b has a blade 1170 attached to one of the two rigid fingers 256b, 258b that keep the support body in place. When the rigid finger 256b pulls away from the support body 110a after the support body is in place, the cutting segment 1172 of the blade structure severs the wire. The operator may pull the external loop after the wire has been severed to keep the stray ends of the wire from moving freely outside of the delivery tool when the tool is being removed from the annulus.

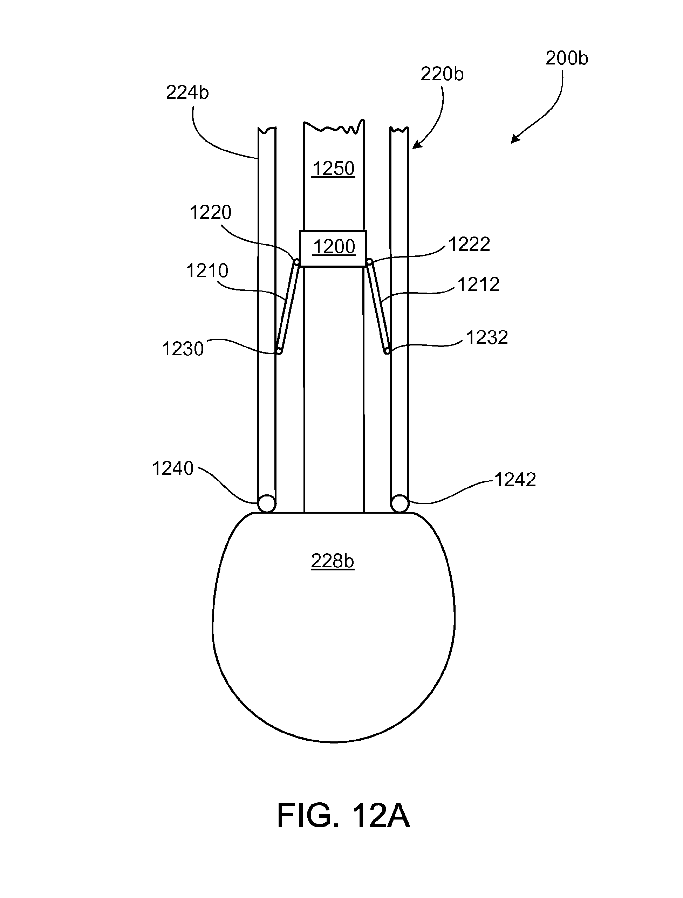

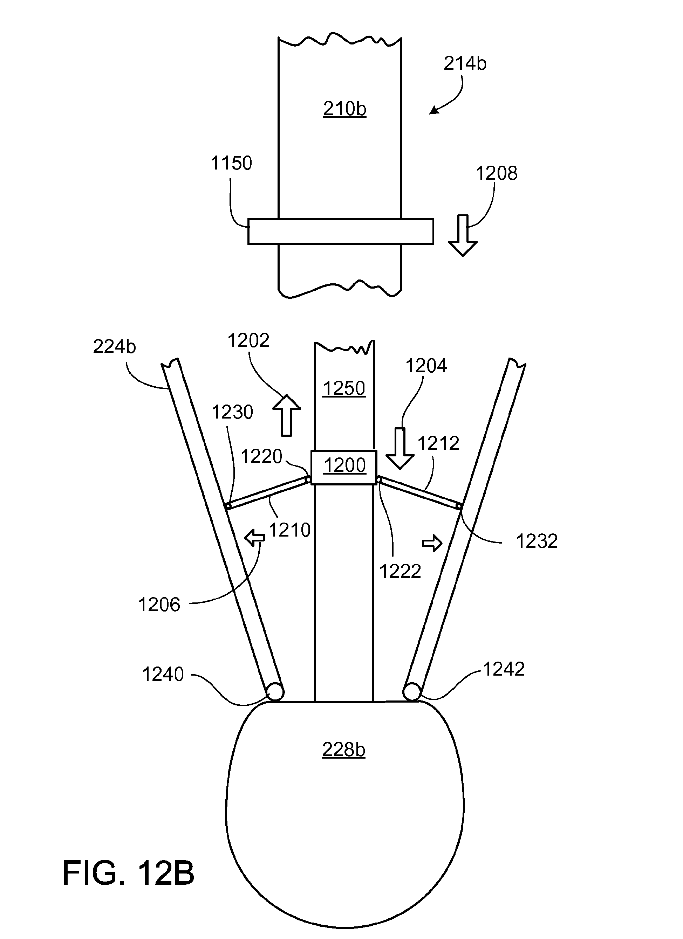

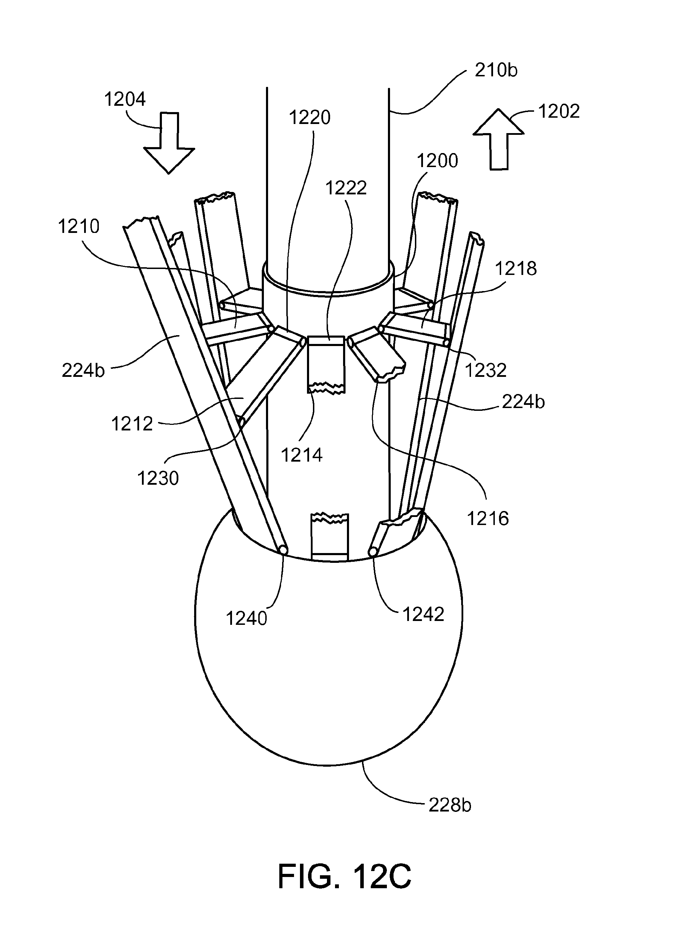

As shown in figures 12A through 12C, a delivery tool 200b for use in (but not only in) a catheterization context shares elements in common with the delivery tools discussed earlier, including the shaft 210b, collapsible conical head end basket 220b, set of struts 224b, and operator end 214b. This delivery tool 200b allows the operator to expand or contract the collapsible conical head-end basket 220b radially from a collapsed (closed) configuration (shown in figure 12A) to an expanded (open) configuration (shown in figure 12B), much in the way that an umbrella can be opened. For this purpose the basket can include a set of spars 1210, 1212, 1214, 1216, 1218 arranged about the axis, as shown in figure 12C. Referring back to figure 12B, each spar has one hinged end 1220, 1222 connected to a central collar 1200 that can ride up 1202 and down 1204 along a central shaft 1250 of the basket. Its other hinged end 1230, 1232 is connected to the hinged 1240, 1242 struts 224b of the basket in such a way that when the opening and closing mechanism is manipulated 1208 by the user to cause the collar 1200 to move back and forth along the shaft 1250, the spars 1210, 1220 force 1206 the basket open or closed, akin to the mechanism of an umbrella. The operator end 214b of the delivery tool has a twist or slide control 1150 that enables the operator to control the collar. In figure 12B, the control is a slide control, and can be slid downward, for example. In this way, the annulus can be expanded to the desired shape by radial forces 1206 that are not imposed by moving the entire basket linearly along the valve axis. Instead the basket is moved into the desired position linearly along the valve axis and then the annulus is expanded to its desired shape. The radial forces could also be imposed by a combination or sequence of moving the entire basket axially and expanding the basket laterally.

As shown in figure 13A, radiopaque measurement marks 1310, 1312 can be placed on the shaft or basket at regular spacings according to a standard measurement unit (e.g., one mark per centimeter). The marks can be used to determine the distance that the delivery tool has traversed inside the heart and the location of the basket as it is inserted into the valve, allowing the operator to place the basket at a good position along the axis of the valve.

The placement of the support from the basket onto the annulus can be done either as part of the operation of opening the basket or following the opening of the basket. In the former case, illustrated in figures 13A through 13D, the basket would be inserted into the valve to a point where the basket is adjacent to the valve annulus. Simultaneously with the opening of the basket, burr hooks on the outer periphery of the support would be forced radially into the annulus tissue. In this method of placing the support, the porous sleeve described earlier and shown in figure 9P would be positioned on the inner periphery 965, away from the embedded hooks.

In the other approach, akin to the process shown in figures 1A through 1D, the basket would be inserted into the valve so that the support on the basket was positioned slightly upstream of the location of the annulus. The basket would then be opened to force the annulus into the desired shape, then the tool and basket would be pushed slightly to force the support into place, embedding the hooks.

In either approach, once the support is placed, the basket would be at least partially closed, releasing the basket from the support, and the tool would be withdrawn from the valve.

Further, in some implementations, a combination of the approaches could be used. For example, the basket could be partially opened, inserted into the annulus, and then fully opened.

The approach of figures 13A through 13D follows these steps:
A. Position 1301 (figure 13A) the collapsed (closed) conical head-end basket 220b of the delivery tool 200b at the medial axis 30 of the valve with the support adjacent the annulus. (The tool and basket are shown in side view and the valve and annulus are shown in sectional side view.)
B. Press a button 1302 on the operator end 214b to inflate a balloon 228b (figure 13B) on the distal end 230b of the delivery tool, allowing the delivery head 220b to float into the correct position in the heart valve 16. If necessary, rotate the delivery head to align any section of the support body not bearing burr hooks, or any gap in the support body, or any portion that is sheathed, with any section of the annulus abutting delicate or sensitive tissue.
C. Slide 1208 or twist the control 1150 to expand 1306 the basket bringing the support body 110a into contact with the distorted annulus 18. The support bears burr hooks that embed themselves in valve tissue at the periphery 121 of the annulus 18 upon contact, thus attaching the support to the tissue (figure 13C).
D. When the basket 220b has reached a desired diameter 1303, the expanded heart valve support 110a forces the annulus 18 to conform to a desired configuration (e.g., a circle) and to a size that is larger (e.g., in diameter) than a desired final diameter of the annulus. Optionally, pull 1104 the wire loop 1102 to tighten the coils of the support body 110a to achieve a smaller final diameter.
E. When the heart valve support is in its final position, to break the tool away from the support attachments 246b, pull 1304 (figure 13D), allowing the support to contract 1308 to its final size (including final diameter 1309) and shape and leaving the support permanently in place to maintain the annulus in the desired final configuration and size. Deflate 1311 the balloon 228b by pressing the button on the operator end.

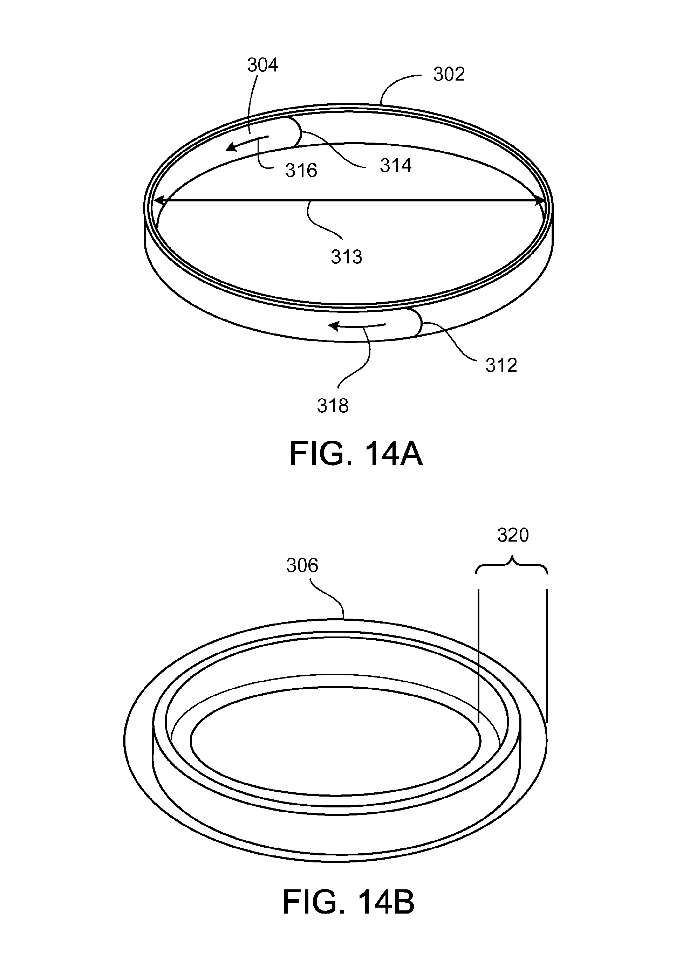

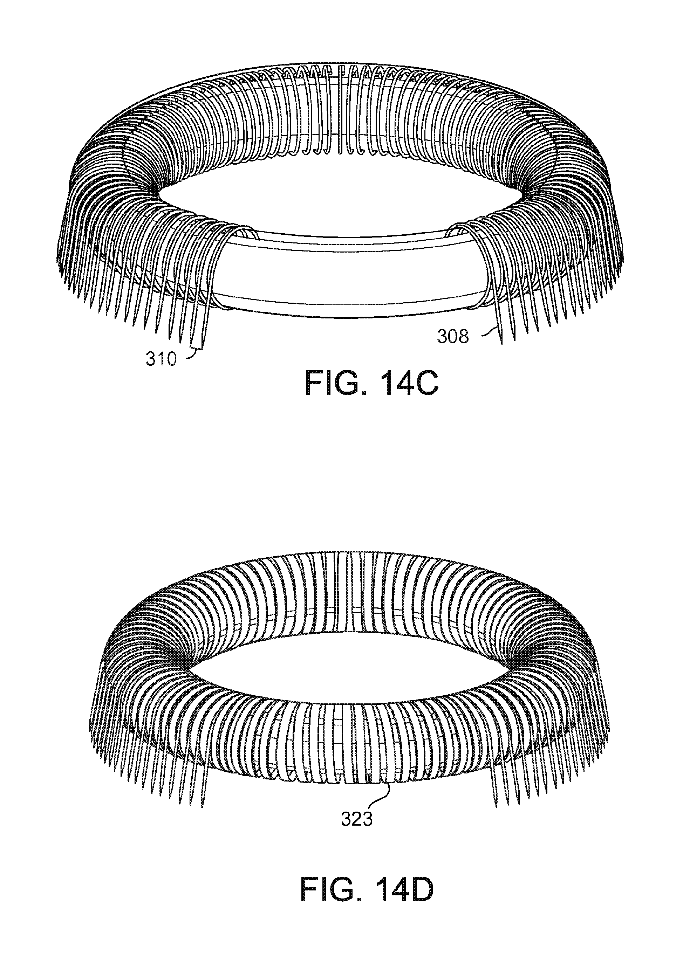

In some implementations, as shown in figures 14A through 14D, the support is constructed from several pieces including an elastic multiple-loop circular coil 302 of strip material 304. The coil is encased in a tubular toroidal sheath 306. A large number of burrs or hooks 308 (the number could be, for example, between 20 and 60, but could also be much larger in number, even orders of magnitude larger, or in some cases smaller) are mounted at regular small intervals 310 around the circumference of the toroidal sheath.

In some implementations, the multiple-loop circular coil is made of Nitinol strip, approximately 1/8 inch wide and approximately 10/1000-15/1000 inch thick. During fabrication, the Nitinol strip is shape set into a coil with final desired implant diameter. For purposes of insertion, the Nitinol coil would be expanded, as explained later. During expansion the ends 312, 314 of the strap would move circumferentially around the coil (in the directions indicated by arrows 316 and 318) to accommodate the increase in diameter of the ring. In figures 14 A through 14 D, the ring is shown in its native, unstressed diameter corresponding to the final desired implant diameter. The numbers of loops can be varied depending on the material used, the thickness, and other considerations. In some implementations the number of loops can be 3.5, or 5 or 8, or other numbers ranging from 1 to 10 or more.

In some implementations, other materials and combinations of them can be used to form the resilient coil. These could include, for example, plastics, metals, and coils of these and other materials.

In some implementations, the overall shape of the coil could be different from the one shown in figure 14A, including non-circular and non-planar shapes.

The coil (or other resilient core ring) needs to have enough strength and durability to be expandable to fit on the delivery tool, to be forced onto the heart valve annulus, to contract to pull the annulus back into the desired shape, to tolerate the force incurred when the insertion tool is disconnected, and to form a long-lasting and strong support for the annulus. It also needs to have enough resiliency to be able to contract the support and the annulus to which it is attached to the desired shape and size after insertion and to retain the support in essentially that shape and size against forces in the heart that may act against the support.

In some implementations, if there is a chance of exposure of the materials of which the coil is made to the blood or tissue of a patient, biocompatible materials are used.

The coil is held within the sheath 306 in a way that permits the coil to slide within the inner lumen of the sheath, especially as the coil is expanding for insertion and contracting after insertion. The sheath has an elasticity that allows it to move radially with the coil during expansion and contraction. Because the burrs or hooks (we sometimes refer to burrs and hooks and a wide variety of other gripping devices as grippers) are mounted on the sheath, and not on the coil, the expansion and contraction of the coil can occur without disruption of the angular locations of the grippers relative to the central axis of the support.

In some implementations, the sheath can be formed of a simple tube. To embed the coil in such a tube the coil can be unwound and wrapped through the tube repeatedly until all turns of the coil have been embedded. Once the coil is completely embedded, in the tube, one end of the tube can be pulled over and glued to the other end to finish the assembly.

In some implementations, the sheath can be formed of a specially molded piece that has the toroidal shape formed during molding and includes a way to secure the two ends together.

In some implementations, the sheath is meant to be sealed to prevent fluids from passing into the chamber that contains the coil. In some cases, the sheath is not sealed and fluid can pass freely. In some implementations, a fluid is used to fill the space within the sheath to provide lubrication for the sliding of the coil within the sheath and to displace air which could cause problems when the support is used inside the heart. The fluid could be blood or saline solution, for example.

The sheath must be strong enough to enclose the coil without breaking even when the support is expanded and contracted prior to, during, and after placement in the valve. As the diameter of the support is expanded and contracted, the cross-sectional diameter will also tend to change, and the amount of that change must not be so great as to disrupt the attachment of the grippers to the valve tissue, to constrain the sliding of the coil within the sheath, or to allow the grippers to become dislodged or disoriented relative to the sheath, among other things. The sheath can be resilient so that when the support is contracted after being expanded, the sheath contracts along with the coil.

A wide variety of materials can be used for the sheath, including silicone, plastics, and fabrics, for example. Combinations of materials can also be used.

As shown in figure 14D, an outer surface 322 of the sheath can bear grooves 323 that accommodate (and hold in place) portions of the grippers, as explained below. In some implementations, the grooves can be parallel and lie at equal small intervals around the perimeter of the sheath.

The cross-sectional diameter of the sheath can be large enough so that the inner lumen accommodates the coil and allows it to slide, and the outer surface supports the grippers, and small enough that the support does not obstruct adequate flow of blood through the heart valve after installation.

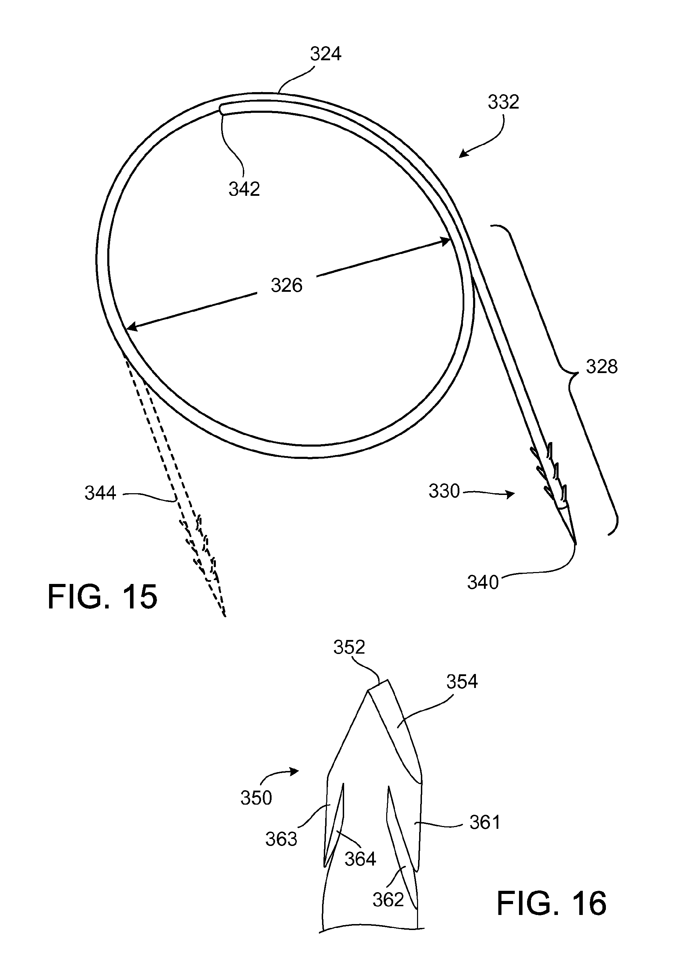

As shown in figure 15, in some implementations, each of the grippers can be formed on a length of wire that includes a closed ring 324 that has about the same diameter 326 as (or slightly smaller than) the diameter of the cross section of the sheath. A straight section 328 extends from the ring and has the gripper 330 formed on its free end.

We sometimes refer to the entire piece that includes the gripper, and a portion to attach the gripper to the support, as an anchor 332.

In some implementations, the anchor is prefabricated with the ring in its final shape and the gripper projecting from the ring. In some examples, the anchor is formed of stainless steel or another biocompatible material.

A wide variety of materials and combinations of them can be used to fabricate each of the anchors or groups of them, including metals and plastics. The cross-sectional shape of the anchors can vary and be, for example, round, oval, flat, or bent, or a variety of other shapes.

In some implementations, the anchors can be made from tiny fishhooks with the hook end serving as the gripper and the other end being bent to fit onto the support.

The thinner the anchors in the direction along the circumference of the sheath, the more anchors that can be fit onto the support. In some implementations, a larger number of thinner anchors would be useful in making the support easy to install and effective. In some cases, the arrangement of the anchors along the sheath can be other than regular and closely spaced. The spacing can be varied along the sheath or the number of anchors can be varied along the sheath, for example.

To install an anchor, its ring portion can be pulled open and slipped over the sheath, then released. In examples in which the outer surface of the sheath is molded to have grooves, the ring portions of the anchors can be seated in the grooves.

In some examples, the anchors can all be mounted to cause their grippers to point at a common angle 336 from a central axis 338 of the support as shown in figure 14D (in which some of the anchors have not yet been mounted). In some examples, the grippers can be pointed at different angles relative to the central axis.

In some examples, the anchors can be mounted in such a way that they do not tend to slip or rotate around the outer surface of the sheath, but rather maintain their installed orientations. In some implementations, when the supported is expanded and contracted prior to, during, and following insertion into the heart valve, the stretching and relaxing of the sheath may cause a change in its cross-sectional diameter and therefore an opening and closing of the rings and a corresponding reorientation of the angles of attack of the points of the grippers. This effect can be useful in installing and providing secure attachment of the grippers in the valve tissue.

In some cases, if the angle of attack of the points is shared in common by all of the grippers, then it may not be desirable to have the successive anchors along the perimeter be spaced too closely 310 because the adjacent gripper points could interfere with each other during insertion, and be less effective in gripping the valve tissue. For this reason, in some implementations, the angles of attack of the points of the grippers can be varied slightly from anchor to anchor which would permit a closer spacing while still allowing some clearance between successive grippers. In some cases the orientations of successive grippers could alternate back and forth around a central line. Other arrangements are also possible.

In figures 14A through 14D and 15, the anchors are shown as each having a single free end bearing a point 340. In some implementations, each anchor could provide for an extension of the other end 342 of the wire (for example, a symmetrical extension), as implied in dashed line 344. A wide variety of other arrangements are also possible.

In figure 15, the gripper has three barbs on each side of the free end of the wire. In some implementations, there could be more or fewer barbs, and the barbs could have a wide variety of other configurations on the gripper.

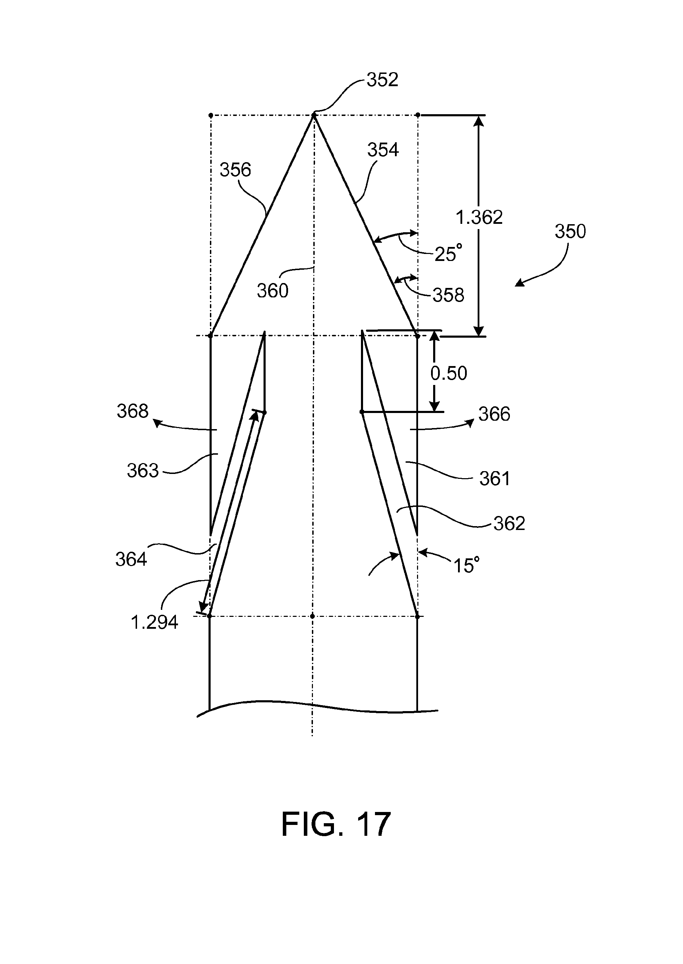

In some implementations, each of the grippers 350 can be formed of wire or other cylindrical material and can be formed, machined, or molded, for example, to have the configuration shown in figures 16 and 17, including a point 352 having two symmetrical faces 354, 356 each at an angle 358 of, for example, 25 degrees relative to a central axis 360 of the gripper. Below the point are two barbs that are formed, by laser cutting, machining or otherwise imparting slots 362 and 364 at a common angle (15 degrees in this example) to the central axis.

Once the barbs are formed they can be bent away from the axis in the directions 366 and 368 to form the final barbs.

A wide variety of other configurations and forms of manufacture are possible for the barbs and the grippers. In the particular example shown in figures 16 and 17, the grippers are formed of Nitinol wire that is 1.26 mm in diameter and the length of the gripper to the bottom edge of the slots is 22.87 mm.

As shown in figure 14D, in some examples, when installed each of the grippers extends from about 2 to about 4 millimeters (dimension 339) from the bottom of the sheath surface.

In some implementations, the support—which includes the coil, the sheath and portions of the anchors—is wrapped in a cloth covering as are many existing rings that are hand-sutured to the valve annulus by a surgeon. The cloth allows the heart tissue to attach itself securely to the support over time, making for a secure repair.

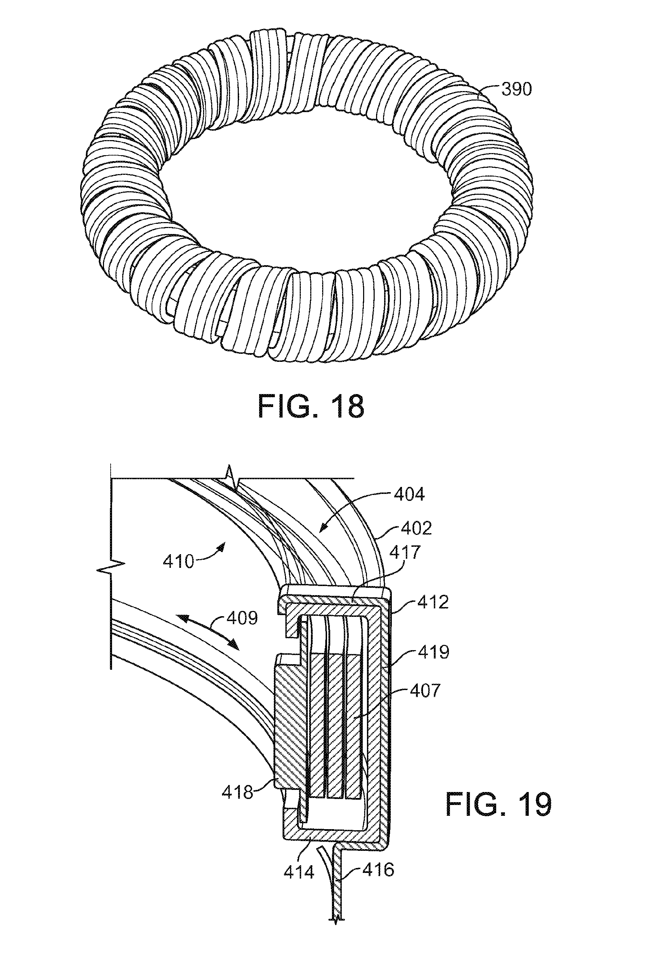

As shown in figure 18, in some cases, the cloth covering can be a thin strip of material that is helically wound around the other parts of the support. The material may be attached to the support by suturing, gluing, or in other ways. The helical winding allows an inelastic material to be employed and still accommodate the circumferential expansion of the support. In some examples, the cloth covering may include a series of independent tubular cloth segments placed over the support. The segmented arrangement will allow inelastic cloth to be used without hindering circumferential expansion of the support.

As the cloth is placed on the support, it is pulled over the grippers, each of which penetrates the cloth and remains ready for insertion. A wide variety of covering materials or combinations of them could be used including metal, fabric, and plastic. The covering should be able to accommodate the expansion and contraction of the support without becoming distorted and should be biocompatible and porous enough to accept and encourage the growth of tissue through its structure,

A wide variety of other configurations of parts and materials, and ways to assemble the parts of a support are possible. Different numbers of pieces can be used, and the functions described can be combined in different ways into different pieces of the support.

In some examples, shown in figures 19, 20, and 21, the sheath can be made of two molded pieces that interlock. An outer annular housing 402 (sometimes called the outer piece) has upper and lower flat rings 404, 406 joined by an outer flat cylindrical wall 408. The coil 407 sits within the housing. The other, inner piece 410 of the sheath is a cylindrical wall that is captured between the upper and lower rings 404, 406 in a way that permits the inner end 408 of the coil to be tightened or loosened by sliding it circumferentially 409, causing the support to be expanded or contracted. During the sliding, the inner piece of the sheath slides circumferentially also.

In this example, the anchors 412 are formed from flat pieces of metal that are bent and then attached to the outer piece of the sheath. Each anchor includes an upper finger 417 that grasps the upper portion of the outer piece of the sheath, a vertical arm 419 and a lower finger 414 that grasps the bottom of the outer piece of the sheath. The gripper 416 extends downward from the lower finger. The inner piece of the sheath has a tab 418 that can be manipulated to pull or release the end of the coil to expand or contract the support. An opposite end of the inner piece of the sheath is attached to the end of the coil for this purpose. As a result, the support can be expanded or contracted without the anchors moving relative to the outer piece of the sheath. The tab 418 can be manipulated in a wide variety of ways, including by direct finger manipulation, use of an insertion tool in open heart surgery, or manipulation at the end of a catheter from a distant position in a catheter laboratory.

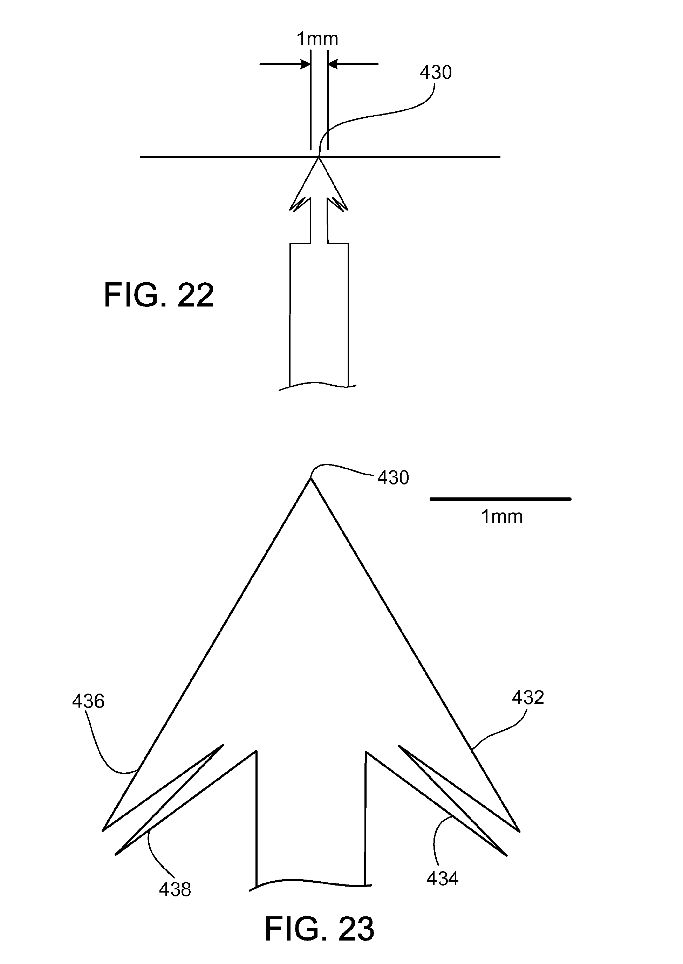

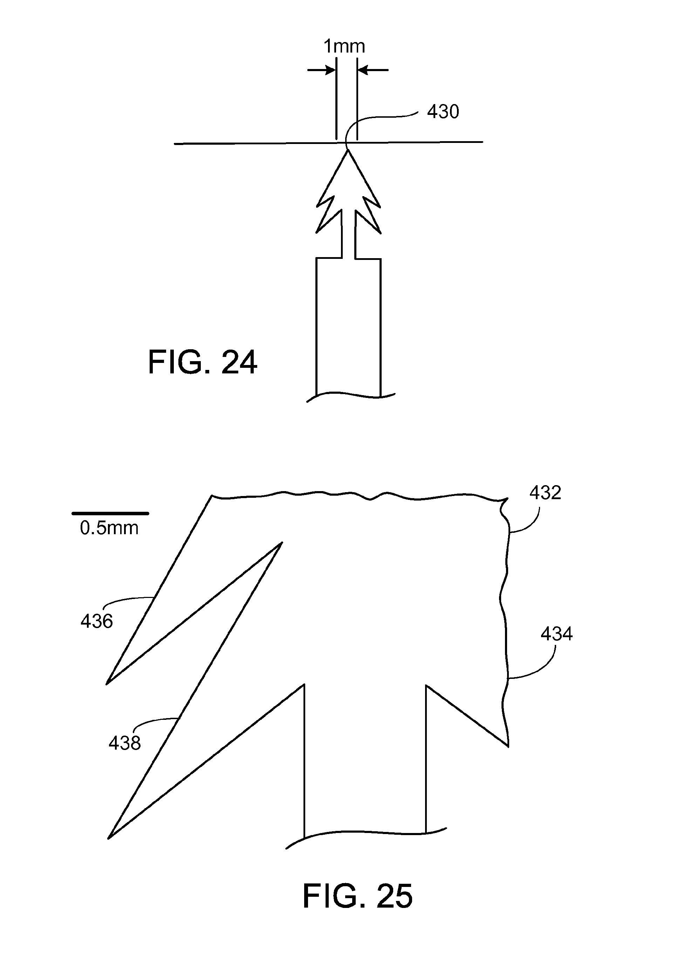

In some implementations of a gripper, as shown in figures 22 through 27, there is a pointed end 430 and on each side of the pointed end, a pair of barbs 432, 434, 436, 438. In the example shown in figures 22 and 23, the barbs 434 and 438 are smaller. In the example of figures 24 and 25, the two barbs on each side of the point have a similar size and shape.

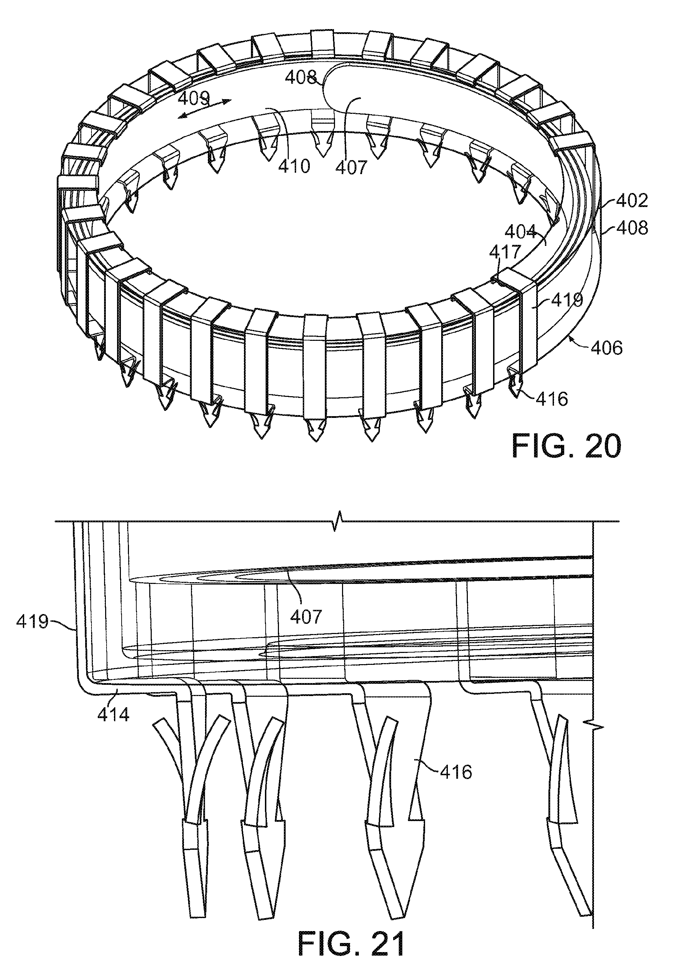

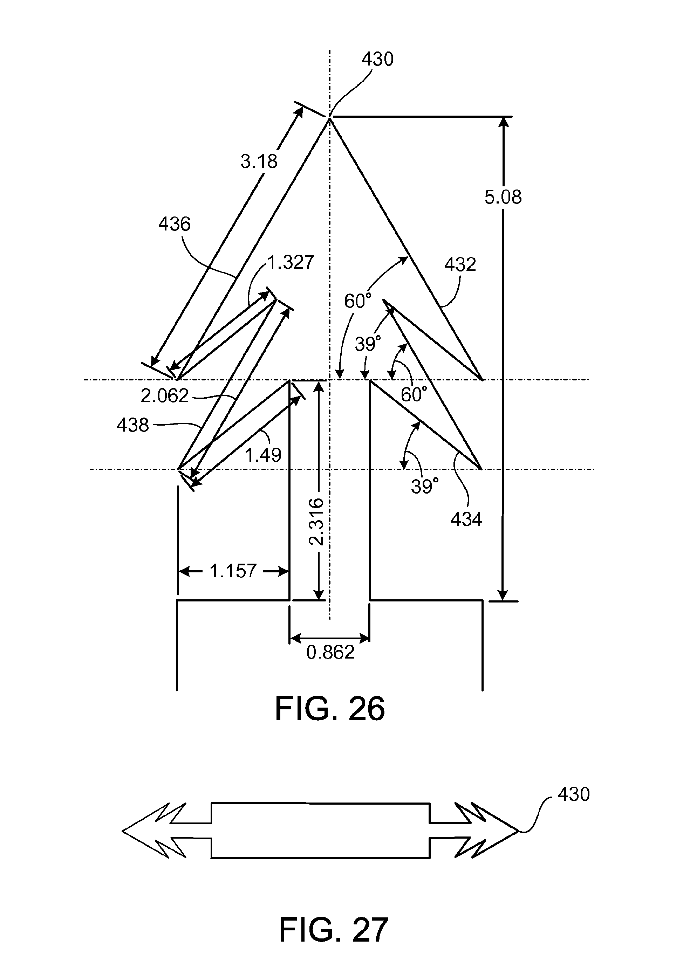

In some examples, as shown in figures 26 and 27, the detailed configuration of a Nitinol strip includes the point and the barbs. As shown in figure 21, in some configurations, the barbs are bent out of the plane of the strip from which the gripper is formed in order to be more effective as barbs.

In general, in some examples, the support to be embedded in the valve tissue can be configured to achieve three related functions: (1) the ability to easily insert the grippers of the support into the tissue once the support has been correctly located at the annulus; (2) the ability to retain the support in the tissue securely in a way that maintains the correct shape for the annulus of the valve and is durable and long lasting, in part by providing a substantial resistance to forces that could cause detachment of all or part of the support after insertion; (3) the ability to deliberately withdraw all or a portion of the grippers during or after the insertion procedure in order to relocate or reorient the support relative to the valve annulus if doing so would be useful. These three functions require a careful and subtle design of the grippers, the anchors, and the other parts of the support, because some design factors that favor one of the functions can be a negative influence on another of the functions. These functions should also be implemented in a device that is simple, foolproof in its operation, and easy to use.

For example, easier insertion of the grippers into the tissue can be achieved by reducing the size and profile of barbs on the grippers and aiming the points of the grippers directly at the tissue. Removal of some or all of the grippers to reposition the support would also be aided. But those same features could reduce the stability and durability of the attachment of the support to the tissue. By giving the barbs a broader or more obstructive profile or aiming the points of the grippers off a direct path to the tissue, the gripping is made more secure, but inserting the grippers is more difficult as is repositioning.

Among the design features that can be adjusted and traded-off to achieve a desired mix of the needed functions are the number, shape, size, orientation, and method of mounting the anchors, the grippers, and the barbs, the shape, size, orientation and other configuration of the body of the support, the materials used for all of the parts of the support, and a wide variety of other factors.

In some cases, a mechanism or configuration can be provided that allows a deliberately reversible process for inserting and removing the grippers in the tissue for repositioning.

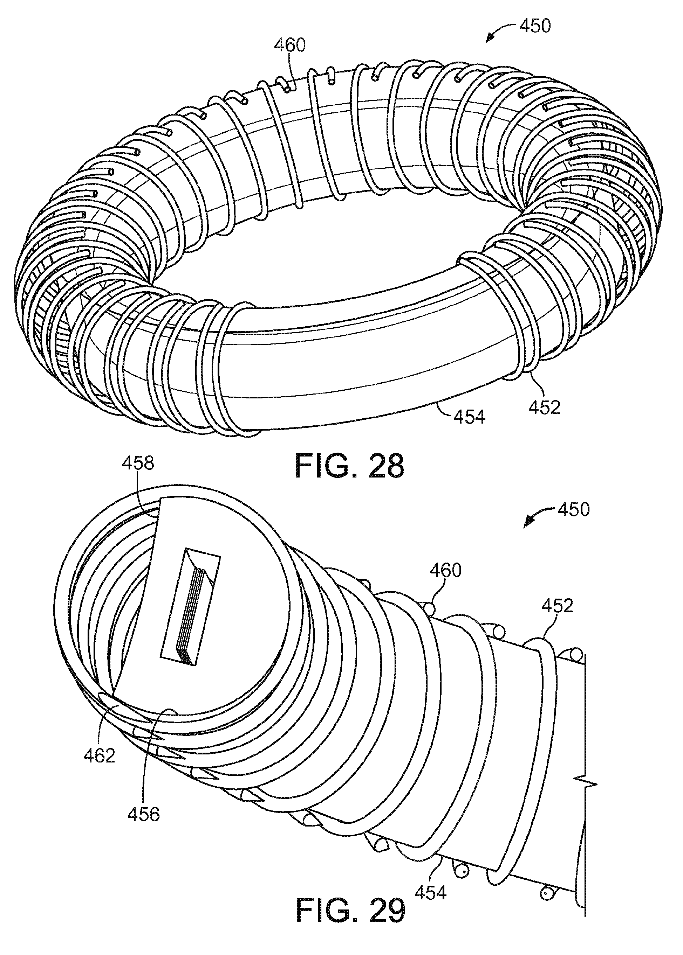

For example, as shown in figures 28 through 31, a support 450 could include anchors in the form of, say, 30 loops 452 equally spaced around the body 454 of the support. A cross-section of the body 454 could include a circular segment 456 along the inner periphery of the body, and a flat or concave section 458 along the outer periphery of the body. Each of the loops could include two free ends 460, 462, one of which 460 is un-pointed and the other of which 462 has a sharp point. The loop does not have any barbed features.

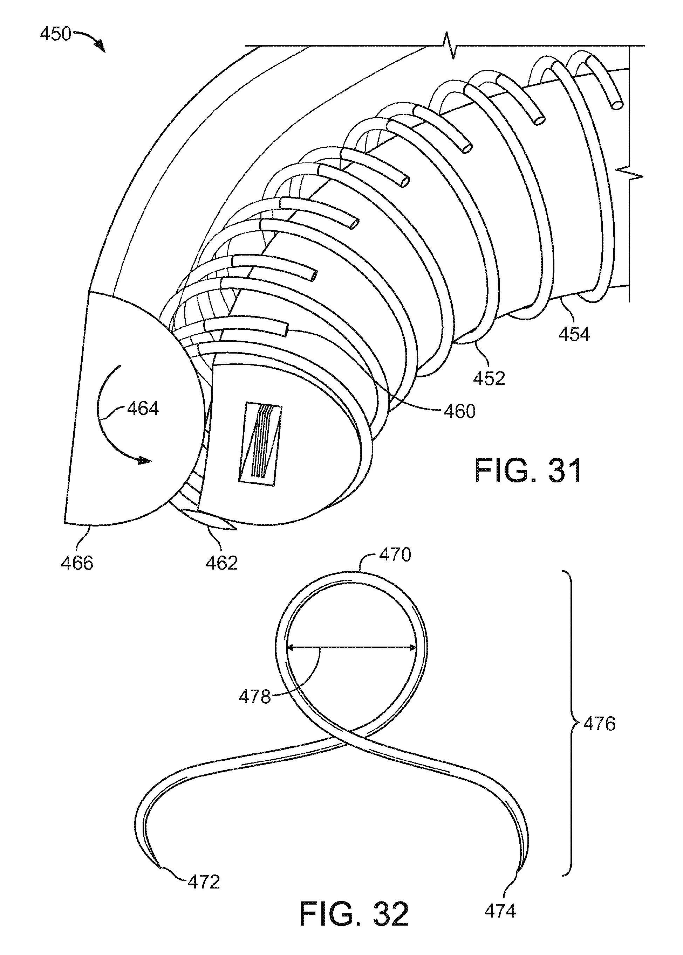

In some modes of operation, prior to insertion, the curved sharp ends 462 of all of the grippers can be held away from body and aimed in the general direction of the annulus tissue. A sheath or other mechanism could be used to move them into and hold them in this temporary insertion position. During insertion, the insertion tool could be applied to force the grippers into the tissue. Once the pointed ends of the grippers are in the tissue, the sheath or mechanism could be manipulated to allow the anchors to assume their final shape, after following curved paths 464 through the tissue 466 and exiting from the tissue to lie next to the support body, as shown in figure 31.

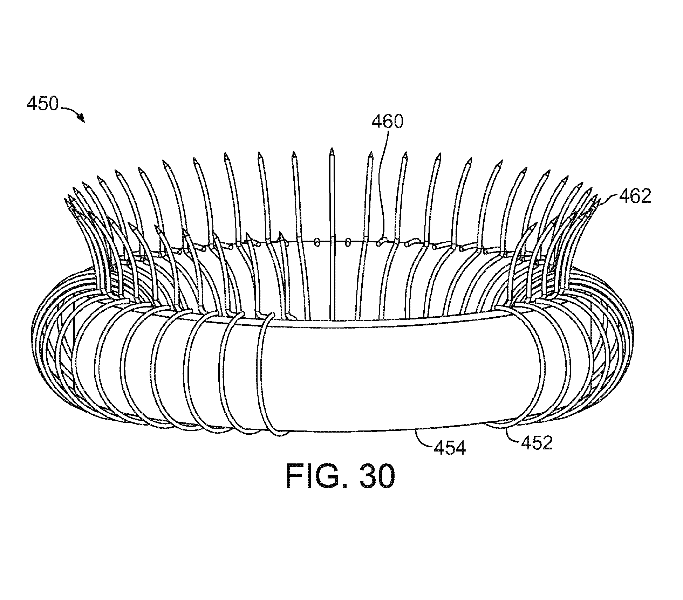

This configuration has the advantage that the process could be reversed using a similar sheath or mechanism to withdraw the grippers through the tissue and back to the configuration of figure 30. Because the gripping has been achieved by the curvature of the shafts of the anchors and not by barbs on the sharp tips, reversing the process is relatively easy. Gripping is also secure. However, insertion may be more difficult than in other implementations, and the reversibility requires an additional mechanism.

In some examples, the support could be provided with an adjustment and locking feature that would permit the size (e.g., the diameter) and possibly the shape of the support to be adjusted or locked or both, by the surgeon or operator at the time of insertion. In some cases, the support could be adjusted to different possible sizes at the time of insertion rather than requiring that it reach only a single non-selectable designed size.

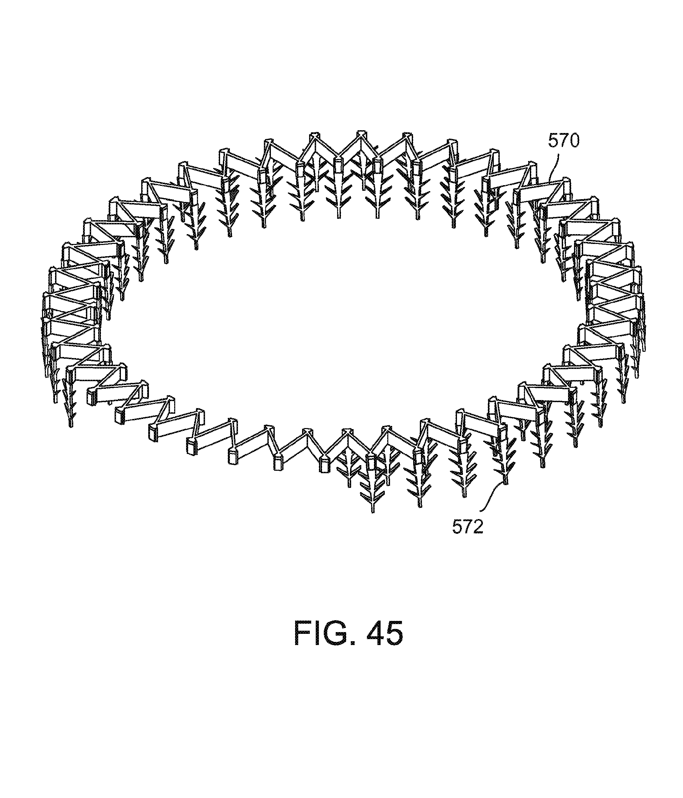

For example, as shown in figure 45, a core structural piece 570 of the support could be made of crimped stainless steel that is plastically deformed by an insertion tool (not shown). The tool could engage the top of the structural piece and force the piece temporarily to have a larger diameter for insertion. After pushing the support into the annulus to cause the grippers to attach to the tissue, the tool could collapse and allow the structural piece to collapse in diameter to its final size.

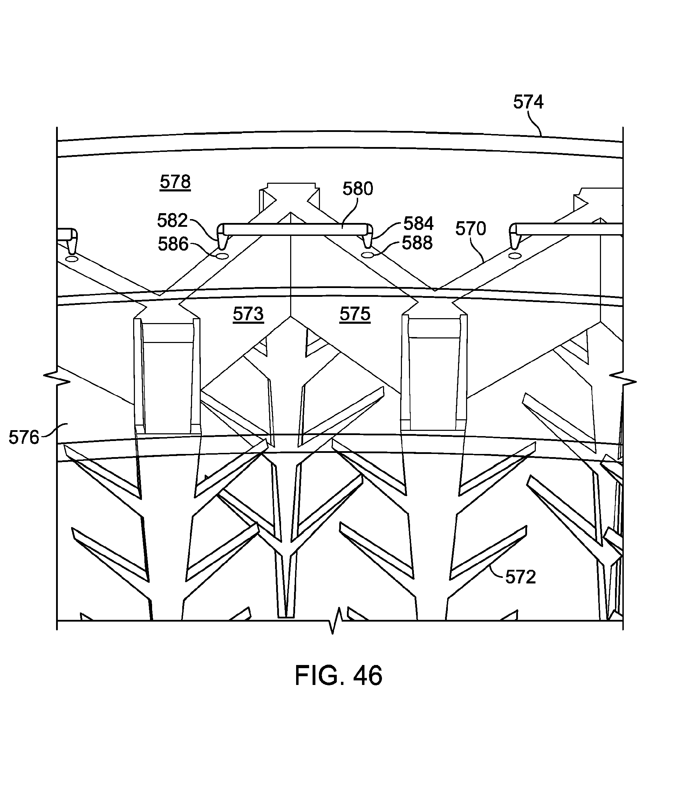

As shown in figure 46, in some cases, individual expansion elements 573, 575 would bear holes 576, 578 that have locations and spacing to mate exactly with the locations and spacings of pins 582, 584 in rigid locking elements 580 once the structural piece has been expanded or contracted to exactly the desired dimension. The locking elements would be held at the proper places in an annular silicone support that has inner and outer peripheral walls 574, 576 joined by an upper annular wall 578. Pushing down on the silicone support when the support is properly sized will force the pins of the locking elements into the holes.

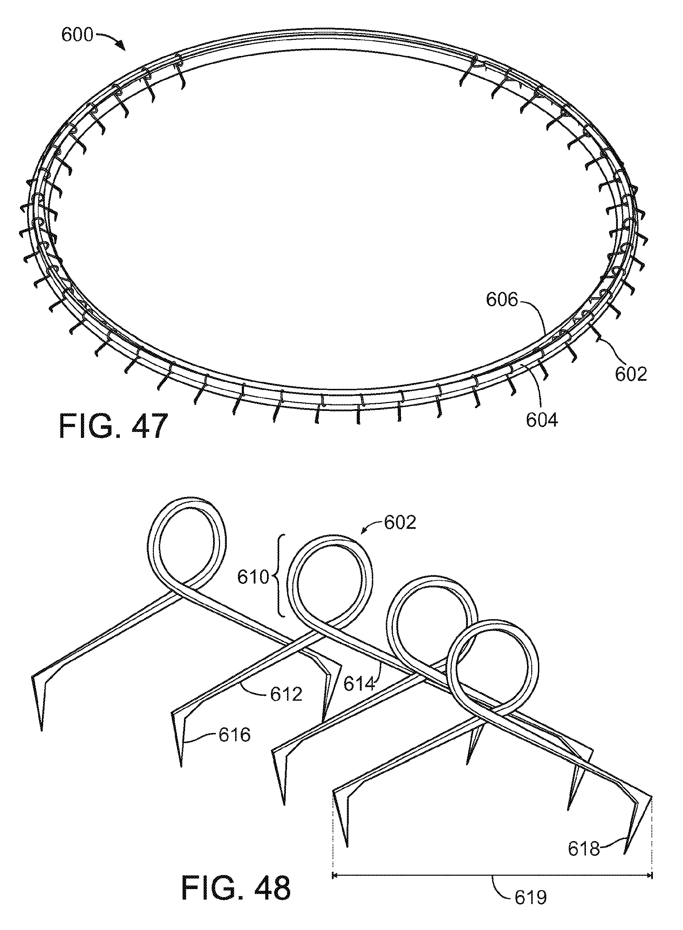

Referring to figures 47 through 53, in some implementations, the support 600 could be formed of three pieces.

One of the pieces, an annular resilient (e.g., silicone) ring 606 has a cross-section that includes four linear segments defining a trapezoid, which provide stability to the shape of the ring. There are four corresponding faces of the ring. Face 632 would have a configuration designed to match surfaces of a face of a dilator part of an insertion tool.

A second of the pieces is a metal ring 604 formed from a strip of, e.g., stainless steel having a curved cross-section and two overlapping ends 620, and 622. The curvature of the cross-section maintains the axial stability of the ring. Near one end 622, the ring has a series of slots that are meant to mate with corresponding tabs 623 formed near the other end 620. During fabrication and assembly the tabbed end of the ring is on the inside of the overlapping section 627 so that no mating and locking can occur. When finally installed, however, the tabbed end is on the outside of the overlapping section to permit locking. During manufacture, the silicone ring is molded around the metal ring. When the silicone ring is stretched and relaxed, the metal ring can expand and contract because the two ends are free to move relative to one another at the overlapping section. The support is essentially spring loaded.

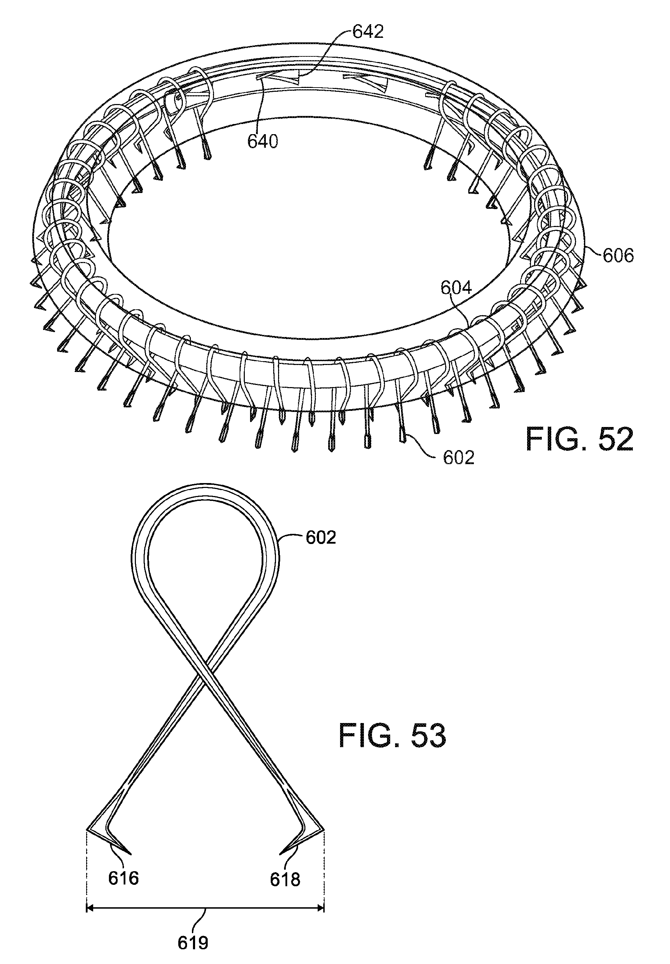

The third piece of this example support is a double-pointed anchor 602, many copies of which are arranged around the ring (in this version, but not necessarily, at regular intervals). In some implementations, each of the anchors is made from a single loop 602 of wire that has a gripper (a barb or a fish hook, for example) at opposite free ends 616, 618. Each of the anchors is resilient and has a relaxed state shown in figure 53, with a distance 619 between the two grippers, and the points of the two grippers pointing generally towards each other. The loops of the anchors are placed on the metal ring and potted in the molded silicone ring.

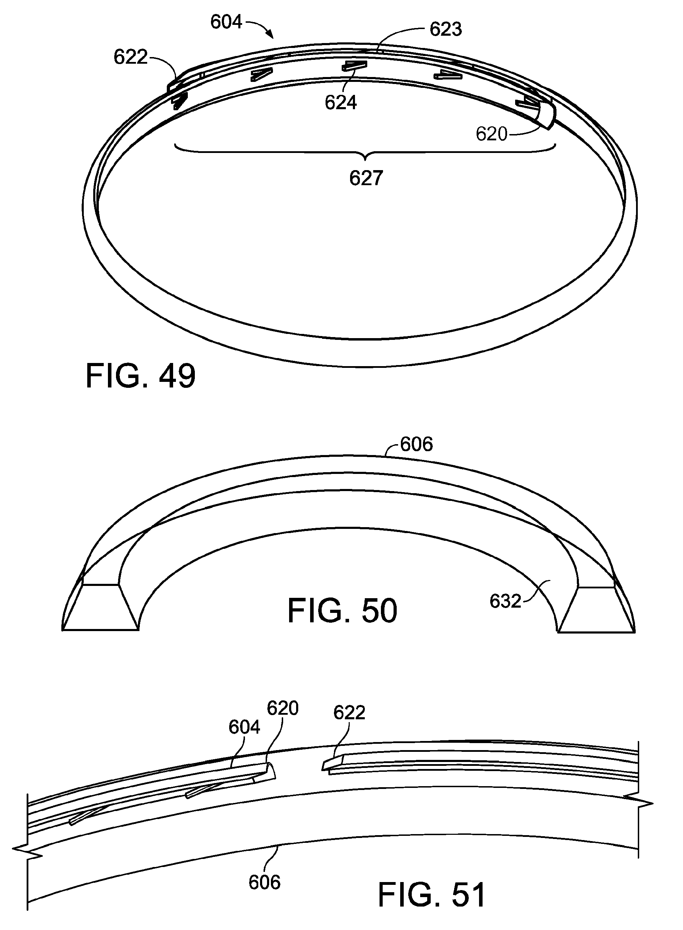

After assembly, the support is stretched to a larger diameter and mounted on an insertion tool, not shown. The stretching has two effects. One, shown in figure 51, is that the two ends of the metal ring are pulled apart sufficiently to eliminate the overlap. The ends of the ring are biased so that the tabbed end moves to the outside relative to the slotted end. So when the two ends again form the overlap upon the later contraction of the ring, the tabs are positioned to mate with the slots. The ends of the metal ring are beveled to assist in achieving this arrangement as the ring contracts.

Also, as the silicone ring expands, the cross-sectional diameter of the silicone ring contracts; because the anchors are potted within the silicone ring, as the ring stretches in length and contracts in diameter, the matrix squeezes the loops 610 of the anchors and forces them into a temporary configuration shown in figure 48, in which the distance 619 has increased and the orientation of the points of the grippers has rotated to face generally in the insertion direction, ready for insertion.

As shown in figure 52, when the insertion tool is removed from the support, the support contracts in diameter, which reconfigures the annulus to the desired shape and size. And the silicone rings expands in cross-sectional diameter, which allows the anchors to relax (figure 53), driving the grippers to rotate and force the points towards each other, to hold onto the tissue securely. As the metal ring contracts, the tabs and slots cooperate in a ratchet action which permits the support to contract to its final shape and size, while prevent a reverse expansion from occurring again.

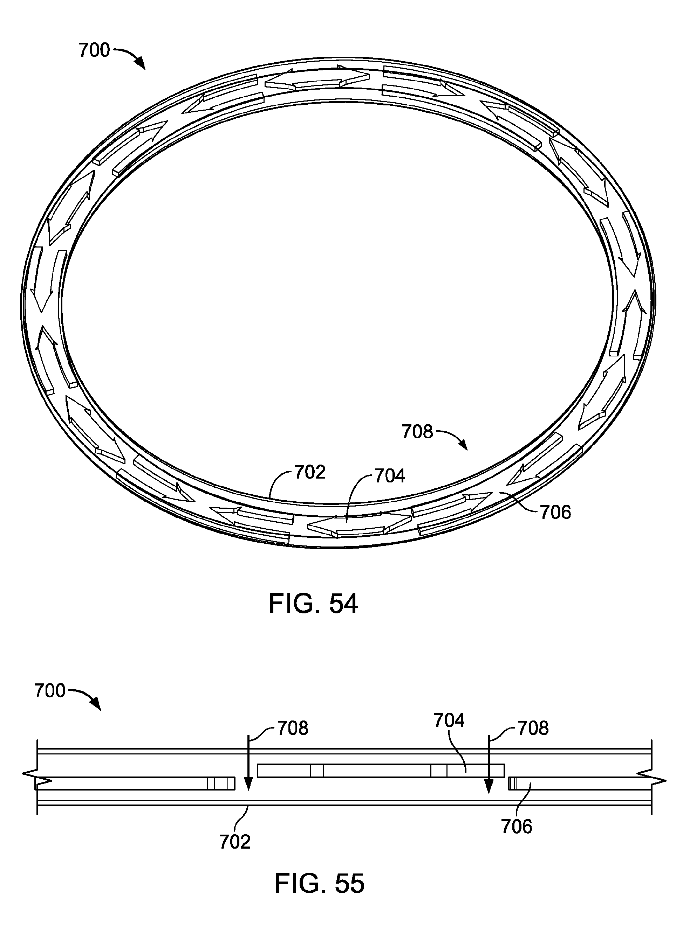

In some cases, shown in figures 54 and 55, the locking of the final diameter of the support can be achieved by embedding mating elements in a resilient ring 700. One set of elements 704 can be embedded in one plane of the ring, and a corresponding set of elements 706 to be mated can be embedded in a second plane of the ring. The embedding is done in a way that permits the two different kinds of mating elements to slide relative to one another as the support is expanded and contracted prior to and during installation. When the proper diameter of the support has been reached, a tool can be used to press down on the silicone ring to cause the mating elements to occupy the same plane and be interlocked.

In some examples, two interlocking elements 722 and 724 can be formed at the ends of a resilient metal coil 720 that forms part of the support. Once installed and properly sized, the support can be locked by pushing down to cause the interlocking elements to mate. In some cases, a support could have a central annular lumen filled with uncured polyurethane and arranged so that the diameter or shape or both of the support could be adjusted at the time of insertion. Once the desired diameter or shape or both have been reached, ultraviolet light, which could be delivered through a delivery tool or in other ways, would be used to cure and harden the polyurethane. Current curable materials and lighting can achieve curing in about 20 to 30 seconds.

Figures 32 through 35 show another example configuration that allows a reversible process for installing and removing the grippers from the annulus tissue for repositioning. Each of the anchors 470 incorporates a scissoring or pincering mechanism that has two pointed (but not barbed) grippers 472, 474 on opposite free ends of a 0.015 inch Nitinol wire loop. To form the each anchor, the wire is wound on a jig in the shape 476 shown in figure 32, which is the open configuration of the anchor. Then heat is used to memory set that open shape. The loop diameter 478 in this example could be about 0.20 inches for mounting on a toroidal resilient stretchable support body having a cross-sectional diameter 480 of about 0.25 inches.

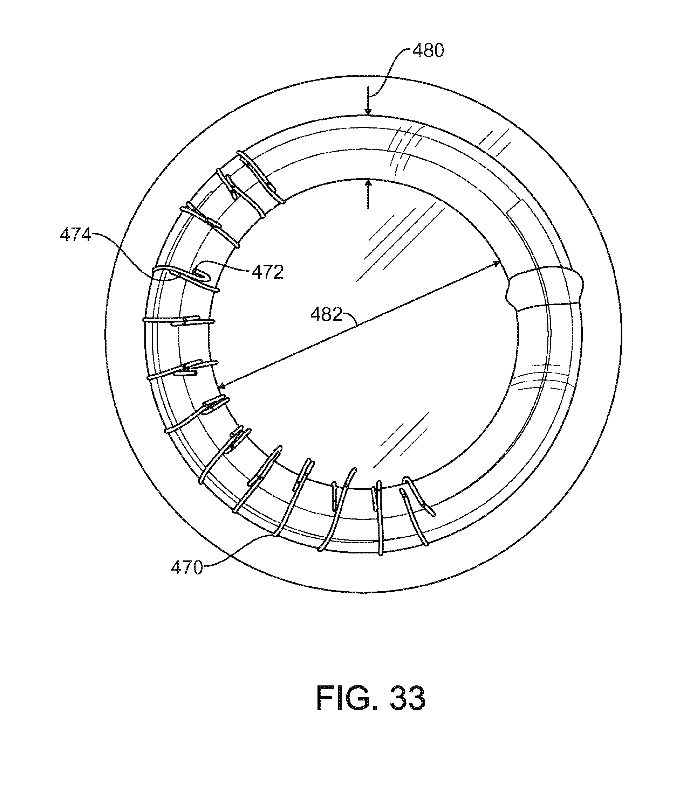

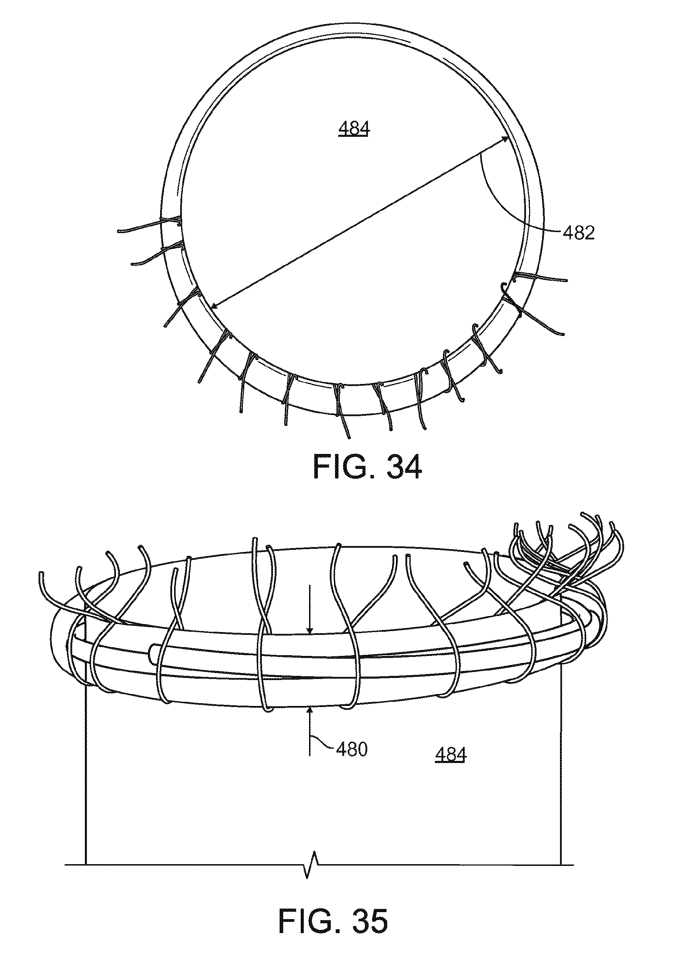

When the loop of each anchor is opened up to force it onto the larger diameter 480 support body, the configuration of the anchor automatically causes the two pointed free ends to close up into a gripping configuration as shown in figure 33. Prior to installation and before the support has been loaded onto the insertion tool, the support body is in its contracted installed shape as shown in figure 33, with all of the pincers closed. In figures 34 and 35 the support has been stretched to its insertion configuration, in which the diameter 482 is larger to fit onto (here a simulated) insertion tool 484. Because of the shape and configuration of the support body (for example, a silicone tube), when the body is stretched, its cross-sectional diameter is reduced allowing the anchors to relax to their native, open shape, ready for insertion.

Insertion proceeds by pushing the support towards the opened and properly shaped annulus causing the sharp points of the grippers to penetrate the tissue. As the insertion tool is removed from the support, the support body contracts to the final desired shape and diameter of the valve annulus. As it contracts, the pincers are forced to grasp the tissue of the annulus and hold the support securely in place. Thus, the support is relatively easy to insert and can be removed and repositioned by reversing the process, that is by expanding the support body, which releases the pincers.

A wide variety of insertion tools (which we also sometimes call dilators) can be used to attach a support to the heart valve annulus tissue. Some have been described earlier and we describe others below.

An important principle of the configuration and operation of at least some examples of insertion tools is that they enable a surgeon or catheter operator to install the support reliably and easily in a wide range of patients having heart valves that are in a wide variety of conditions and have a wide variety of shapes and sizes,. In other words, insertion can be achieved routinely and simply. This can be done by an insertion tool that automatically and easily temporarily expands and reconfigures any heart valve annulus to adopt a common expanded shape or size or both so that a support that has been pre-expanded to the common shape or size or both can be attached without concern for the unstreteched context and configuration of the patient's valve annulus. The support is configured so that after insertion the support can be reconfigured automatically or by manipulation to a final secure stable desired shape and size, with the insertion tool removed.

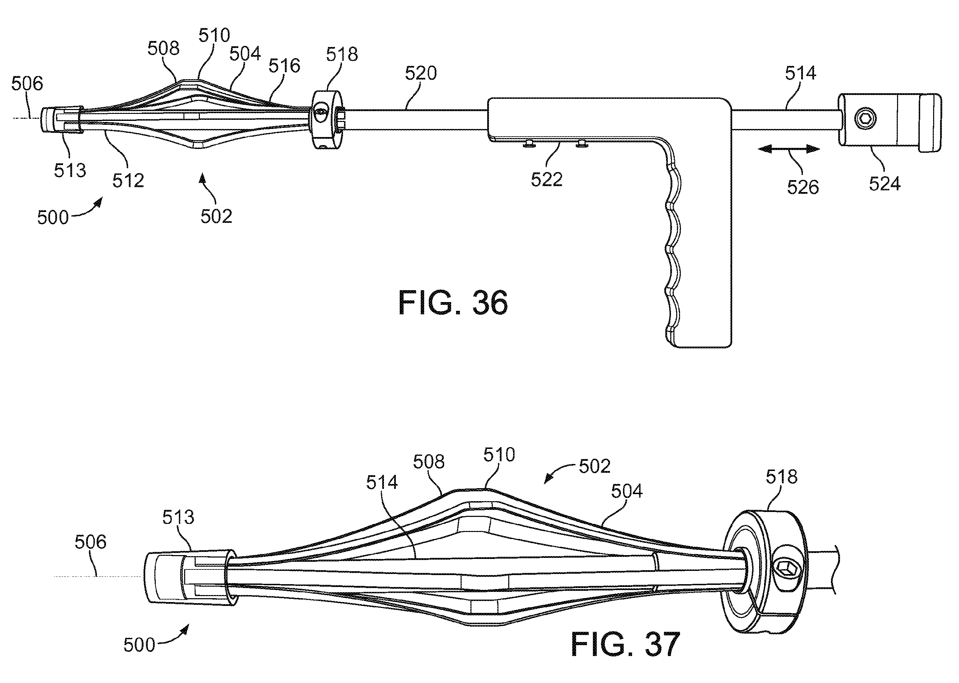

Figures 36 through 39 illustrate an example of an insertion tool 500 that includes a dilator 502 formed of six arms 504 arranged at equal intervals around an insertion axis 506. Each of the arms is formed of a 0.125" wide spring steel metal strip that is bent at two places 508 and 510. Ends 512 of the arms are gathered together and held by a segment of plastic tubing 513 on the end of an aluminum inner tube 514 (0.28" outside diameter, 0.24" inside diameter). The opposite ends 516 of the arms are gathered together and held by a segment of tubing and a shaft collar 518 to an aluminum outer tube 520 (0.37" outer diameter, 0.30" inner diameter). The outer tube is connected to a handle 522. The inner tube, which slides within the outer tube along the insertion axis, is manipulated by a second handle 524.

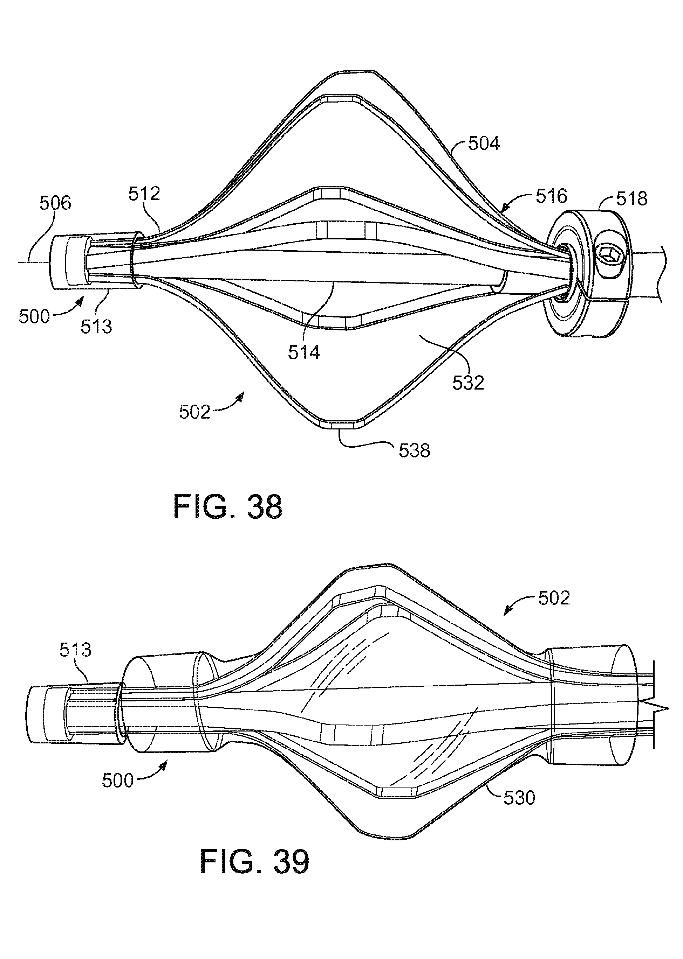

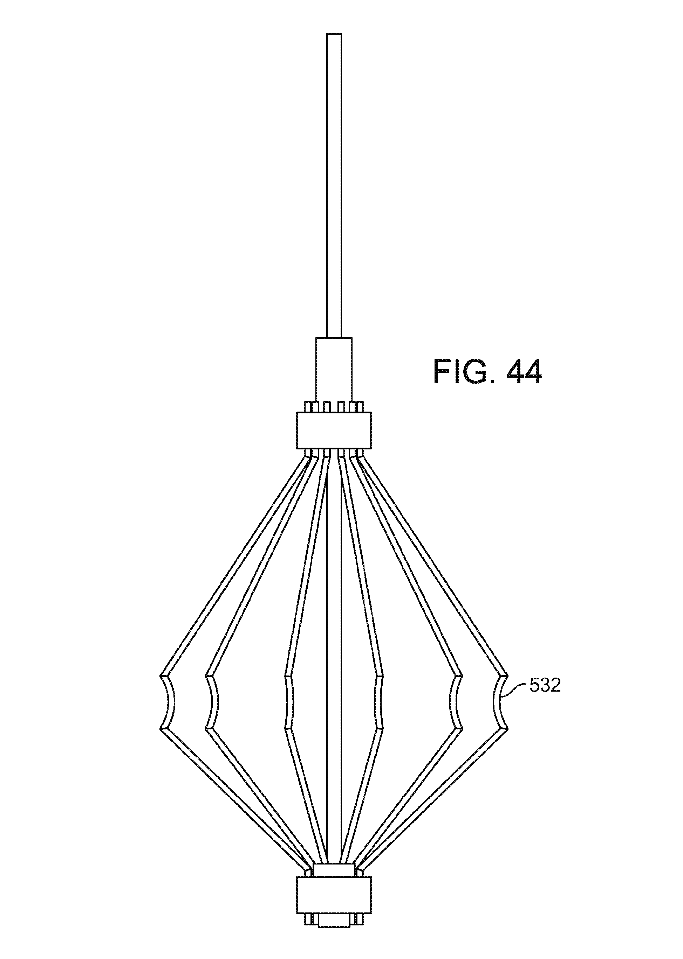

By pushing or pulling 526 on the second handle relative to the first handle, the inner tube is moved back and forth relative to the outer tube, which causes the arms to dilate as in figure 38 or contract as in figure 37. A thin molded sleeve of, e.g., silicone, 530 protects the mechanism and protects the heart tissue and the support from damage. Prior to installation of the support in the heart valve, the support is stretched and mounted on the dilator at the central ridge 532. It can be held in place by force and friction or can be lashed with sutures that are cut after installation, or the central ridge can be provided with a concavity in which the support is seated. Another view of the central ridge 532 is shown in figure 44.

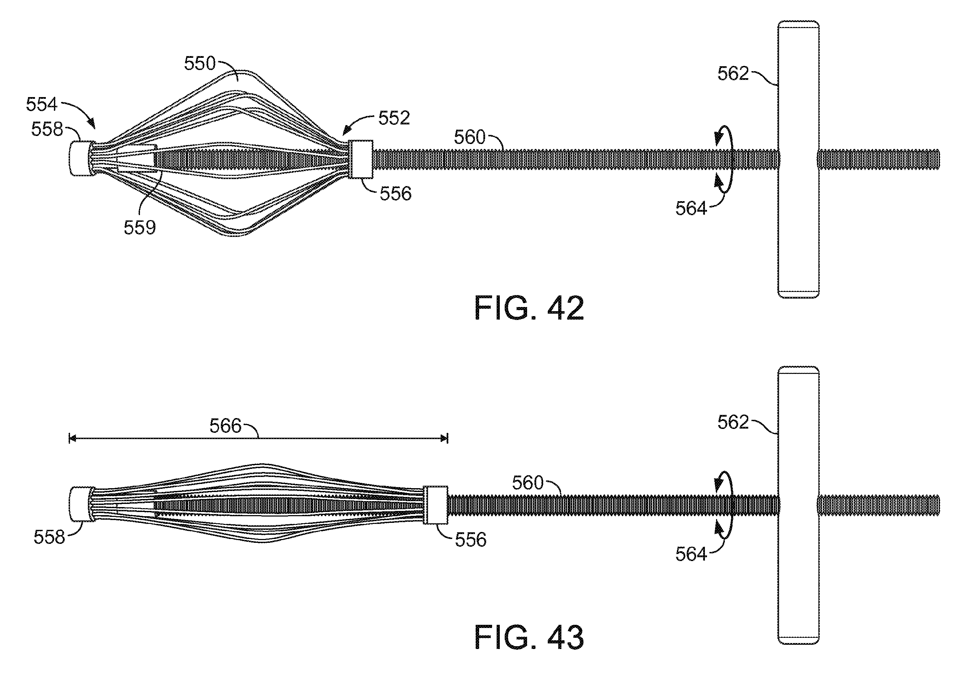

As shown in figures 42 and 43, in some examples, a dilator can include round wire arms 550 that are evenly spaced around the insertion axis and have each been shape set to the expanded configuration shown in figure 42. The ends 552, 554 of each wire are secured respectively to two circular hubs 556 558. The upper hub 556 has a central hole (not shown) that is threaded to receive a threaded rod 560 to which a handle 562 is clamped. The other end 559 of the threaded rod is fixed to the hub 558. Using the handle to turn 564 the threaded rod advances it or withdraws it (depending on the direction of rotation) through the upper hub, toward or away from the lower hub. The rod pushes or pulls on the lower hub, thereby increasing or decreasing the distance 566 between the two hubs and forcing the arms to contract or allowing them to expand to the shape set expanded configuration.

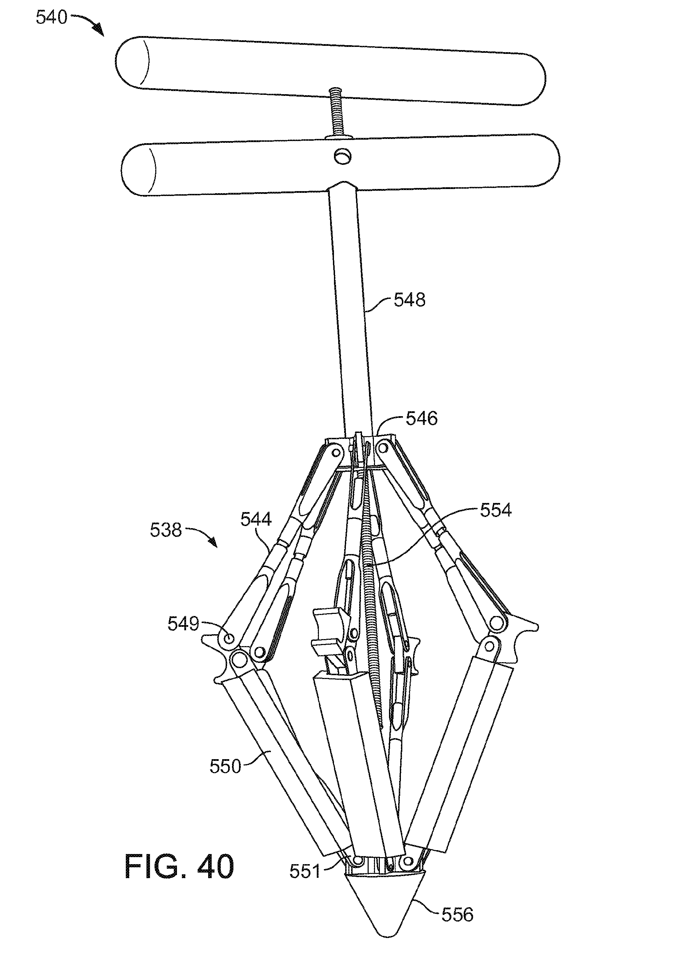

As shown in figure 40, in some implementations each arm 538 of an insertion tool 540 is formed of a stiff limb 544 connected at one end 546 to the outer tube 548, and at another end 549 to a broader limb 550. The other end 551 of the second limb is connected to the inner tube 554 at a tip 556. The limbs are joined by a hinged element that allows them to pivot relative to each other. On each of the arms, a clip 560 has a recess to capture the support at one location along its perimeter.

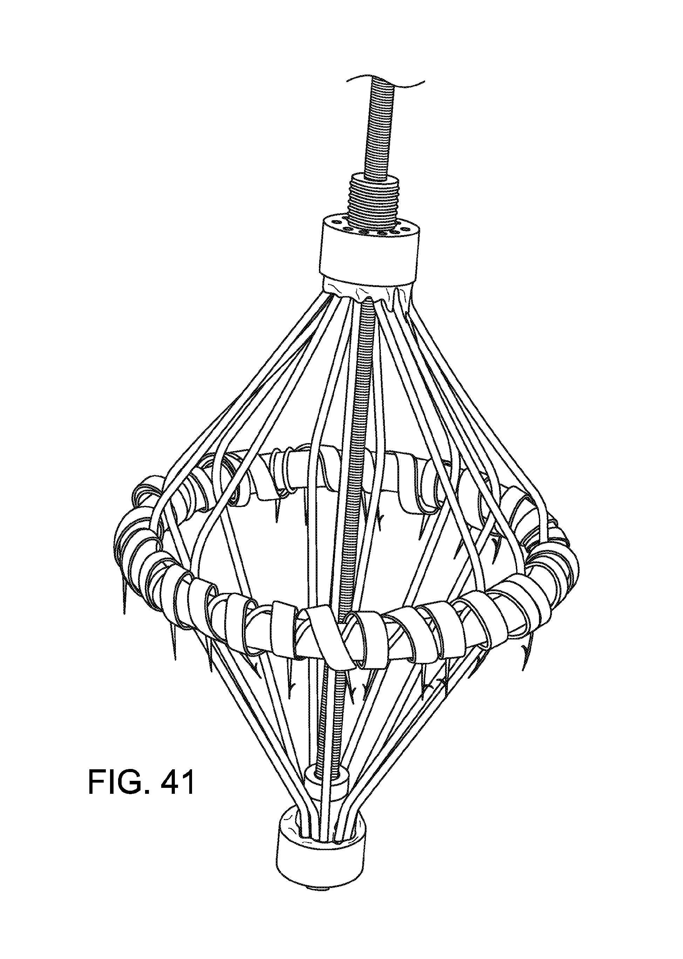

Figure 41 shows a support mounted on an insertion tool ready for insertion.

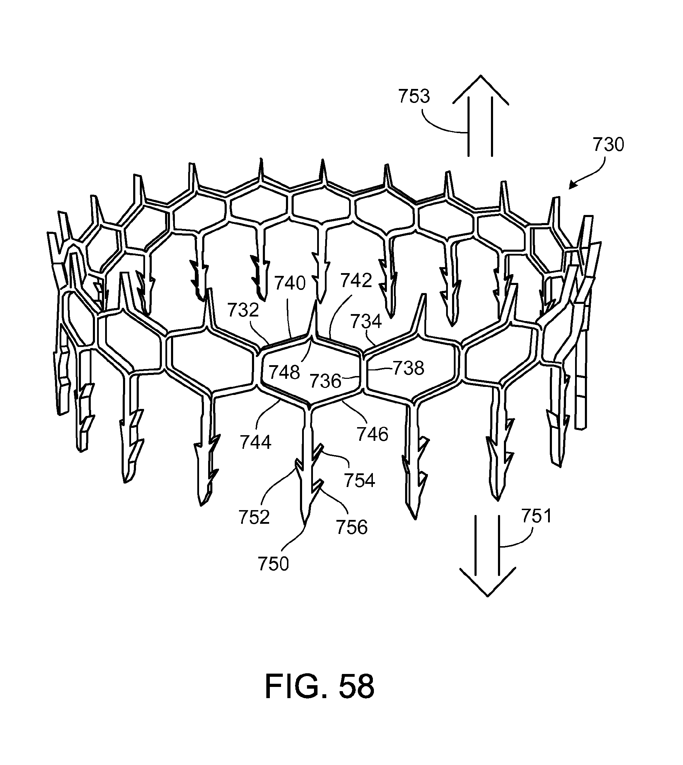

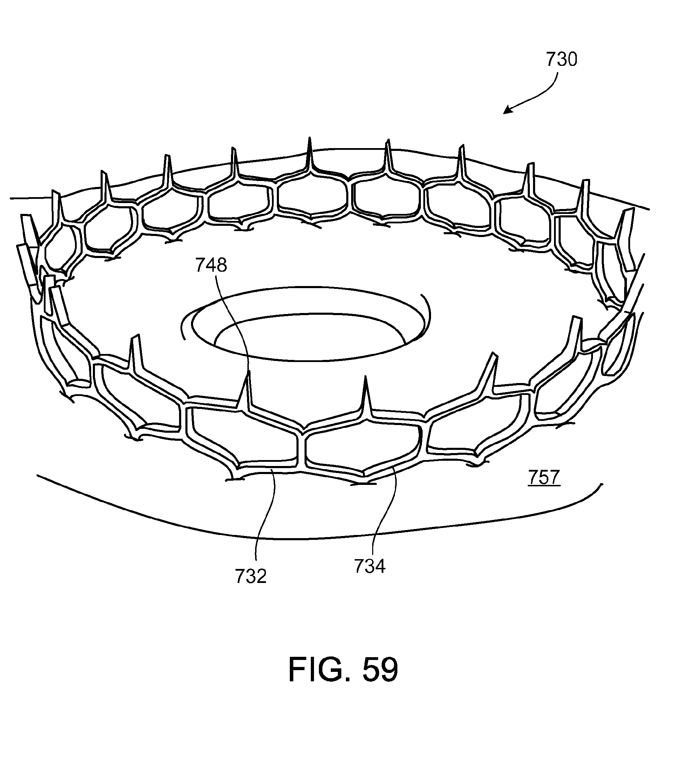

Figures 58 and 59 show a version 730 of the support. This version 730 has a ring of successive hexagonal sections 732, 734 touching at short edges 736, 738. At the junction of longer edges 740, 742, 744, 746 of the hexagonal sections are sharp free ends 748, 750, pointing in opposite directions. Further, on each hexagonal section, one sharp free end 750 is longer than the other sharp free end 748 and has barbs 752, 754, 756 for gripping tissue 757 that the barbed sharp free end 750 has pierced. All of the barbed sharp free ends 750 point in the same direction 751 on all of the hexagonal sections 732, 734. The other set of free ends 748 have no barbs and can further stabilize the support by piercing other adjacent tissue if any is present, lodging themselves inside and further securing the support to the tissue. All of the other free ends 748 point in the same direction 753 which is opposite the direction 751 that the barbed sharp free ends 750 point to.

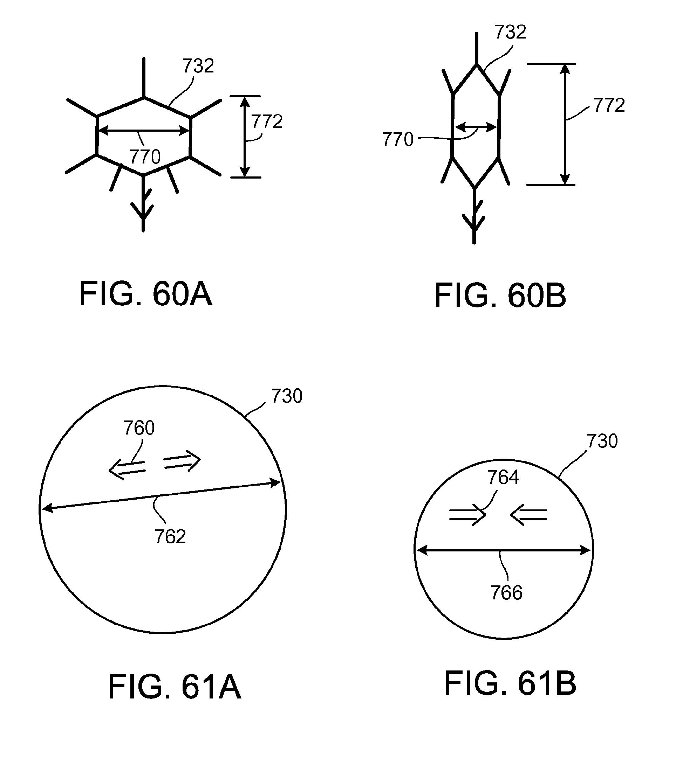

This version 730 of the support is resilient and can be expanded to a delivery configuration and later will contract to a final configuration. As shown in Figures 60A and 61A, when the support is expanded 760 to a larger diameter 762 in a delivery configuration, e.g. by a delivery tool, each hexagonal section 732 increases in width 770 and decreases in height 772. As shown in Figures 60B and 61B, when the support contracts 764 to a smaller diameter 766 in a final configuration, each hexagonal section 732 decreases in width 770 and increases in height 772. In some implementations, this version 730 of the support can be made of a flexible shape memory material such as Nitinol or a biologically compatible elastomer (or other material) that is configured to contract 764 the support to the final configuration after insertion into tissue. For example, the support may be configured to contract upon a period of exposure to the temperature of the human body.

Other implementations are within the scope of the following claims.

Although the present invention is defined in the attached claims, it should be understood that the present invention can also (alternatively) be defined in accordance with the following embodiments:
1. An apparatus comprising:
   a heart tissue support having gripping elements,
   each gripping element having a free end that is sharp enough to penetrate heart tissue when pushed against the tissue, and a feature to resist withdrawal of the gripping element from the tissue after the sharp free end has penetrated the tissue.
2. The apparatus of embodiment 1 in which the free ends of the gripping elements project away from a surface of the support.
3. The apparatus of embodiment 1 in which the feature that resists withdrawal of the gripping element from the tissue comprises a finger projecting laterally from the gripping element.
4. The apparatus of embodiment 1 in which the heart tissue support comprises an annular surface bearing the gripping elements.
5. The apparatus of embodiment 1 in which the support is expandable and contractible.
6. The apparatus of embodiment 5 in which the support has a native size that is configurable.
7. The apparatus of embodiment 6 in which a wire configures the native size.
8. The apparatus of embodiment 1 in which the support comprises at least one of stainless steel, gold, Nitinol, or a biologically compatible elastomer.
9. The apparatus of embodiment 1 in which the support comprises a torus.
10. The apparatus of embodiment 1 in which the support comprises a helically wound portion.
11. The apparatus of embodiment 1 in which some portions of the support bear no gripping elements.
12. The apparatus of embodiment 1 in which the gripping elements are organized in a pattern.
13. The apparatus of embodiment 12 in which the pattern comprises rows.
14. The apparatus of embodiment 12 in which the pattern comprises a group in which the gripping elements are more densely placed and a group in which the gripping elements are less densely placed.
15. The apparatus of embodiment 12 in which the pattern comprises arcs.
16. The apparatus of embodiment 12 in which the pattern comprises clusters.
17. The apparatus of embodiment 12 in which the pattern comprises random placement.
18. The apparatus of embodiment 1 in which at least some of the gripping elements comprise at least one of platinum, gold, palladium, rhenium, tantalum, tungsten, molybdenum, nickel, cobalt, stainless steel, Nitinol, and alloys of any combination of them.
19. The apparatus of embodiment 1 in which the gripping elements have the same size.
20. The apparatus of embodiment 1 in which some of the gripping elements are of different sizes.
21. The apparatus of embodiment 1 in which at least some of the gripping elements have more than one of the feature that resists withdrawal.
22. The apparatus of embodiment 2 in which at least some of the gripping elements project from the surface orthogonally.
23. The apparatus of embodiment 1 in which at least some of the gripping elements are curved.
24. The apparatus of embodiment 1 also including a sleeve through which tissue can grow.
25. The apparatus of embodiment 24 in which the sleeve comprises polyethylene terephthalate.
26. The apparatus of embodiment 1 in which there are between about 15 and a million gripping elements on the support.
27. The apparatus of embodiment 1 in which there are between about 100 and about 100,000 gripping elements.
28. The apparatus of embodiment 1 in which the gripping elements comprise burr hooks.
29. The apparatus of embodiment 1 in which the gripping elements comprise arrows.
30. The apparatus of embodiment 1 in which the gripping elements comprise hooks.
31. A method comprising:
   correcting the shape of a heart valve annulus in a catheter laboratory by orienting a tip of a catheter holding a heart tissue support that has gripping elements at the valve annulus, applying a radial force from the catheter against the annulus by opening a structure at the tip of the catheter, and while the structure is opened, forcing the support onto the valve annulus.
32. A method comprising:
   correcting the shape of a heart valve annulus during a surgical procedure by pushing a heart tissue support that has gripping elements onto the valve annulus.
33. A method comprising
   attaching, to different sized heart valve annuli in different patients, supports that can be expanded in preparation for attachment and allowed to contract to a common relaxed, non-expanded native size when they are in place on the annuli, and reducing the sizes of at least some of the in-place supports to be smaller than the common relaxed non-expanded native size, to accommodate the different sized heart valve annuli of different patients.
34. A heart tissue support comprising:
   a large number of small grippers, each having a tissue penetration feature and a retention feature, and the configuration of the grippers relative to a configuration of a given area of heart tissue to which the support is to be attached by force being such that
      (1) the penetration features of a failed set of the grippers will fail to penetrate the tissue,
      (2) the penetration features of a second set of the grippers will successfully penetrate the tissue,
      (3) the retention features of a subset of the second set of grippers will fail to retain the grippers in the tissue, and
      (4) the retention features of the remaining grippers of the second set will successfully retain the grippers in the tissue and hold the support in an intended configuration on the tissue.
35. A method comprising:
   pushing a support onto a region of heart tissue to cause only a portion of a number of small grippers on the support to embed themselves and be retained in the tissue, the portion being sufficient to attach the support securely to the heart tissue.
36. An apparatus comprising:
   an annular heart valve support that is expandable and contractible and bears gripping elements configured to penetrate heart tissue and to retain the elements in the tissue after penetration.
37. A tool to attach a support to a heart valve annulus, the tool comprising mechanisms to hold the support in an expanded configuration prior to attachment, to expand the heart valve annulus prior to attachment, to enable the attachment of the support in its expanded configuration to the expanded valve annulus, and to release the expanded support to a contracted configuration after the attachment.
38. The tool of embodiment 37 attached to an end of a catheter.
39. The tool of embodiment 37 also comprising an inflatable balloon.
40. The tool of embodiment 39 in which the balloon plays a role in positioning the tool.
41. The tool of embodiment 37 also in which the mechanisms are also to remove the tool from the heart after attachment.
42. A tool to attach a support to a heart valve annulus, the tool comprising a structure to expand the annulus of the heart to a predetermined shape under control of an operator.
43. The tool of embodiment 42 in which the structure has a conical outer surface at least a portion of which conforms to the predetermined shape.
44. The tool of embodiment 42 in which the structure has an outer surface that can be expanded to the predetermined shape.
45. An apparatus comprising
   a support for living tissue, the support having
   gripping elements to attach to the living tissue and to hold the support securely in place, and an annular structure that is coupled to the gripping elements,
   adjusts the support between a first configuration for installing the support and a second configuration in which the support is held securely in place after installation, and
   is self-supporting in the first and second configurations.
46. The apparatus of embodiment 45 in which the living tissue comprises heart tissue.
47. The apparatus of embodiment 45 in which the living tissue comprises a heart valve annulus.
48. The apparatus of embodiment 45 in which the gripping elements are configured to penetrate the tissue during installation and to grip the tissue after installation.
49. The apparatus of embodiment 45 in which the annular structure is round.
50. The apparatus of embodiment 45 in which the annular structure has elements that move annularly relative to one another to adjust the support between the first configuration and the second configuration.
51. The apparatus of embodiment 45 in which the annular structure is larger when the support is in the first configuration than when the support is in the second configuration.
52. The apparatus of embodiment 45 in which the annular structure changes its shape to adjust the support.
53. The apparatus of embodiment 45 in which the annular structure changes its shape during installation without altering locations of the gripping elements relative to the living tissue.
54. The apparatus of embodiment 45 in which the annular structure includes a first element to which the gripping elements are attached and a second element that can slide relative to the first element when the annular structure adjusts the support between the first configuration and the second configuration.
55. The apparatus of embodiment 54 in which the first element comprises a resilient annular tube to which the gripping elements are attached and the second element comprises a rigid adjustable piece that can slide within the tube.
56. The apparatus of embodiment 54 in which the second element comprises a self-supporting coil.
57. The apparatus of embodiment 55 in which the self-supporting coil comprises a rigid strip material having two free ends that are movable relative to one another to adjust the support between the first configuration and the second configuration.
58. The apparatus of embodiment 45 in which the annular structure comprises features that define spacings of the gripping elements that are coupled to the annular structure.
59. The apparatus of embodiment 45 in which the gripping elements are adjustable between a first arrangement for installing the support and a second arrangement in which the support is held securely in place after installation.
60. The apparatus of embodiment 59 in which, the annular structure and the gripping elements are configured so that, when the support is adjusted between the first configuration and the second configuration, the gripping elements are automatically adjusted between the first arrangement and the second arrangement.
61. The apparatus of embodiment 60 in which the annular structure includes a cross-sectional area that increases when the support is adjusted between the first configuration and the second configuration, and the gripping elements are coupled to the annular structure to cause the gripping elements to be adjusted between the first arrangement and the second arrangement when the cross-sectional area of the annular structure decreases.
62. The apparatus of embodiment 59 in which each of the gripping elements includes a loop and a piercing element attached to the loop, and the orientation of the piercing element changes as a configuration of the loop changes between the first arrangement and the second arrangement.
63. The apparatus of embodiment 45 in which the adjustment of the support between the first configuration and the second configuration is reversible without the gripping elements damaging the tissue.
64. The apparatus of embodiment 45 in which the annular structure includes a lock that prevents a change in the configuration of the support.
65. The apparatus of embodiment 64 in which the lock comprises a pair of mating elements.
66. The apparatus of embodiment 65 in which one of the mating elements includes a tab and the other includes a slot.
67. The apparatus of embodiment 65 in which one of the mating elements includes a pin and the other includes a hole for the pin.
68. The apparatus of embodiment 64 in which the annular structure has a central axis and includes two portions that can be moved relative to one another around the central axis to a position at which the mating elements mate.
69. An apparatus comprising
   a gripper including
   a point to penetrate living tissue,
   a resilient loop to support the point, the resilient loop having a relaxed configuration and a non-relaxed configuration,
   the point having different orientations respectively associated with the relaxed configuration and the non-relaxed configuration.
70. The apparatus of embodiment 69 in which the point has at least one barb.
71. The apparatus of embodiment 69 in which the gripper includes more than one such point.
72. The apparatus of embodiment 69 the orientation of the point the resilient support having a configuration that
73. The apparatus of embodiment 69 in which the gripper comprises wire.
74. The apparatus of embodiment 69 in which the loop is round.
75. The apparatus of embodiment 69 in which the gripper comprises a formed strip of a material.
76. The apparatus of embodiment 69 in which there are two such points that face each other and act as a pincer in at least one of the relaxed configuration and the non-relaxed configuration.
77. A heart valve repair ring comprising
   a round self-supporting resilient ring that has a relaxed diameter that corresponds to a healthy heart valve and an enlarged diameter that fits an insertion tool,
   grippers attached to the resilient ring and oriented in a direction that changes automatically between an insertion orientation and an installed orientation during installation.
78. A method comprising
   forcing pointed grippers on a support to penetrate tissue of a heart valve annulus and to hold the support securely on the annulus to correct a shape of the annulus, and
   removing at least a portion of the support from the annulus by withdrawing at least some of the pointed grippers without damaging the heart value annulus.
79. A method comprising mounting an expanded resilient support on a heart valve annulus,
   allowing the support to contract to a diameter of a healthy annulus, and locking the contracted support in its contracted diameter by causing elements of the support to mate.
80. A method comprising
   causing gripping elements that are coupled to a heart annulus support ring to seat in tissue of the annulus by permitting the support ring to contract from an expanded size to a smaller size during installation.
81. The apparatus of embodiment 1 in which
   the support comprises annular elements, pairs of which are connected along edges of the elements to form a ring.
82. The apparatus of embodiment 81 in which
   each of annular elements bears at least one of the grippers.
83. The apparatus of embodiment 82 in which each of the annular elements also bears a pointed element aimed in an opposite direction from the gripper.
84. The apparatus of embodiment 81 in which each of the annular elements comprises a hexagon.

## Claims

1. An apparatus comprising
a support for living tissue, the support having gripping elements to attach to the living tissue and to hold the support securely in place, and
an annular structure that is coupled to the gripping elements, adjusts the support between a first configuration for installing the support and a second configuration in which the support is held securely in place after installation, and is self-supporting in the first and second configurations.

2. The apparatus of claim 1 in which the living tissue comprises heart tissue, or in which the living tissue comprises a heart valve annulus.

3. The apparatus of claim 1 in which the gripping elements are configured to penetrate the tissue during installation and to grip the tissue after installation.

4. The apparatus of claim 1 in which the annular structure is round, or in which the annular structure has elements that move annularly relative to one another to adjust the support between the first configuration and the second configuration, or in which the annular structure is larger when the support is in the first configuration than when the support is in the second configuration.

5. The apparatus of claim 1 in which the annular structure changes its shape to adjust the support, or in which the annular structure changes its shape during installation without altering locations of the gripping elements relative to the living tissue.

6. The apparatus of claim 1 in which the annular structure includes a first element to which the gripping elements are attached and a second element that can slide relative to the first element when the annular structure adjusts the support between the first configuration and the second configuration, in particular in which the first element comprises a resilient annular tube to which the gripping elements are attached and the second element comprises a rigid adjustable piece that can slide within the tube, or in which the second element comprises a self-supporting coil.

7. The apparatus of claim 1 in which the annular structure comprises features that define spacings of the gripping elements that are coupled to the annular structure.

8. The apparatus of claim 1 in which the gripping elements are adjustable between a first arrangement for installing the support and a second arrangement in which the support is held securely in place after installation, in particular in which, the annular structure and the gripping elements are configured so that, when the support is adjusted between the first configuration and the second configuration, the gripping elements are automatically adjusted between the first arrangement and the second arrangement, in particular in which the annular structure includes a cross-sectional area that increases when the support is adjusted between the first configuration and the second configuration, and the gripping elements are coupled to the annular structure to cause the gripping elements to be adjusted between the first arrangement and the second arrangement when the cross-sectional area of the annular structure decreases.

9. The apparatus of claim 1 in which the adjustment of the support between the first configuration and the second configuration is reversible without the gripping elements damaging the tissue.

10. The apparatus of claim 1 in which the annular structure includes a lock that prevents a change in the configuration of the support.

11. The apparatus of claim 10 in which the lock comprises a pair of mating elements, in particular in which one of the mating elements includes a tab and the other includes a slot, or in which one of the mating elements includes a pin and the other includes a hole for the pin.

12. An apparatus comprising
a gripper including a point to penetrate living tissue, a resilient loop to support the point, the resilient loop having a relaxed configuration and a non-relaxed configuration, the point having different orientations respectively associated with the relaxed configuration and the non-relaxed configuration.

13. The apparatus of claim 12 in which the point has at least one barb, or in which the gripper includes more than one such point.

14. The apparatus of claim 12 in which the gripper comprises wire, or in which the loop is round, or in which the gripper comprises a formed strip of a material.

15. The apparatus of claim 12 in which there are two such points that face each other and act as a pincer in at least one of the relaxed configuration and the non-relaxed configuration.
